(19) Europäisches Patentamt
European Patent Office
Office européen des brevets

(11) EP 4 741 504 A1

(12) **EUROPEAN PATENT APPLICATION**
published in accordance with Art. 153(4) EPC

(43) Date of publication:
**13.05.2026 Bulletin 2026/20**

(21) Application number: **24835439.1**

(22) Date of filing: **05.07.2024**

(51) International Patent Classification (IPC):
***C12N 15/113*** *(2010.01)* ***A61K 31/713*** *(2006.01)*
***A61P 9/12*** *(2006.01)*

(52) Cooperative Patent Classification (CPC):
**A61K 31/713; A61P 9/12; C12N 15/113**

(86) International application number:
**PCT/CN2024/103907**

(87) International publication number:
**WO 2025/007961 (09.01.2025 Gazette 2025/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.07.2023 CN 202310826720**

(71) Applicant: **Rona Bioscience, Limited**
**Admiralty, Hong Kong (HK)**

(72) Inventors:
- **HUANG, Jinyu**
**Shanghai 201315 (CN)**
- **YE, Piao**
**Shanghai 201315 (CN)**

(74) Representative: **Maiwald GmbH**
**Elisenhof**
**Elisenstraße 3**
**80335 München (DE)**

Remarks:
The complete document including Reference Table(s) and the Sequence Listing(s) can be downloaded from the EPO website

(54) **DSRNA MOLECULE FOR REGULATING EXPRESSION OF AGT**

(57) Provided are a double-stranded RNA for inhibiting the expression of angiotensinogen (AGT) in cells; a vector and cell comprising an encoding nucleotide thereof; and a method for using the dsRNA, vector or cell to treat a disease or symptom mediated by or associated with the expression of AGT in a subject.

EP 4 741 504 A1

## Description

### TECHNICAL FIELD

The present disclosure relates to the field of RNA interference.

### BACKGROUND

Angiotensinogen (AGT), an alpha globulin, is located upstream most in the blood pressure regulation system of the renin-angiotensin-aldosterone system (RAAS) and is the only precursor of all angiotensin peptides. Inactive AGT is converted to active angiotensin II (Ang-II) by sequential cleavage with renin and angiotensin converting enzymes. Then, the Ang-II acts through binding to its receptor (AT1-4).

Angiotensinogen is mainly synthesized and secreted by the liver and is present in the plasma $\alpha$-globulin fraction. In addition, angiotensinogen is also present in a variety of tissues that express local RAAS. Elevated angiotensinogen levels are associated with essential hypertension. Transgenic mice expressing rat angiotensinogen gene had hypertension, while mice lacking angiotensinogen gene had hypotension.

Regulation of AGT production is multifactorial and occurs at the transcriptional and posttranscriptional levels. AngII itself, glucocorticoids, estrogens, thyroxine, growth hormone, and various cytokines affect the production of AGT. Physiological and pathological conditions such as pregnancy, use of oral contraceptives, increased glucocorticoid secretion (due to hyperadrenal function or chronic stress), and chronic inflammation can lead to increased AGT production. Conversely, deficiency of the pituitary, thyroid, gonadal, or adrenal is associated with low plasma AGT levels.

The main source of circulating AGT is hepatocytes, which secrete AGT through constitutive pathways, resulting in relatively stable plasma levels, regardless of acute stress or cardiovascular changes. In addition to the liver, tissues such as adipocytes and epithelial cells of the proximal tubules of the kidney, and the brain also express Agt, and the generation of these tissues contributes to the activation of local ras. In the brain, AGT is mainly expressed in astrocytes (Milsted et al., 1990), while AT1 is present in brain endothelial cells (Wosik et al., 2007). Interestingly, mice lacking AGT showed blood-brain barrier rupture and disordered occlusion at tight junctions (Wosik et al., 2007), which strongly suggests that astrocytes maintain blood-brain barrier properties through the AGT-Ang-II-AT1 pathway. Furthermore, evidence of a positive correlation between BMI and circulating AGT levels suggests that AGT production in adipocytes contributes to circulating AGT levels.

AGT is the first gene found to be associated with essential hypertension. Human genetic evidence and animal research evidence show that blocking or inhibiting the expression of AGT can bring significant antihypertensive effects. In this regard, one method of treating hypertension is to reduce AGT expression through small interfering RNA (siRNA) based on RNA interference mechanism.

There is a need in the art for compositions and methods for treating diseases, disorders and conditions associated with angiotensinogen.

Reducing AGT expression via double-stranded RNA (dsRNA), particularly small interfering RNA (siRNA), based on RNA interference mechanisms, is a novel approach to treat diseases, disorders and conditions associated with angiotensinogen.

### SUMMARY

The present disclosure provides novel double-stranded RNAs (dsRNAs) for inhibiting the expression of angiotensinogen (AGT) in a cell, as well as a vector, a cell, and a kit and a pharmaceutical composition comprising the same, and a method of inhibiting or reducing angiotensinogen (AGT) gene expression or treating diseases or disorders benefiting from reduced angiotensinogen (AGT) expression with the dsRNA, vector, cell, as well as the pharmaceutical composition, and kit.

In a first aspect, the present disclosure provides a double stranded nucleotide (dsRNA) for inhibiting the expression of angiotensinogen (AGT) in a cell, comprising a sense strand and an antisense strand that form a double-stranded region, wherein said sense strand and said antisense strand each independently have a length of 15-30 nucleotides, and said antisense strand comprises a nucleotide sequence of at least 15 contiguous nucleotides from the nucleotide sequence set forth in any one of SEQ ID Nos: 21-40.

In some embodiments, the sense strand comprises a nucleotide sequence of at least 15 contiguous nucleotides from the nucleotide sequence set forth in any one of SEQ ID Nos: 1-20.

In some embodiments, the dsRNA is an siRNA.

In some embodiments, the double-stranded region is 15-25 nucleotide pairs, preferably 16-23 nucleotide pairs, or more preferably 18-20 nucleotide pairs in length.

In some embodiments, the antisense strand comprises a nucleotide sequence of at least 16 contiguous

nucleotides, a nucleotide sequence of at least 17 contiguous nucleotides, a nucleotide sequence of at least 18 contiguous nucleotides, a nucleotide sequence of at least 19 contiguous nucleotides, or a nucleotide sequence of at least 20 contiguous nucleotides from the nucleotide sequence set forth in any one of SEQ ID Nos: 21-40. In some preferable embodiments, the antisense strand comprises the nucleotide sequence set forth in any one of SEQ ID NOs: 21-40.

In some embodiments, the sense strand comprises a nucleotide sequence of at least 16 contiguous nucleotides, a nucleotide sequence of at least 17 contiguous nucleotides, or a nucleotide sequence of at least 18 contiguous nucleotides from the nucleotide sequence set forth in any one of SEQ ID Nos: 1-20. In some preferred embodiments, the sense strand comprises the nucleotide sequence set forth in any one of SEQ ID Nos: 1-20.

In one preferred embodiment, the siRNA comprises any of the following paired of sense strand sequences and antisense strand sequences:

(1) sense strand: SEQ ID NO: 1, antisense strand: SEQ ID NO: 21;
(2) sense strand: SEQ ID NO: 2, antisense strand: SEQ ID NO: 22;
(3) sense strand: SEQ ID NO: 3, antisense strand: SEQ ID NO: 23;
(4) sense strand: SEQ ID NO: 4, antisense strand: SEQ ID NO: 24;
(5) sense strand: SEQ ID NO: 5, antisense strand: SEQ ID NO: 25;
(6) sense strand: SEQ ID NO: 6, antisense strand: SEQ ID NO: 26;
(7) sense strand: SEQ ID NO: 7, antisense strand: SEQ ID NO: 27;
(8) sense strand: SEQ ID NO: 8, antisense strand: SEQ ID NO: 28;
(9) sense strand: SEQ ID NO: 9, antisense strand: SEQ ID NO: 29;
(10) sense strand: SEQ ID NO: 10,antisense strand: SEQ ID NO: 30;
(11) sense strand: SEQ ID NO: 11,antisense strand: SEQ ID NO: 31;
(12) sense strand: SEQ ID NO: 12,antisense strand: SEQ ID NO: 32;
(13) sense strand: SEQ ID NO: 13,antisense strand: SEQ ID NO: 33;
(14) sense strand: SEQ ID NO: 14,antisense strand: SEQ ID NO: 34;
(15) sense strand: SEQ ID NO: 15,antisense strand: SEQ ID NO: 35;
(16) sense strand: SEQ ID NO: 16,antisense strand: SEQ ID NO: 36;
(17) sense strand: SEQ ID NO: 17,antisense strand: SEQ ID NO: 37;
(18) sense strand: SEQ ID NO: 18,antisense strand: SEQ ID NO: 38;
(19) sense strand: SEQ ID NO: 19,antisense strand: SEQ ID NO: 39; and
(20) sense strand: SEQ ID NO: 20,antisense strand: SEQ ID NO: 40.

In some embodiments, substantially all nucleotides of the sense strand and substantially all nucleotides of the antisense strand are modified nucleotides. In some other embodiments, all nucleotides of the sense strand and all nucleotides of the antisense strand are modified nucleotides.

In some other embodiments, each of the sense strand and the antisense strand independently comprises one or more nucleotide modifications selected from the group consisting of a 2'-O-methyl modification, a 2'-fluoro modification, a SCP modification, a glycol modification, and a phosphorothioate internucleotide linkage modification.

In some embodiments, the antisense strand has a length of 21 nucleotides and has

(i) 2'-O-methyl modified nucleotides at positions 1, 3, 5, 9, 11, 13, 15, 17, 19, and 21, and 2'-fluoro modified nucleotides at positions 2, 4, 6, 8, 10, 12, 14, 16, 18, and 20 (numbered from the 5' end); and/or

(ii) phosphorothioate internucleotide linkages between nucleotide positions 1 and 2, between nucleotide positions 2 and 3, between nucleotide positions 19 and 20, and between nucleotide positions 20 and 21 (numbered from the 5' end).

In some embodiments, the antisense strand has a length of 21 nucleotides and has

(i) 2'-O-methyl modified nucleotides at positions 1, 3, 5, 9, 11, 13, 15, 17, 19, 20, and 21, and 2'-fluoro modified nucleotides at positions 2, 4, 6, 8, 10, 12, 14, 16, and 18 (numbered from the 5' end); and/or

(ii) phosphorothioate internucleotide linkages between nucleotide positions 1 and 2, between nucleotide positions 2 and 3, between nucleotide positions 19 and 20, and between nucleotide positions 20 and 21 (numbered from the 5' end).

In some embodiments, the antisense strand has a length of 21 nucleotides and has

(i) 2'-O-methyl modified nucleotides at positions 3, 4, 6, 8, 9, 10, 11, 13, 15, 16, 17, 18, 19, 20, and 21 (numbered from the 5' end);
(ii) a 2'-fluoro modified nucleotide at position 14 (numbered from the 5' end);
(iii) 2'-deoxy modifications at positions 2, 5, 7, and 12 (numbered from the 5' end);
(iv) an SCP modification at position 1 (numbered from the 5' end); and/or
(v) phosphorothioate internucleotide linkages between nucleotide positions 1 and 2, between nucleotide positions 2 and 3, between nucleotide positions 19 and 20, and between nucleotide positions 20 and 21 (numbered from the 5' end).

In some embodiments, the antisense strand has a length of 21 nucleotides and has

(i) 2'-O-methyl modified nucleotides at positions 3, 5, 9, 11, 13, 15, 17, 19, 20, and 21 (numbered from the 5' end);
(ii) 2'-fluoro modified nucleotides at positions 2, 4, 6, 8, 10, 12, 14, 16, and 18 (numbered from the 5' end);
(iii) a GNA modification at position 7 (numbered from the 5' end);
(iv) an SCP modification at position 1 (numbered from the 5' end); and/or
(v) phosphorothioate internucleotide linkages between nucleotide positions 1 and 2, between nucleotide positions 2 and 3, between nucleotide positions 19 and 20, and between nucleotide positions 20 and 21 (numbered from the 5' end).

In some embodiments, the sense strand has a length of 19 nucleotides and has:

(i) 2'-O-methyl modified nucleotides at positions 1 to 6, and 10 to 19, and 2'-fluoro modified nucleotides at positions 7-9 (numbered from the 5' end); and/or
(ii) phosphorothioate internucleotide linkages between nucleotide positions 1 and 2, between nucleotide positions 2 and 3, and between nucleotide positions 18 and 19 (numbered from the 5' end).

In some embodiments, the antisense strand comprises a sequence selected from any one of the sequences set forth in SEQ ID NOs: 81-105.

In some embodiments, the sense strand comprises a sequence selected from any one of the sequences set forth in SEQ ID NOs: 41-59.

In some preferred embodiments, the dsRNA comprises any of the paired of sense strand sequences and antisense strand sequences selected from:

| | | | |
|---|---|---|---|
| sense strand: | SEQ ID NO: 41, | antisense strand: | SEQ ID NO: 81; |
| sense strand: | SEQ ID NO: 42, | antisense strand: | SEQ ID NO: 82; |
| sense strand: | SEQ ID NO: 43, | antisense strand: | SEQ ID NO: 83; |
| sense strand: | SEQ ID NO: 44, | antisense strand: | SEQ ID NO: 84; |
| sense strand: | SEQ ID NO: 45, | antisense strand: | SEQ ID NO: 85; |
| sense strand: | SEQ ID NO: 46, | antisense strand: | SEQ ID NO: 86; |
| sense strand: | SEQ ID NO: 47, | antisense strand: | SEQ ID NO: 87; |
| sense strand: | SEQ ID NO: 48, | antisense strand: | SEQ ID NO: 88; |
| sense strand: | SEQ ID NO: 49, | antisense strand: | SEQ ID NO: 89; |
| sense strand: | SEQ ID NO: 50, | antisense strand: | SEQ ID NO: 90; |
| sense strand: | SEQ ID NO: 51, | antisense strand: | SEQ ID NO: 91; |
| sense strand: | SEQ ID NO: 52, | antisense strand: | SEQ ID NO: 92; |
| sense strand: | SEQ ID NO: 53, | antisense strand: | SEQ ID NO: 93; |
| sense strand: | SEQ ID NO: 54, | antisense strand: | SEQ ID NO: 94; |
| sense strand: | SEQ ID NO: 55, | antisense strand: | SEQ ID NO: 95; |
| sense strand: | SEQ ID NO: 56, | antisense strand: | SEQ ID NO: 96; |
| sense strand: | SEQ ID NO: 57, | antisense strand: | SEQ ID NO: 97; |
| sense strand: | SEQ ID NO: 58, | antisense strand: | SEQ ID NO: 98; and |
| sense strand: | SEQ ID NO: 59, | antisense strand: | SEQ ID NO: 99. |

In some embodiments, the sense strand comprises a sequence selected from any one of the sequences set forth in SEQ ID NOs: 113-131.

In some embodiments, the dsRNA comprises any of the paired of sense strand sequences and antisense strand sequences selected from:

| | | | |
|---|---|---|---|
| sense strand: | SEQ ID NO: 113, | antisense strand: | SEQ ID NO: 81; |
| sense strand: | SEQ ID NO: 114, | antisense strand: | SEQ ID NO: 82; |
| sense strand: | SEQ ID NO: 115, | antisense strand: | SEQ ID NO: 83; |
| sense strand: | SEQ ID NO: 116, | antisense strand: | SEQ ID NO: 84; |
| sense strand: | SEQ ID NO: 117, | antisense strand: | SEQ ID NO: 85; |
| sense strand: | SEQ ID NO: 118, | antisense strand: | SEQ ID NO: 86; |
| sense strand: | SEQ ID NO: 119, | antisense strand: | SEQ ID NO: 87; |
| sense strand: | SEQ ID NO: 120, | antisense strand: | SEQ ID NO: 88; |
| sense strand: | SEQ ID NO: 121, | antisense strand: | SEQ ID NO: 89; |
| sense strand: | SEQ ID NO: 122, | antisense strand: | SEQ ID NO: 90; |
| sense strand: | SEQ ID NO: 123, | antisense strand: | SEQ ID NO: 91; |
| sense strand: | SEQ ID NO: 124, | antisense strand: | SEQ ID NO: 92; |
| sense strand: | SEQ ID NO: 125, | antisense strand: | SEQ ID NO: 93; |
| sense strand: | SEQ ID NO: 126, | antisense strand: | SEQ ID NO: 94; |
| sense strand: | SEQ ID NO: 127, | antisense strand: | SEQ ID NO: 95; |
| sense strand: | SEQ ID NO: 128, | antisense strand: | SEQ ID NO: 96; |
| sense strand: | SEQ ID NO: 129, | antisense strand: | SEQ ID NO: 97; |
| sense strand: | SEQ ID NO: 130, | antisense strand: | SEQ ID NO: 98; and |
| sense strand: | SEQ ID NO: 131, | antisense strand: | SEQ ID NO: 99. |

In some embodiments, the dsRNA is further conjugated to a ligand moiety comprising N-acetylgalactosamine, and preferably the 3' end of the sense strand is conjugated to the ligand moiety.

In some embodiments, the ligand has the following structure:

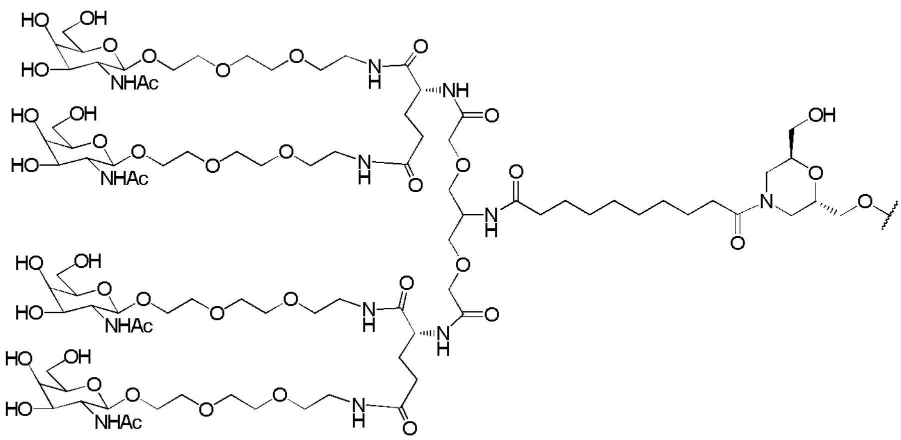

,

wherein

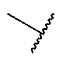

represents the point of attachment to the sense strand of the dsRNA via a phosphate group or a phosphorothioate group.

In some preferred embodiments, the dsRNA comprises any of the paired of sense strand sequences and antisense strand sequences selected from:

| | | | |
|---|---|---|---|
| sense strand: | SEQ ID NO: 60, | antisense strand: | SEQ ID NO: 81; |
| sense strand: | SEQ ID NO: 61, | antisense strand: | SEQ ID NO: 82; |
| sense strand: | SEQ ID NO: 62, | antisense strand: | SEQ ID NO: 83; |

(continued)

| | | | |
|---|---|---|---|
| sense strand: | SEQ ID NO: 63, | antisense strand: | SEQ ID NO: 84; |
| sense strand: | SEQ ID NO: 64, | antisense strand: | SEQ ID NO: 85; |
| sense strand: | SEQ ID NO: 65, | antisense strand: | SEQ ID NO: 86; |
| sense strand: | SEQ ID NO: 66, | antisense strand: | SEQ ID NO: 87; |
| sense strand: | SEQ ID NO: 67, | antisense strand: | SEQ ID NO: 88; |
| sense strand: | SEQ ID NO: 68, | antisense strand: | SEQ ID NO: 89; |
| sense strand: | SEQ ID NO: 69, | antisense strand: | SEQ ID NO: 90; |
| sense strand: | SEQ ID NO: 70, | antisense strand: | SEQ ID NO: 91; |
| sense strand: | SEQ ID NO: 71, | antisense strand: | SEQ ID NO: 92; |
| sense strand: | SEQ ID NO: 72, | antisense strand: | SEQ ID NO: 93; |
| sense strand: | SEQ ID NO: 73, | antisense strand: | SEQ ID NO: 94; |
| sense strand: | SEQ ID NO: 74, | antisense strand: | SEQ ID NO: 95; |
| sense strand: | SEQ ID NO: 75, | antisense strand: | SEQ ID NO: 96; |
| sense strand: | SEQ ID NO: 76, | antisense strand: | SEQ ID NO: 97; |
| sense strand: | SEQ ID NO: 77, | antisense strand: | SEQ ID NO: 98; |
| sense strand: | SEQ ID NO: 78, | antisense strand: | SEQ ID NO: 99; |
| sense strand: | SEQ ID NO: 72, | antisense strand: | SEQ ID NO: 100; |
| sense strand: | SEQ ID NO: 72, | antisense strand: | SEQ ID NO: 101; |
| sense strand: | SEQ ID NO: 72, | antisense strand: | SEQ ID NO: 102; |
| sense strand: | SEQ ID NO: 72, | antisense strand: | SEQ ID NO: 103; |
| sense strand: | SEQ ID NO: 79, | antisense strand: | SEQ ID NO: 104; and |
| sense strand: | SEQ ID NO: 79, | antisense strand: | SEQ ID NO: 105. |

In a second aspect, the disclosure provides a vector comprising a nucleotide sequence encoding the dsRNA of the disclosure.

In a third aspect, the disclosure provides a cell comprising the dsRNA or vector of the disclosure.

In a fourth aspect, the disclosure provides a pharmaceutical composition comprising the dsRNA, vector or cell of the disclosure, and optionally a pharmaceutically acceptable carrier or excipient.

In a fifth aspect, the disclosure provides a kit comprising the dsRNA, vector or cell of the disclosure.

In a sixth aspect, the disclosure provides a method of reducing angiotensinogen (AGT) in a subject, comprising a step of administering to the subject the dsRNA, vector, cell, or pharmaceutical composition of the disclosure. The present disclosure also provides a method of treating a disease or disorder benefiting from reduced expression of angiotensinogen (AGT) in a subject, comprising a step of administering to the subject the dsRNA, vector, cell, or pharmaceutical composition of the present disclosure. The disclosure also provides a method of preventing at least one symptom in a subject suffering from a disease or disorder benefiting from reduced expression of angiotensinogen (AGT), comprising a step of administering to the subject the dsRNA, vector, cell, or pharmaceutical composition of the disclosure.

In some embodiments, the disease or disorder benefiting from reduced expression of angiotensinogen (AGT) is an AGT-mediated or AGT-related disease.

In some embodiments, the AGT-mediated or AGT-related disease is selected from the group consisting of: hypertension, ocular hypertension, glaucoma, hypertensive heart disease, hypertensive nephropathy, atherosclerosis, arteriosclerosis, vasculopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, heart failure, myocardial infarction, angina pectoris, stroke, nephropathy, renal failure, systemic sclerosis, intrauterine growth restriction (IUGR), fetal growth restriction, obesity, hepatic steatosis/fatty liver disease, non-alcoholic steatohepatitis (NASH), non-alcoholic fatty liver disease (NAFLD); Glucose intolerance, type 2 diabetes (non-insulin-dependent diabetes ), and metabolic syndrome. In some embodiments, the hypertension is selected from the group consisting of borderline hypertension, essential hypertension, secondary hypertension, isolated systolic or diastolic hypertension, pregnancy-related hypertension, diabetic hypertension, resistant hypertension, refractory hypertension, paroxysmal hypertension, renovascular hypertension, Goldblatt hypertension, pulmonary hypertension, portal hypertension, systemic venous hypertension, systolic hypertension, and unstable hypertension.

In some embodiments, the method of treating a disease or disorder benefiting from reduced expression of angiotensinogen (AGT) in a subject, the method of preventing at least one symptom in a patient suffering from a disease or disorder benefiting from reduced expression of angiotensinogen (AGT), or the method of reducing angiotensinogen (AGT) in a subject provided in the present disclosure comprises subcutaneously, topically, or intravenously administering the

dsRNA, vector, cell, or pharmaceutical composition to the subject.

In some embodiments, the subject is a human patient.

## DESCRIPTION OF THE DRAWINGS

FIG. 1 shows the effect of test compounds on reducing serum AGT levels in cynomolgus monkeys.

FIG. 2 shows the effect of test compounds on reducing systolic blood pressure in cynomolgus monkeys.

FIG. 3 shows the effect of test compounds on reducing diastolic blood pressure in cynomolgus monkeys.

FIG. 4 shows the effect of test compounds on reducing mean arterial pressure in cynomolgus monkeys.

## DETAILED DESCRIPTION

The following describes the embodiments of the present disclosure through specific examples. Those skilled in the art can easily understand other advantages and effects of the present disclosure from the disclosure of the present specification. The present disclosure can also be implemented or applied through other different specific embodiments. Various details in this specification can also be modified or changed in various ways based on different viewpoints and applications without departing from the spirit of the present disclosure.

It should be understood that the protection scope of the present disclosure is not limited to the following specific embodiments; it should also be understood that the terms used in the embodiments of the present disclosure are for describing specific embodiments, not for limiting the protection scope of the present disclosure.

In the specification and claims of the present disclosure, the singular forms "a", "an", and "the" include plural referents unless the context clearly dictates otherwise.

When the embodiments give numerical ranges, it should be understood that, unless otherwise stated in the present disclosure, both endpoints of each numerical range and any value between the two endpoints can be selected. Unless defined otherwise, all technical and scientific terms used herein have the same meaning as commonly understood by one of ordinary skill in the art. In addition to the specific methods, equipment, and materials used in the embodiments, those skilled in the art can also use methods, equipment, and materials described in the embodiments of the present disclosure based on their understanding of the prior art and the description of the present disclosure. Any methods, equipment, and materials that are similar or equivalent to the prior art to implement the present disclosure shall fall within the protection scope of the present disclosure. Embodiments of the present disclosure are described in greater detail below.

### Definition

A "double-stranded region" refers herein to a region comprising two nucleic acid strands that are antiparallel and complementary or substantially complementary to each other.

As used herein, the term "double-stranded RNA" or "dsRNA" refers to a ribonucleic acid molecule or a complex of ribonucleic acid molecules comprising a double-stranded region as defined above. The two portions forming the double-stranded region may be two different moieties of a larger RNA molecule, or they may be separate RNA molecules.

When the two portions are separate RNA molecules, the dsRNA herein is referred to as a Small interfering RNA or a short interfering RNA, abbreviated as siRNA.

When the two portions are two different moieties of a larger molecule, that is, when the 3' end of one moiety is connected to the 5' end of the other moiety by one or more uninterrupted nucleotides therebetween to form a double-stranded region, the uninterrupted nucleotide(s) used for this connection is(are) referred to as a "hairpin loop". When the two moieties are covalently linked by means other than a hairpin loop to form a double-stranded region, the linking structure is referred to as a "linker". Such a dsRNA, when introduced into a cell, may be cleaved by an endoribonuclease called Dicer enzyme within the cell into siRNA.

The term "siRNA" herein is a class of dsRNA molecule comprising a sense strand and an antisense strand, which can mediate the silencing of target RNA (e.g., mRNA, e.g., transcript of a protein-encoding gene) complementary or substantially complementary to the antisense strand. A siRNA is generally double-stranded, including an antisense strand complementary to the target RNA thereof and a sense strand complementary or substantially complementary to this antisense strand. For convenience, such an mRNA is also referred to herein as an mRNA to be silenced, and such a gene is also called a target gene. Typically, the RNA to be silenced is an endogenous gene or a pathogen gene. In addition, RNAs

other than mRNA (for example, tRNA) and viral RNA may also be targeted.

As used herein, the term "antisense strand" refers to a strand in a dsRNA, in particular, an siRNA, wherein said strand contains a region that is completely or substantially complementary to the target sequence thereof.

As used herein, the term "complementary region" refers to a region on an antisense strand that is completely or substantially complementary to the target mRNA sequence thereof. In cases where a complementary region is incompletely complementary to the target sequence thereof, a mismatch may be located in an internal or terminal region of the molecule. Typically, the most tolerated mismatch is located in a terminal region, e.g., within 5, 4, 3, 2 or 1 nucleotide at the 5' and/or 3' end. The portion of an antisense strand most sensitive to a mismatch is called the "seed region". For example, in a siRNA containing a strand of 19 nt, position 19 (from the 5' to the 3') may tolerate some mismatches.

As used herein, the term "complementary" refers to the ability of a first polynucleotide to hybridize with a second polynucleotide under certain conditions, such as stringent conditions. For example, stringent conditions may include 400 mM NaCl, 40 mM PIPES, pH 6.4, 1 mM EDTA, and 50°C or 70°C for 12-16 hours.

As used herein, with respect to fulfilling the above required capabilities related to the hybridization ability thereof, the "complementary" sequences may also include or be entirely composed of non-Watson-Crick base pairs and/or base pairs formed from non-natural as well as modified nucleotides. Such non-Watson-Crick base pairs include, but are not limited to, G:U wobble base pairing or Hoogsteen base pairing.

As used herein, a polynucleotide that is "at least partially complementary" or "substantially complementary" to a messenger RNA (mRNA) refers to a polynucleotide that is substantially complementary to a contiguous portion of an mRNA of interest (e.g., an mRNA encoding AGT). For example, a polynucleotide is at least partially complementary to an mRNA encoding AGT, when the sequence thereof is substantially complementary to an uninterrupted portion of the AGT mRNA.

The terms "complementary," "completely complementary," and "substantially complementary" as used herein may be used with respect to base pairing between the sense strand and the antisense strand of a dsRNA, in particular, an siRNA, or between the antisense strand of a dsRNA, in particular, an siRNA, and a target sequence.

As used herein, the term "sense strand" refers to one strand of an siRNA that comprises a region substantially complementary to the region of an antisense strand as defined herein.

A "nucleoside" is a compound comprising two components: one is a purine base or a pyrimidine base, and the other is a ribose or a deoxyribose. A "nucleotide" is a compound comprising three components: one is a purine base or a pyrimidine base, another is a ribose or deoxyribose, and the third is a phosphoric acid. An "oligonucleotide" refers to, for example, a nucleic acid molecule (RNA or DNA) having a length of less than 100, 200, 300, or 400 nucleotides.

The term "base" is a fundamental building block of nucleosides, nucleotides, and nucleic acids; also referred to as "nitrogenous base," as it always contains nitrogen. Unless otherwise specified, the capital letters herein, i.e., A, U, T, G, and C, denote the bases of nucleotides, representing adenine, uracil, thymine, guanine and cytosine, respectively.

As used herein, the term "nucleotide overhang" refers to at least one unpaired nucleotide that protrudes from the double-stranded region of the siRNA. Nucleotide overhangs exist, for example, when the 3'-end of one strand of the siRNA extends beyond the 5'-end of the other strand, or vice versa. The siRNA may comprise an overhang having at least one nucleotide, an overhang having at least two nucleotides, an overhang having at least three nucleotides, an overhang having at least four nucleotides, or an overhang having at least five nucleotides or more. A nucleotide overhang may comprise or consist of a nucleotide/modified nucleotide (including a deoxynucleotide/nucleoside). One or more overhangs can be on the sense strand or the antisense strand, or any combination thereof. An overhang having one or more nucleotides may be present at the 5'-end, 3'-end, or both ends of the antisense or sense strand of the siRNA.

"Blunt end" means that there are no unpaired nucleotides, i.e., no nucleotide overhang at that end of the double-stranded siRNA. A "blunt-ended siRNA" is a siRNA that is double-stranded throughout its length, i.e., there is no nucleotide overhang at either end of the molecule.

Substantially all nucleotides of the dsRNA, in particular, the siRNA, of the present disclosure are modified. For example, substantially all nucleotides of the sense strand are modified nucleotides, or substantially all nucleotides of the antisense strand are modified nucleotides, or substantially all nucleotides of both the sense and antisense strands are modified nucleotides. In other embodiments of the present disclosure, all nucleotides of the dsRNA, in particular, the siRNA, of the present disclosure are modified nucleotides. For example, all nucleotides of the sense strand are modified nucleotides, or all nucleotides of the antisense strand are modified nucleotides, or all nucleotides of both the sense and antisense strands are modified nucleotides. As used herein, "substantially all nucleotides are modified" refers to that the majority, but not necessarily all, the nucleotides of the dsRNA, in particular, the siRNA, of the present disclosure are modified and may include no more than 5, 4, 3, 2, or 1 unmodified nucleotide(s).

A "modified nucleotide" herein include, but are not limited to, a 2'-O-alkyl modified nucleotide (e.g., a 2'-O-methyl modified nucleotide, or a 2'-methoxyethyl modified nucleotide), a 2'-fluoro modified nucleotide, a 2'-deoxy modified nucleotide, an SCP modification, an inosine ribonucleotide, an abasic nucleotide, an inverted abasic deoxyribonucleotide, a nucleotide comprising a phosphorothioate group, a vinylphosphate modified nucleotide, a locked nucleotide, an unlocked nucleotide, a 2'-amino modified nucleotide, a 2'-C-alkyl modified nucleotide, a 2'-O-allyl modified nucleotide,

a morpholino nucleotide, a phosphoramidate, a nucleotide comprising a non-natural base, a terminal nucleotide linked to a cholesteryl derivative or a dodecanoic acid bisdecylamide group, a deoxyribonucleotide, a 3'-terminal deoxythymine (dT) nucleotide, a conformationally restricted nucleotide, a constrained ethyl nucleotide, a 2'-hydroxy modified nucleotide, a nucleotide comprising methylphosphonate group, a nucleotide comprising 5'-phosphate, a nucleotide comprising 5'-phosphate mimic, a glycol modified nucleotide (GNA), a phosphorothioate internucleotide linkage modification and a 2-O-(N-methylacetamide) modified nucleotide, and the like.

For example, a "2'-fluoro modified nucleotide" refers to a nucleotide in which the hydroxyl at the 2' position of the ribosyl in a nucleotide is replaced with a fluorine atom. A "2'-O-methyl-modified nucleotide" refers to a nucleotide in which the 2'-hydroxyl of the ribosyl group is replaced with a methoxyl.

A "nucleotide comprising a phosphorothioate group" refers to a nucleotide in which one or more oxygen atoms on the phosphate in a nucleotide are replaced with a sulfur atom. A "modification of a phosphorothioate internucleotide linkage" refers to a modification in which two adjacent nucleotides are linked by a phosphorothioate.

In some embodiments, the sense strand of the dsRNA, in particular, the siRNA, of the present disclosure has a modification of 1 or 2 phosphorothioate internucleotide linkages at positions 1 to 5 (numbered from the 5' end) and/or has a modification of 1 or 2 phosphorothioate internucleotide linkages at positions 1 to 5 (numbered from the 3' end), and/or the antisense strand of the dsRNA, in particular, the siRNA of the present disclosure has a modification of 1 or 2 phosphorothioate internucleotide linkages at positions 1 to 5 (numbered from the 5' end) and/or has a modification of 1 or 2 phosphorothioate internucleotide linkages at positions 1 to 5 (numbered from the 3' end).

In some embodiments, the sense strand of the dsRNA, in particular, the siRNA, of the present disclosure has a modification of 1 or 2 phosphorothioate internucleotide linkages at positions 1 to 4 (numbered from the 5' end) and/or has a modification of 1 or 2 phosphorothioate internucleotide linkages at positions 1 to 4 (numbered from the 3' end), and/or the antisense strand of the dsRNA, in particular, the siRNA of the present disclosure has a modification of 1 or 2 phosphorothioate internucleotide linkages at positions 1 to 4 (numbered from the 5' end) and/or has a modification of 1 or 2 phosphorothioate internucleotide linkages at positions 1 to 4 (numbered from the 3' end).

In some embodiments, the sense strand of the dsRNA, in particular, the siRNA, of the present disclosure has a modification of 1 or 2 phosphorothioate internucleotide linkages at positions 1 to 3 (numbered from the 5' end) and/or has a modification of 1 or 2 phosphorothioate internucleotide linkages at positions 1 to 3 (numbered from the 3' end), and/or the antisense strand of the dsRNA, in particular, the siRNA of the present disclosure has a modification of 1 or 2 phosphorothioate internucleotide linkages at positions 1 to 3 (numbered from the 5' end) and/or has a modification of 1 or 2 phosphorothioate internucleotide linkages at positions 1 to 3 (numbered from the 3' end).

In some embodiments, the sense strand of the dsRNA, in particular, the siRNA, of the present disclosure has a modification of 1 or 2 phosphorothioate internucleotide linkages at positions 1 and 2 (numbered from the 5' end) and/or has a modification of 1 or 2 phosphorothioate internucleotide linkages at positions 1 and 2 (numbered from the 3' end), and/or the antisense strand of the dsRNA, in particular, the siRNA of the present disclosure has a modification of 1 or 2 phosphorothioate internucleotide linkages at positions 1 to 2 (numbered from the 5' end) and/or has a modification of 1 or 2 phosphorothioate internucleotide linkages at positions 1 to 2 (numbered from the 3' end).

In some embodiments, the sense strand of the dsRNA, in particular, the siRNA, of the present disclosure has a modification of 1 or 2 phosphorothioate internucleotide linkages at positions 1 and 2 (numbered from the 5' end) and/or has a modification of 1 or 2 phosphorothioate internucleotide linkages at positions 1 and 2 (numbered from the 3' end), and/or the antisense strand of the dsRNA, in particular, the siRNA of the present disclosure has a modification of 1 or 2 phosphorothioate internucleotide linkages at positions 1 to 2 (numbered from the 5' end) and/or has a modification of 1 or 2 phosphorothioate internucleotide linkages at positions 1 to 2 (numbered from the 3' end), wherein the 3' end of the sense strand is linked to a ligand moiety and the nucleotide at the 3' end of the sense strand is linked to the ligand moiety by a phosphate linkage.

In some embodiments, the sense strand of the dsRNA, in particular, the siRNA, of the present disclosure has a modification of 1 or 2 phosphorothioate internucleotide linkages at positions 1 and 2 (numbered from the 5' end) and/or has a modification of 1 or 2 phosphorothioate internucleotide linkages at positions 1 and 2 (numbered from the 3' end), and/or the antisense strand of the dsRNA, in particular, the siRNA of the present disclosure has a modification of 1 or 2 phosphorothioate internucleotide linkages at positions 1 to 2 (numbered from the 5' end) and/or has a modification of 1 or 2 phosphorothioate internucleotide linkages at positions 1 to 2 (numbered from the 3' end), wherein the 3' end of the sense strand is linked to a ligand moiety and the nucleotide at the 3' end of the sense strand is linked to the ligand moiety by a phosphorothioate linkage.

As used herein, the term "ligand moiety" refers to a chemical component conjugated with a dsRNA, in particular, a siRNA, wherein the moiety is capable of altering the distribution, targeting, or lifespan of the dsRNA, in particular, the siRNA. In some embodiments, the ligand moiety offers enhanced affinity for selected targets, such as molecules, cells or cell types, and compartments (e.g., cellular or organ compartments, tissues, organs, or regions of the body), compared to for example a siRNA without such a ligand moiety. In some embodiments, the ligand moiety targets the asialoglycoprotein receptor (ASGPR) on hepatocytes. Binding of the ligand moiety to ASGPR mediates internalization via clathrin-coated

vesicles. Maturation of endosomes leads to a decrease in the pH of lysosomes, which promotes the dissociation of the ligand-receptor complex, thereby releasing the dsRNA, in particular, the siRNA. Conjugation of the ligand moiety targeting the asialoglycoprotein receptor (ASGPR) on hepatocytes leads to efficacy and stability of the dsRNA, in particular, the siRNA, in vivo or intracellularly. This facilitates subcutaneous administration of the dsRNA, in particular, the siRNA.

As used herein, the term "inhibition" is used interchangeably with "reduction," "silencing," "downregulation," and other similar terms and includes any level of inhibition.

The phrase "inhibiting the expression of angiotensinogen (AGT)" refers to inhibiting the expression of any *AGT* gene as well as variants or mutants of the *AGT* gene. Thus, the *AGT* gene may be a wild-type *AGT* gene, a mutant *AGT* gene, or a transgenic *AGT* gene in the case of a genetically manipulated cell, cell population, or organism.

"Inhibition of *AGT* gene expression" includes any level of inhibition of the *AGT* gene, such as at least partial inhibition of *AGT* gene expression. *AGT* gene expression can be assessed based on the level or change in the level of any variable associated with *AGT* gene expression, such as *AGT* mRNA level or AGT protein level. This level may be assessed in a single cell or population of cells (including, for example, samples derived from a subject).

Inhibition can be assessed by a reduction in absolute or relative levels of one or more variables associated with AGT expression compared to control levels. The control level can be any type of control level utilized in the art, such as a pre-dose baseline level or a level determined from a similar untreated or control (e.g., a buffer-only control or an inert control)-treated subject, cell, or sample.

A "hydroxyl protecting group" refers to a group that can prevent a hydroxyl from undergoing chemical reactions and can be removed under specific conditions to restore the hydroxyl. The hydroxyprotecting groups mainly include silane-type, acyl-type, or ether-type protecting groups, preferably the following: trimethylsilyl (TMS), triethylsilyl (TES), dimethylisopropylsilyl (DMIPS), diethylisopropylsilyl (DEIPS), tert-butyldimethylsilyl (TBDMS), tert-butyldiphenylsilyl (TBDPS), triisopropylsilyl (TIPS), acetyl (Ac), chloroacetyl, dichloroacetyl, trichloroacetyl, trifluoroacetyl (TFA), benzoyl, p-methoxybenzoyl, 9-fluorenylmethoxycarbonyl (Fmoc), allyloxycarbonyl (Alloc), 2,2,2-trichloroethoxycarbonyl (Troc), benzyloxycarbonyl (Cbz), tert-butoxycarbonyl (Boc), benzyl (Bn), p-methoxybenzyl (PMB), allyl, triphenylmethyl (Tr), di-p-methoxytrityl (DMTr), methoxymethyl (MOM), benzyloxymethyl (BOM), 2,2,2-trichloroethoxymethyl, 2-methoxyethoxymethyl (MEM), methylthiomethyl (MTM), p-methoxybenzyloxymethyl (PMBM).

"Halo-" or "halogen" refers to (substitution by) fluorine (F), chlorine (Cl), bromine (Br), and iodine (I).

"$C_{1-6}$ haloalkyl" refers to the aforementioned "$C_{1-6}$ alkyl", with one or more halogen groups. In some embodiments, a $C_{1-4}$ haloalkyl is particularly preferred, and a $C_{1-2}$ haloalkyl is even more preferred. Exemplary haloalkyls include, but are not limited to: $-CF_3$, $-CH_2F$, $-CHF_2$, $-CHFCH_2F$, $-CH_2CHF_2$, $-CF_2CF_3$, $-CCl_3$, $-CH_2Cl$, $-CHCl_2$, and 2,2,2-trifluoro-1,1-dimethyl-ethyl. The haloalkyls may be substituted at any substitutable connection site, for example, 1 to 5 substituent(s), 1 to 3 substituent(s), or 1 substituent.

"$C_{1-6}$ alkylene" refers to a divalent group formed by removing another hydrogen of $C_{1-6}$ alkyl and can be substituted or unsubstituted. In some embodiments, $C_{1-4}$ alkylene, $C_{2-4}$ alkylene, and $C_{1-2}$ alkylene are preferred. The unsubstituted alkylenes include, but are not limited to: methylene group ($-CH_2-$), ethylene group ($-CH_2CH_2-$), propylene group ($-CH_2CH_2CH_2-$), butylene group ($-CH_2CH_2CH_2CH_2-$), pentylene group ($-CH_2CH_2CH_2CH_2CH_2-$), and hexylene group ($-CH_2CH_2CH_2CH_2CH_2CH_2-$). Examples of said substituted alkylenes, such as a alkylene substituted by one or more alkyl (methyl) groups, include but are not limited to: substituted methylene ($-CH(CH_3)-$, and $-C(CH_3)_2-$), substituted ethylene ($-CH(CH_3)CH_2-$, $-CH_2CH(CH_3)-$, $-C(CH_3)_2CH_2-$, and $-CH_2C(CH_3)_2-$), substituted propylene ($-CH(CH_3)CH_2CH_2-$, $-CH_2CH(CH_3)CH_2-$, $-CH_2CH_2CH(CH_3)-$, $-C(CH_3)_2CH_2CH_2-$, $-CH_2C(CH_3)_2CH_2-$, $-CH_2CH_2C(CH_3)_2-$), etc.

As used herein, the term "vector" refers to a nucleic acid molecule capable of amplifying or expressing another nucleic acid to which it is linked. A "vector" herein is generally a vector that can self-replicate in a host cell, which is preferably a multi-copy vector. In addition, vectors typically have markers such as antibiotic resistance genes for selecting transformants. Furthermore, the vector may have a promoter and/or a terminator for expression of an introduced gene. The vector may be, for example, a vector derived from a bacterial plasmid, a viral vector, a vector derived from a yeast plasmid, a vector derived from a bacteriophage, a cosmid, a phagemid, and the like.

As used herein, the terms "treat," "treatment," and the like refer to the administration of an agent or the performing of a procedure in order to achieve effects. These effects may be prophylactic in terms of complete or partial prevention of a disease or a symptom thereof, and/or may be therapeutic in terms of affecting partial or complete cure of a disease and/or a symptom thereof. As used herein, "treating" may include treating a disease or disorder (e.g., cancer) in a mammal, particularly a human, and comprises: (a) preventing the occurrence of a disease or the symptom thereof (e.g., including a disease that may be associated with or caused by a primary disease) in a subject who is susceptible to but has not been diagnosed with the disease; (b) inhibiting, i.e. preventing the progression of, a disease; (c) relieving, i.e. causing regression of, a disease. Treatment may refer to any reference to success in treating or ameliorating or preventing a cancer, including any objective or subjective parameter, such as elimination; remission; diminishing of a symptom, or making the disease condition more tolerable to the patient; slowing down the deterioration or decline; or debilitation or reduction of the worsening endpoint. Treatment or amelioration of a symptom is based on one or more objective or subjective parameters, including the results of the examination by a doctor. Accordingly, the term "treatment" includes administration of the

antibody or the composition or the conjugate disclosed herein to prevent or delay, alleviate or arrest, or inhibit the development of the symptom or condition associated with a disease (e.g., cancer). The term "therapeutic effect" refers to the reduction, elimination, or prevention of a disease, a symptom of the disease, or a side effect of the disease in a subject.

As used herein, the term "effective amount" refers to an amount sufficient to effect treatment of a disease when administered to a subject for treating the disease.

As used herein, the term "subject" refers to any mammalian subject for whom diagnosis, cure, or treatment is desired. A "mammal" for therapeutic purposes refers to any animal classified as a mammal, including humans, domestic and farm animals, as well as laboratory animals, zoo animals, sports animals, or pet animals, such as dogs, horses, cats, cows, sheep, goats, pigs, mice, rats, rabbits, guinea pigs, monkeys, and the like.

**I. dsRNA**

The present disclosure provides a double-stranded RNA (dsRNA) for inhibiting the expression of angiotensinogen (AGT) in a cell, comprising a sense strand and an antisense strand that form a double-stranded region, wherein the sense strand and the antisense strand are each independently 15-30 nucleotides in length, and the antisense strand comprises a nucleotide sequence of at least 15 contiguous nucleotides from the nucleotide sequence set forth in any one of SEQ ID Nos: 21-40.

In some embodiments, the double-stranded region formed by the sense strand and the antisense strand is completely complementary. In other embodiments, the double-stranded region formed by the sense strand and the antisense strand is substantially complementary and may contain 1, 2, 3, 4, or 5 non-complementary sites.

In some specific embodiments, the sense strand comprises a nucleotide sequence of at least 15 contiguous nucleotides from the nucleotide sequence set forth in any one of SEQ ID Nos: 1-20.

In some embodiments, the sense strand and the antisense strand are each independently 15-27 nucleotides in length, preferably 18-25 nucleotides, more preferably 19-21 nucleotides in length.

In some embodiments, the length of the double-stranded region is 15-25 nucleotide pairs, preferably 16-23 nucleotide pairs, more preferably 18-20 nucleotide pairs.

In some embodiments, the dsRNA of the disclosure is an siRNA. In other embodiments, a hairpin loop is formed between the sense strand and the antisense strand of the dsRNA of the disclosure.

One or both of the sense strand and the antisense strand comprise a 3' overhang and/or a 5' overhang having at least 1 nucleotide. In some embodiments, one or both of the sense strand and the antisense strand comprise(s) a 3' overhang and/or a 5' overhang having at least 1 nucleotide. In some specific embodiments, one or both of the sense strand and the antisense strand comprise a 3' overhang and/or a 5' overhang having 1 nucleotide. In some specific embodiments, one or both of the sense strand and the antisense strand comprise a 3' overhang and/or a 5' overhang having 2 nucleotides. In some specific embodiments, one or both of the sense strand and the antisense strand comprise a 3' overhang and/or a 5' overhang having 3 nucleotides. In some specific embodiments, one or both of the sense strand and the antisense strand comprise a 3' overhang and/or a 5' overhang having 4 nucleotides.

In some specific embodiments, the antisense strand comprises a 3' overhang and/or a 5' overhang having 1 nucleotide. In some embodiments, the antisense strand comprises a 3' overhang and/or a 5' overhang having 2 nucleotides. In some embodiments, the antisense strand comprises a 3' overhang and/or a 5' overhang having 3 nucleotides. In some embodiments, the antisense strand comprises a 3' overhang and/or a 5' overhang having 4 nucleotides. In some preferred embodiments, the antisense strand comprises a 3' overhang and/or a 5' overhang having at least 2 nucleotides.

Preferably, the antisense strand comprises a 3' overhang and/or a 5' overhang having 2 nucleotides.

In some embodiments, the sense strand and the antisense strand are of the same length.

In some embodiments, the full length of the sense strand is complementary to the full length of the antisense strand to form a double strand, i.e., having blunt ends.

In some other embodiments, the sense strand and the antisense strand are of the same length, and a portion of the sense strand is complementary to a portion of the antisense strand, that is, both the sense strand and the antisense strand have 5' overhangs. In some embodiments, the sense strand and the antisense strand are of different lengths. In a preferred embodiment, the 5' end of the antisense strand has an overhang of at least 1 nucleotide, more preferably 2 or 3 nucleotides.

The dsRNA of the present disclosure includes a dsRNA having a nucleotide overhang at one end (i.e., an agent having one overhang and one blunt end) or having nucleotide overhangs at both ends. For example, the 5'-end of the sense strand of a dsRNA comprises an overhang having one or more nucleotides and the 3'-end of the sense strand comprises an overhang having one or more nucleotides. For example, the 5'-end of the antisense strand of a dsRNA comprises an overhang having one or more nucleotides and the 3'-end of the antisense strand comprises an overhang having one or more nucleotides. For example, the 5'-end of the sense strand of a dsRNA comprises an overhang having one or more nucleotides and the 5'-end of the antisense strand comprises an overhang having one or more nucleotides.

For example, the 3'-end of the sense strand of a dsRNA comprises an overhang having one or more nucleotides and the 3'-end of the antisense strand comprises an overhang having one or more nucleotides. For example, the 5'-end of the sense strand of a dsRNA comprises an overhang having one or more nucleotides and the 3'-end of the sense strand comprises a blunt end. For example, the 3'-end of the sense strand of a dsRNA comprises an overhang having one or more nucleotides and the 3'-end of the sense strand comprises a blunt end. For example, the 5'-end of the antisense strand of a dsRNA comprises an overhang having one or more nucleotides and the 3'-end of the antisense strand comprises a blunt end. For example, the 3'-end of the antisense strand of a dsRNA comprises an overhang having one or more nucleotides and the 5'-end of the antisense strand comprises a blunt end.

In some preferred embodiments, the 3'-end of the antisense strand of the dsRNA of the disclosure comprises an overhang having one or more nucleotides and the 5'-end of the antisense strand comprises a blunt end. In some more preferred embodiments, the 3'-end of the antisense strand of the dsRNA of the disclosure comprises an overhang having 1, 2, 3 or 4 nucleotides and the 5'-end of the antisense strand comprises a blunt end. In some more preferred embodiments, the 3'-end of the antisense strand of the dsRNA of the disclosure comprises an overhang having 2 nucleotides and the 5'-end of the antisense strand comprises a blunt end.

In some embodiments, the antisense strand comprises a nucleotide sequence of at least 16 contiguous nucleotides, a nucleotide sequence of at least 17 contiguous nucleotides, a nucleotide sequence of at least 18 contiguous nucleotides, a nucleotide sequence of at least 19 contiguous nucleotides, or a nucleotide sequence of at least 20 contiguous nucleotides from the nucleotide sequence set forth in any one of SEQ ID Nos: 21-40, preferably the antisense strand comprises the nucleotide sequence set forth in any one of SEQ ID Nos: 21-40.

In some embodiments, the sense strand comprises a nucleotide sequence of at least 16 contiguous nucleotides, a nucleotide sequence of at least 17 contiguous nucleotides, or a nucleotide sequence of at least 18 contiguous nucleotides from any one of the nucleotide sequences set forth in SEQ ID Nos: 1-20, preferably the sense strand comprises the nucleotide sequence set forth in any one of SEQ ID Nos: 1-20.

In some embodiments, the dsRNA comprises any paired sense strand sequence and antisense strand sequence of the paired sense strand sequences and antisense strand sequences set forth in Table 3.

**II. Modification of Nucleotides**

In some embodiments, substantially all nucleotides of the sense strand and substantially all nucleotides of the antisense strand are modified nucleotides. In some embodiments, at least 80% of the nucleotides of the sense strand are modified nucleotides, and/or at least 80%, at least 85%, at least 90%, at least 92%, at least 95% of the nucleotides of the antisense strand are modified nucleotides. In some embodiments, at least 80% of the nucleotides of the antisense strand are modified nucleotides, and/or at least 80%, at least 85%, at least 90%, at least 92%, at least 95% of the nucleotides of the sense strand are modified nucleotides.

In some embodiments, all nucleotides of the sense strand are modified nucleotides and/or all nucleotides of the antisense strand are modified nucleotides.

The modification of the nucleotide of the present disclosure may be a modification on the phosphate group, the ribose group and/or the base group of the nucleotide.

In some specific embodiments, the sense strand and the antisense strand each independently comprise one or more nucleotide modifications selected from the group consisting of: 2'-O-alkyl-modified nucleotides (e.g., 2'-O-methyl-modified nucleotides), a 2'-fluoro modified nucleotide, a 2'-deoxy modified nucleotide, an inosine ribonucleotide, an abasic nucleotide, an inverted abasic deoxyribonucleotide, a nucleotide comprising a phosphorothioate group, a vinylphosphate modified nucleotide, a locked nucleotide, an unlocked nucleotide, a 2'-amino modified nucleotide, a 2'-C-alkyl modified nucleotide, a 2'-O-allyl modified nucleotide, a morpholino nucleotide, a phosphoramidate, a nucleotide comprising a non-natural base, a terminal nucleotide linked to a cholesteryl derivative or a dodecanoic acid bisdecylamide group, a deoxyribonucleotide, a 3'-terminal deoxythymine (dT) nucleotide, a conformationally restricted nucleotide, a constrained ethyl nucleotide, a 2'-hydroxy modified nucleotide, a nucleotide comprising methylphosphonate group, a nucleotide comprising 5'-phosphate, a nucleotide comprising 5'-phosphate mimic, a SCP modification, a glycol modified nucleotide (GNA), a phosphorothioate internucleotide linkage modification and a 2-O-(N-methylacetamide) modified nucleotide.

In some preferred embodiments, the sense strand and the antisense strand each independently comprise one or more nucleotide modifications selected from the group consisting of 2'-O-methyl-modifications, 2'-fluoro-modifications, SCP-modifications, and modifications of phosphorothioate internucleotide linkage.

In some preferred embodiments, the sense strand and/or the antisense strand comprises at least two 2'-fluoro-modified nucleotides. In some preferred embodiments, the sense strand and/or the antisense strand comprises at least 8 2'-O-methyl-modified nucleotides. In some preferred embodiments, the 3' and/or 5' ends of the sense strand and/or the antisense strand comprise 1-5 phosphorothioate internucleotide linkages, preferably 2-3 phosphorothioate internucleotide linkages. In some embodiments, the sense strand and/or antisense strand comprises an adenine deoxyribonucleotide, a thymine deoxyribonucleotide, a guanine deoxyribonucleotide, and/or a cytosine deoxyribonucleotide. In a more

preferred embodiment, the sense strand and/or antisense strand comprises a thymine deoxyribonucleotide. In a most preferred embodiment, the sense strand comprises a thymine deoxyribonucleotide.

In some embodiments, the antisense strand is 21 nucleotides in length and has

(i) 2'-O-methyl modified nucleotides at positions 1, 3, 5, 9, 11, 13, 15, 17, 19, and 21, and 2'-fluoro modified nucleotides at positions 2, 4, 6, 8, 10, 12, 14, 16, 18, and 20 (numbered from the 5' end); and/or

(ii) phosphorothioate internucleotide linkages between nucleotide positions 1 and 2, between nucleotide positions 2 and 3, between nucleotide positions 19 and 20, and between nucleotide positions 20 and 21 (numbered from the 5' end).

In some other embodiments, the antisense strand has a length of 21 nucleotides and has

(i) 2'-O-methyl modified nucleotides at positions 1, 3, 5, 9, 11, 13, 15, 17, 19, and 21, and 2'-fluoro modified nucleotides at positions 2, 4, 6, 8, 10, 12, 14, 16, 18, and 20 (numbered from the 5' end); and/or

(ii) phosphorothioate internucleotide linkages between nucleotide positions 1 and 2, between nucleotide positions 2 and 3, between nucleotide positions 19 and 20, and between nucleotide positions 20 and 21 (numbered from the 5' end).

In some other embodiments, the antisense strand has a length of 21 nucleotides and has

(i) 2'-O-methyl modified nucleotides at positions 1, 3, 5, 9, 11, 13, 15, 17, 19, 20, and 21, and 2'-fluoro modified nucleotides at positions 2, 4, 6, 8, 10, 12, 14, 16, and 18 (numbered from the 5' end); and/or

(ii) phosphorothioate internucleotide linkages between nucleotide positions 1 and 2, between nucleotide positions 2 and 3, between nucleotide positions 19 and 20, and between nucleotide positions 20 and 21 (numbered from the 5' end).

In some embodiments, the antisense strand has a length of 21 nucleotides and has

(i) 2'-O-methyl modified nucleotides at positions 3, 4, 6, 8, 9, 10, 11, 13, 15, 16, 17, 18, 19, 20, and 21 (numbered from the 5' end);
(ii) a 2'-fluoro modified nucleotide at position 14 (numbered from the 5' end);
(iii) 2'-deoxy modifications at positions 2, 5, 7, and 12 (numbered from the 5' end);
(iv) an SCP modification at position 1 (numbered from the 5' end); and/or
(v) phosphorothioate internucleotide linkages between nucleotide positions 1 and 2, between nucleotide positions 2 and 3, between nucleotide positions 19 and 20, and between nucleotide positions 20 and 21 (numbered from the 5' end).

In some embodiments, the antisense strand has a length of 21 nucleotides and has

(i) 2'-O-methyl modified nucleotides at positions 3, 5, 9, 11, 13, 15, 17, 19, 20, and 21 (numbered from the 5' end);
(ii) 2'-fluoro modified nucleotides at positions 2, 4, 6, 8, 10, 12, 14, 16, and 18 (numbered from the 5' end);
(iii) a GNA modification at position 7 (numbered from the 5' end);
(iv) an SCP modification at position 1 (numbered from the 5' end); and/or
(v) phosphorothioate internucleotide linkages between nucleotide positions 1 and 2, between nucleotide positions 2 and 3, between nucleotide positions 19 and 20, and between nucleotide positions 20 and 21 (numbered from the 5' end).

In some embodiments, the sense strand has a length of 19 nucleotides and has:

(i) 2'-O-methyl modified nucleotides at positions 1 to 6, and 10 to 19, and 2'-fluoro modified nucleotides at positions 7-9 (numbered from the 5' end); and/or
(ii) phosphorothioate internucleotide linkages between nucleotide positions 1 and 2, between nucleotide positions 2 and 3, and between nucleotide positions 18 and 19 (numbered from the 5' end).

In some embodiments, the antisense strand comprises a sequence selected from any one of the sequences set

forth in SEQ ID NOs: 81-105.

In some embodiments, the sense strand comprises a sequence selected from any one of the sequences set forth in SEQ ID NOs: 41-59. In some preferred embodiments, the dsRNA comprises any of the paired modified sense strand sequences and modified antisense strand sequences set forth in Table 6.

In some embodiments, the 3' end of the sense strand is modified to conjugate a ligand. In some specific embodiments, the sense strand comprises a sequence selected from any one set forth in SEQ ID NOs: 113-131.

In some preferred embodiments, the dsRNA comprises any of paired modified sense strand sequences and modified antisense strand sequences selected from:

| | | | |
|---|---|---|---|
| sense strand: | SEQ ID NO: 113, | antisense strand: | SEQ ID NO: 81; |
| sense strand: | SEQ ID NO: 114, | antisense strand: | SEQ ID NO: 82; |
| sense strand: | SEQ ID NO: 115, | antisense strand: | SEQ ID NO: 83; |
| sense strand: | SEQ ID NO: 116, | antisense strand: | SEQ ID NO: 84; |
| sense strand: | SEQ ID NO: 117, | antisense strand: | SEQ ID NO: 85; |
| sense strand: | SEQ ID NO: 118, | antisense strand: | SEQ ID NO: 86; |
| sense strand: | SEQ ID NO: 119, | antisense strand: | SEQ ID NO: 87; |
| sense strand: | SEQ ID NO: 120, | antisense strand: | SEQ ID NO: 88; |
| sense strand: | SEQ ID NO: 121, | antisense strand: | SEQ ID NO: 89; |
| sense strand: | SEQ ID NO: 122, | antisense strand: | SEQ ID NO: 90; |
| sense strand: | SEQ ID NO: 123, | antisense strand: | SEQ ID NO: 91; |
| sense strand: | SEQ ID NO: 124, | antisense strand: | SEQ ID NO: 92; |
| sense strand: | SEQ ID NO: 125, | antisense strand: | SEQ ID NO: 93; |
| sense strand: | SEQ ID NO: 126, | antisense strand: | SEQ ID NO: 94; |
| sense strand: | SEQ ID NO: 127, | antisense strand: | SEQ ID NO: 95; |
| sense strand: | SEQ ID NO: 128, | antisense strand: | SEQ ID NO: 96; |
| sense strand: | SEQ ID NO: 129, | antisense strand: | SEQ ID NO: 97; |
| sense strand: | SEQ ID NO: 130, | antisense strand: | SEQ ID NO: 98; and |
| sense strand: | SEQ ID NO: 131, | antisense strand: | SEQ ID NO: 99. |

### III. Ligand Moiety

The dsRNA of the disclosure is further conjugated to a ligand moiety comprising N-acetylgalactosamine. In a preferred embodiment, the sense strand of the dsRNA is conjugated to the ligand moiety. In some embodiments, the 3' end of the sense strand is conjugated to the ligand moiety. In other embodiments, the 5' end of the sense strand is conjugated to the ligand moiety.

In some embodiments, the ligand moiety comprises a conjugate group represented by formula (X'):

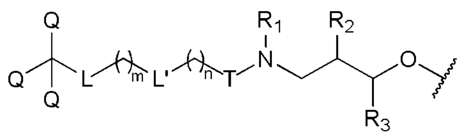

(X')

wherein

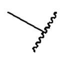

represents the point of attachment to the dsRNA;

Q is independently H,

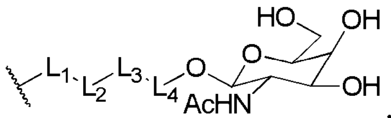

,

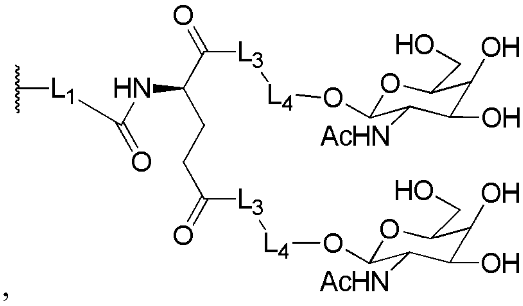

,

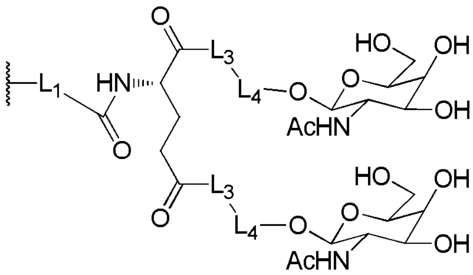

,

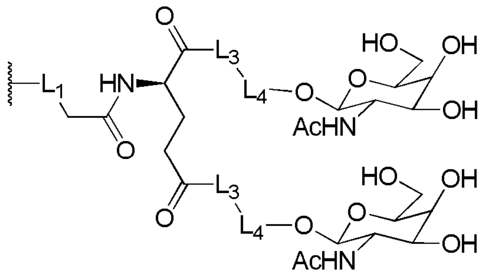

,

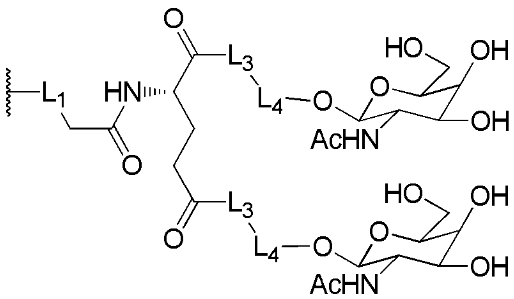

,

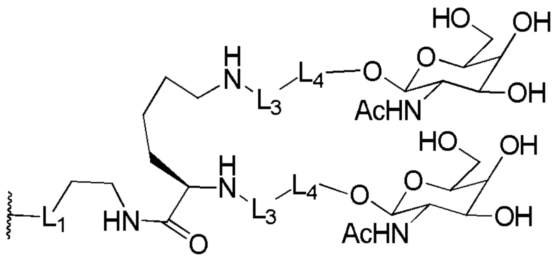

or

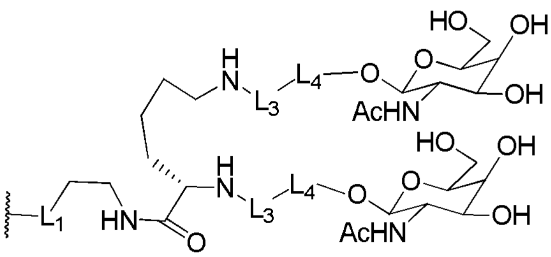

;

wherein $L_1$ is a chemical bond, $-CH_2-$, $-CH_2CH_2-$, -C(O)-, $-CH_2O-$, $-CH_2O-CH_2CH_2O-$ or - $NHC(O)-(CH_2NHC(O))_a-$;
$L_2$ is a chemical bond or $-CH_2CH_2C(O)-$;
$L_3$ is a chemical bond, $-(NHCH_2CH_2)_b-$, $-(NHCH_2CH_2CH_2)_b-$ or $-C(O)CH_2-$;
$L_4$ is $-(OCH_2CH_2)_c-$, $-(OCH_2CH_2CH_2)_c-$, $-(OCH_2CH_2CH_2CH_2)_c-$, $-(OCH_2CH_2CH_2CH_2CH_2)_c-$ or $-NHC(O)-(CH_2)_d-$;
wherein a = 0, 1, 2, or 3;
b = 1, 2, 3, 4 or 5;
c = 1, 2, 3, 4 or 5;
d = 1, 2, 3, 4, 5, 6, 7, or 8;
L is a chemical bond, $-CH_2O-$ or -NHC(O)-;
L' is a chemical bond, -C(O)NH-, -NHC(O)- or $-O(CH_2CH_2O)_e-$;
wherein e is 1, 2, 3, 4 or 5;
T is a chemical bond, $-CH_2-$, -C(O)-, -M-, $-CH_2-M-$ or -C(O)-M-;
wherein M is

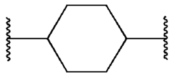, or 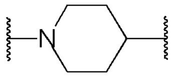;

$R_1$ and $R_2$ together form $-CH_2CH_2O-$ or $-CH_2CH(R)-O-$, and $R_3$ is H;
or $R_1$ and $R_3$ together form $-C_{1-2}$ alkylene-, and $R_2$ is H;
wherein R is-OR', $-CH_2OR'$ or $-CH_2CH_2OR'$, wherein R' is H, a hydroxyl protecting group, or a solid support, preferably wherein the hydroxyl protecting group is $-C(O)CH_2CH_2C(O)OH$ or 4,4'-dimethoxytrityl;
m = 0, 1, 2, 3, 4, 5, 6, 7, 8, 9 or 10;
n = 0, 1, 2, 3, 4, 5, 6, 7, 8, 9, or 10.

In some embodiments, the conjugating group is represented by Formula (I'):

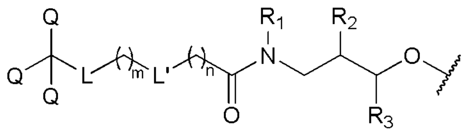

(I')

wherein

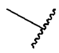

represents the point of attachment to the dsRNA;

Q is independently H,

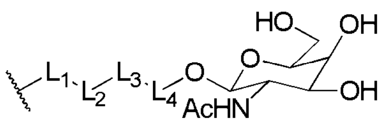,

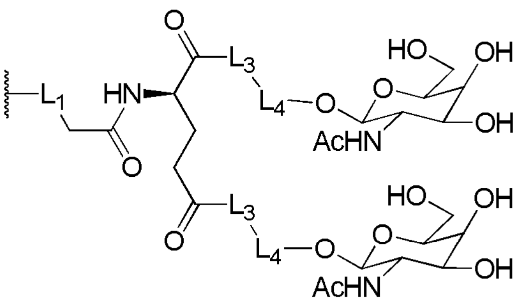,

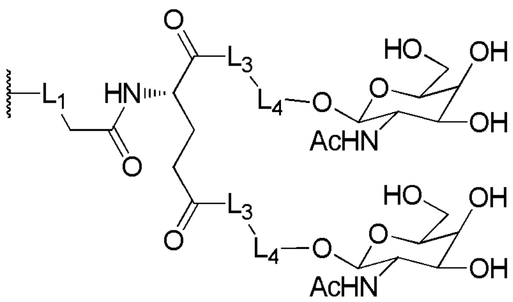,

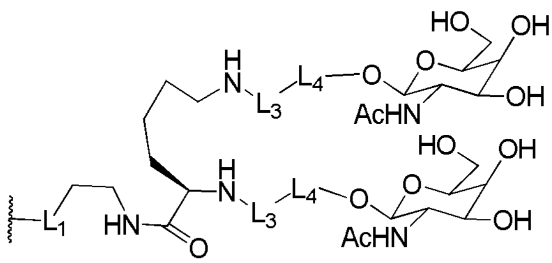

or

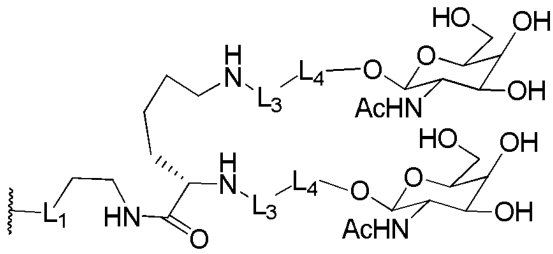

;

wherein $L_1$ is a chemical bond, $-CH_2-$, $-CH_2CH_2-$, -C(O)- , $-CH_2O-$, $-CH_2O-CH_2CH_2O-$ or - $NHC(O)-(CH_2NHC(O))_a-$;

$L_2$ is a chemical bond or $-CH_2CH_2C(O)-$;

$L_3$ is a chemical bond, $-(NHCH_2CH_2)_b-$, $-(NHCH_2CH_2CH_2)_b-$ or $-C(O)CH_2-$;

$L_4$ is $-(OCH_2CH_2)_c-$, $-(OCH_2CH_2CH_2)_c-$, $-(OCH_2CH_2CH_2CH_2)_c-$, $-(OCH_2CH_2CH_2CH_2CH_2)_c-$ or $-NHC(O)-(CH_2)_d-$;

wherein a = 0, 1, 2, or 3;

b = 1, 2, 3, 4 or 5;

c = 1, 2, 3, 4 or 5;

d = 1, 2, 3, 4, 5, 6, 7, or 8;

L is $-CH_2O-$ or -NHC(O)-;

L 'is a chemical bond, -C(O)NH- or -NHC(O)-;

$R_1$ and $R_2$ together form $-CH_2CH_2O-$ or $-CH_2CH(R)-O-$, and $R_3$ is H;

or R1 and R3 together form-C1-2 alkylene-, and R2 is H;

wherein R is -OR', $-CH_2OR'$ or $-CH_2CH_2OR'$, wherein R' is H, a hydroxyl protecting group, or a solid support, wherein the hydroxyl protecting group is preferably $-C(O)CH_2CH_2C(O)OH$ or 4,4'-dimethoxytrityl;

m = 0, 1, 2, 3, 4, 5, 6, 7, 8, 9 or 10;

n = 0, 1, 2, 3, 4, 5, 6, 7, 8, 9, or 10.

In some specific embodiments, wherein,

Q is independently H or

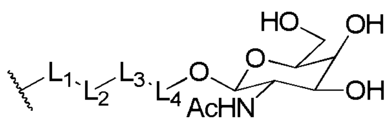

;

wherein $L_1$ is $-CH_2O-$ or $-NHC(O)-(CH_2NHC(O))_a-$;

$L_2$ is $-CH_2CH_2C(O)-$;

$L_3$ is $-(NHCH_2CH_2)_b-$ or $-(NHCH_2CH_2CH_2)_b-$;

$L_4$ is $-(OCH_2CH_2)_c-$ or $-NHC(O)-(CH_2)_d-$;

wherein a = 0, 1, 2, or 3;

b = 1, 2, 3, 4 or 5;

c = 1, 2, 3, 4 or 5;

d = 1, 2, 3, 4, 5, 6, 7, or 8;

L is $-CH_2O-$;

L' is a chemical bond;

$R_1$ and $R_2$ together form $-CH_2CH_2O-$ or $-CH_2CH(R)-O-$, and $R_3$ is H;

or $R_1$ and $R_3$ together form $-C_{1-2}$ alkylene-, and $R_2$ is H;

wherein R is -OR', $-CH_2OR'$ or $-CH_2CH_2OR'$, wherein R' is H, a hydroxyl protecting group, or a solid support, wherein the hydroxyl protecting group is preferably $-C(O)CH_2CH_2C(O)OH$ or 4,4' -dimethoxytrityl;

m = 0, 1, 2, 3, 4, 5, 6, 7, 8, 9 or 10;

n = 0, 1, 2, 3, 4, 5, 6, 7, 8, 9, or 10.

In some embodiments, the conjugating group is represented by Formula (I'-1), Formula (I'-2), or Formula (I'-3):

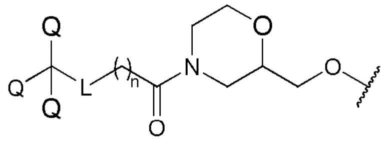

(I'-1),

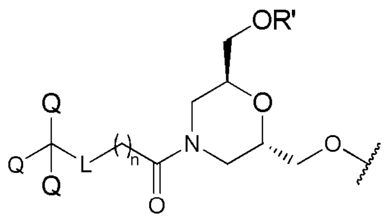

(I'-2)

or

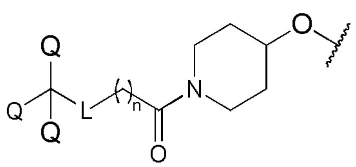

(I'-3)

wherein

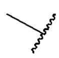

represents the point of attachment to the dsRNA;
Q is

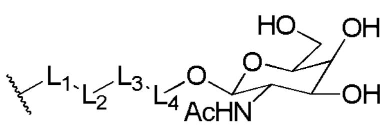

;

wherein $L_1$ is $-CH_2O-$ or $-NHC(O)-$;
$L_2$ is $-CH_2CH_2C(O)-$;
$L_3$ is $-(NHCH_2CH_2)_b-$ or $-(NHCH_2CH_2CH_2)_b-$;

$L_4$ is $-(OCH_2CH_2)_c-$ or $-NHC(O)-(CH_2)_d-$;
wherein b = 1, 2, 3, 4 or 5;
c = 1, 2, 3, 4 or 5;
d = 1, 2, 3, 4, 5, 6, 7, or 8;
L is $-CH_2O-$;
R' is H, a hydroxyl protecting group, or a solid support, preferably the hydroxyl protecting group is $-C(O)CH_2CH_2C(O)OH$ or 4,4'-dimethoxytrityl;
n = 0, 1, 2, 3, 4, 5, 6, 7, 8, 9, or 10.

In some specific embodiments, wherein,

Q is independently H,

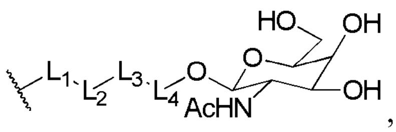
,
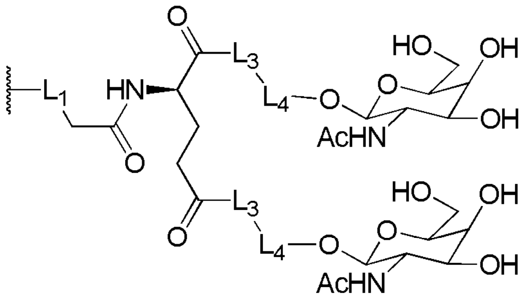
,
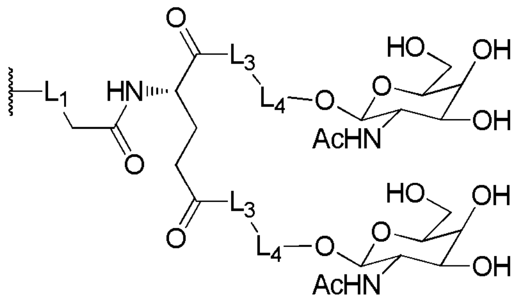
,
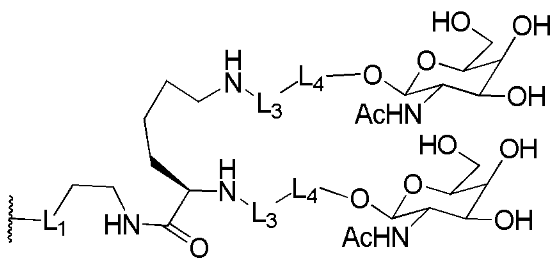

or

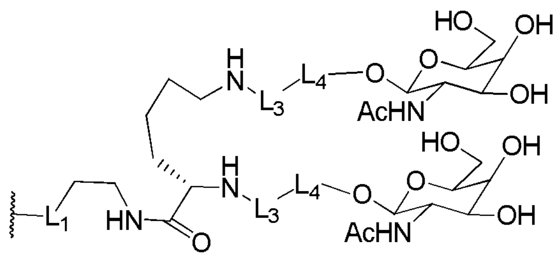
;

wherein $L_1$ is $-CH_2O-$, $-CH_2O-CH_2CH_2O-$ or $-NHC(O)-(CH_2NHC(O))_a-$;
$L_2$ is $-CH_2CH_2C(O)-$;
$L_3$ is $-(NHCH_2CH_2)_b-$, $-(NHCH_2CH_2CH_2)_b-$ or $-C(O)CH_2-$;
$L_4$ is $-(OCH_2CH_2)_c-$ or $-NHC(O)-(CH_2)_d-$;
wherein a = 0, 1, 2, or 3;
b = 1, 2, 3, 4 or 5;
c = 1, 2, 3, 4 or 5;
d = 1, 2, 3, 4, 5, 6, 7, or 8;
L is $-CH_2O-$ or $-NHC(O)-$;
L' is a chemical bond or $-C(O)NH-$;
$R_1$ and $R_2$ together form $-CH_2CH_2O-$ or $-CH_2CH(R)-O-$, and $R_3$ is H;

or $R_1$ and $R_3$ together form $-C_{1-2}$ alkylene-, and $R_2$ is H;
wherein R is -OR', $-CH_2OR'$ or $-CH_2CH_2OR'$, wherein R' is H, a hydroxyl protecting group, or a solid support, preferably the hydroxyl protecting group is $-C(O)CH_2CH_2C(O)OH$ or 4,4'-dimethoxytrityl;
m = 0, 1, 2, 3, 4, 5, 6, 7, 8, 9 or 10;
n = 0, 1, 2, 3, 4, 5, 6, 7, 8, 9, or 10.

In some embodiments, the conjugating group is represented by Formula (II'-1) or Formula (II'-2):

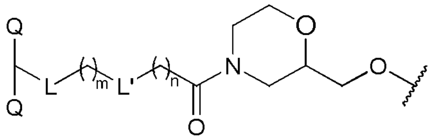 (II'-1) or 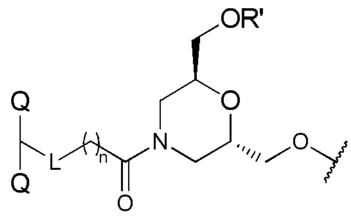 (II'-2)

wherein

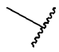

represents the point of point of attachmentto the dsRNA;

Q is independently

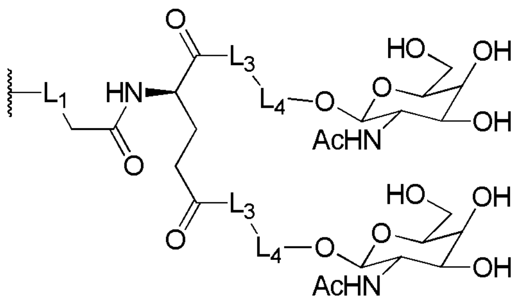,

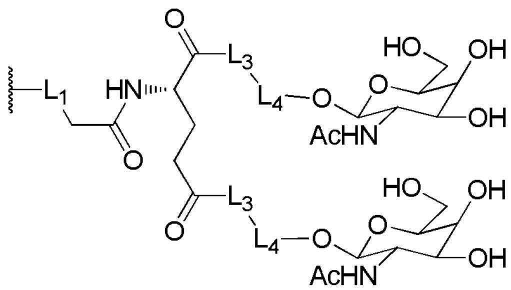, 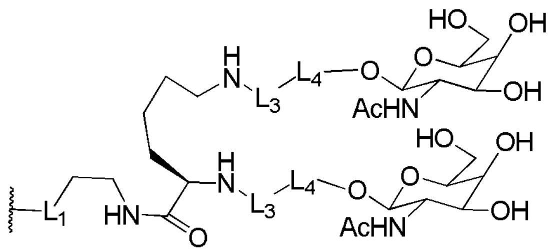

or

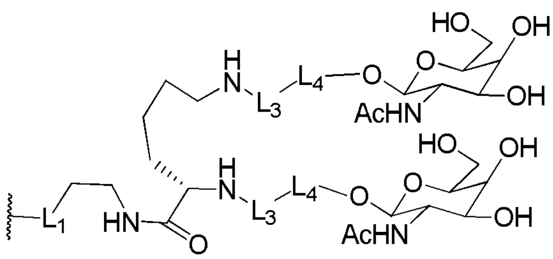;

wherein $L_1$ is $-CH_2O-$ or $-CH_2O-CH_2CH_2O-$;

$L_3$ is $-(NHCH_2CH_2)_b-$, $-(NHCH_2CH_2CH_2)_b-$ or $-C(O)CH_2-$;

$L_4$ is $-(OCH_2CH_2)_c-$ or $-NHC(O)-(CH_2)_d-$;

wherein b = 1, 2, 3, 4 or 5;

c = 1, 2, 3, 4 or 5;

d = 1, 2, 3, 4, 5, 6, 7, or 8;

L is -NHC(O)-;

L' is a chemical bond or -C(O)NH-;

R' is H, a hydroxyl protecting group, or a solid support, preferably the hydroxyl protecting group is $-C(O)CH_2CH_2C(O)OH$ or 4,4'-dimethoxytrityl;

m = 0, 1, 2, 3, 4, 5, 6, 7, 8, 9 or 10;

n = 0, 1, 2, 3, 4, 5, 6, 7, 8, 9, or 10.

In some specific embodiments, wherein,

Q is independently H,

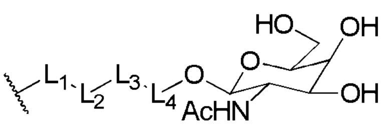,

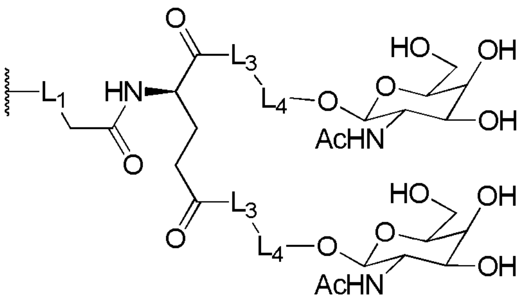,

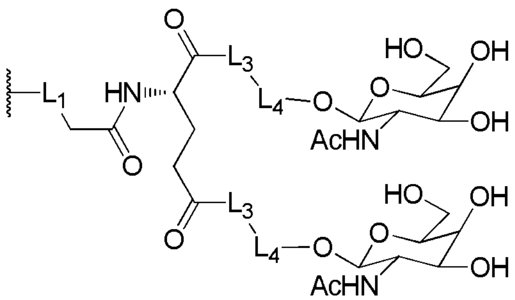,

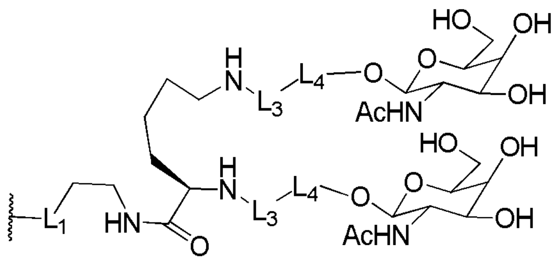

or

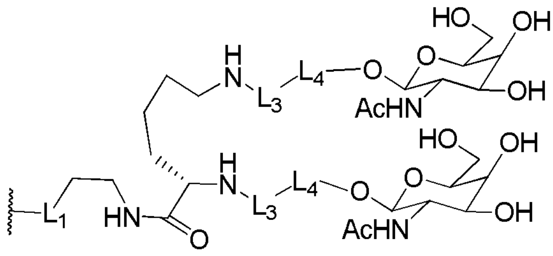
;

wherein $L_1$ is $-CH_2-$, $-C(O)-$, $-CH_2O-$, $-CH_2O-CH_2CH_2O-$ or $-NHC(O)-(CH_2NHC(O))_a-$;
$L_2$ is a chemical bond;
$L_3$ is $-(NHCH_2CH_2)_b-$, $-(NHCH_2CH_2CH_2)_b-$ or $-C(O)CH_2-$;
$L_4$ is $-(OCH_2CH_2)_c-$ or $-NHC(O)-(CH_2)_d-$;
wherein a = 0, 1, 2, or 3;
b = 1, 2, 3, 4 or 5;
c = 1, 2, 3, 4 or 5;
d = 1, 2, 3, 4, 5, 6, 7, or 8;
L is $-CH_2O-$ or $-NHC(O)-$;
L' is a chemical bond or $-C(O)NH-$;
$R_1$ and $R_2$ together form $-CH_2CH_2O-$ or $-CH_2CH(R)-O-$, and $R_3$ is H;
or $R_1$ and $R_3$ together form $-C_{1-2}$ alkylene-, and $R_2$ is H;
wherein R is -OR', $-CH_2OR'$ or $-CH_2CH_2OR'$, wherein R' is H, a hydroxyl protecting group, or a solid support, preferably the hydroxyl protecting group is $-C(O)CH_2CH_2C(O)OH$ or 4,4'-dimethoxytrityl, ;
m = 0, 1, 2, 3, 4, 5, 6, 7, 8, 9 or 10;
n = 0, 1, 2, 3, 4, 5, 6, 7, 8, 9, or 10.

In some embodiments, wherein the conjugating group is as represented by Formula (II'-2):

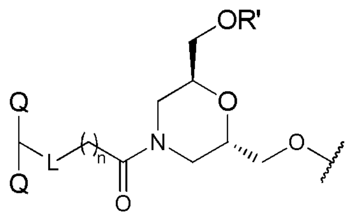
(II'-2)

wherein

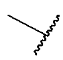

represents the point of attachement to the dsRNA;

Q is independently

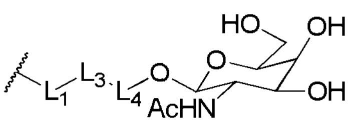
,
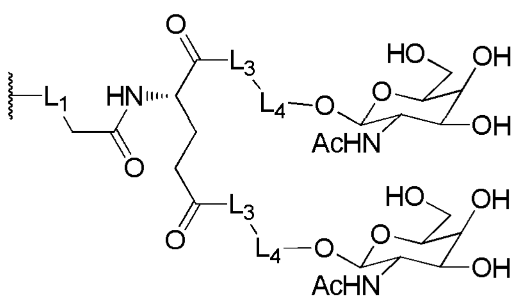

or

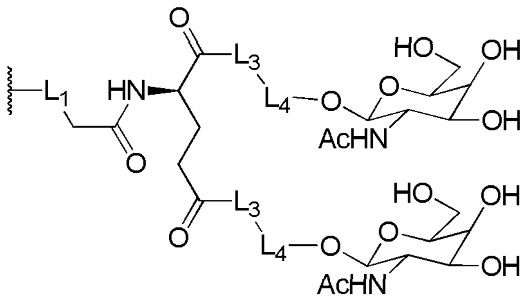;

wherein $L_1$ is $-CH_2-$ or-C(O)-;

$L_3$ is $-(NHCH_2CH_2)_b-$;

$L_4$ is $-(OCH_2CH_2)_c-$;

wherein b = 1, 2, 3, 4 or 5;

c = 1, 2, 3, 4 or 5;

L is $-CH_2O-$ or -NHC(O)-;

R' is H, a hydroxyl protecting group, or a solid support, preferably the hydroxyl protecting group is $-C(O)CH_2CH_2C(O)OH$ or 4,4'-dimethoxytrityl, ;

n = 0, 1, 2, 3, 4, 5, 6, 7, 8, 9, or 10.

In some specific embodiments, wherein:

Q is independently H,

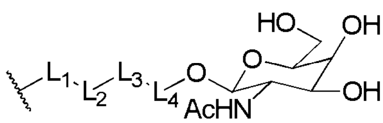, 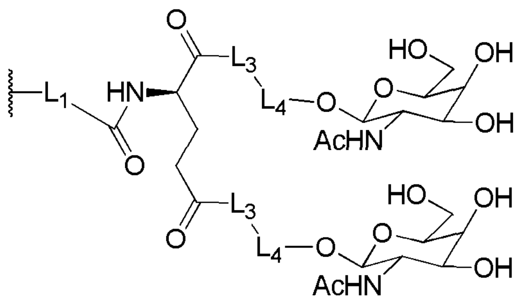,

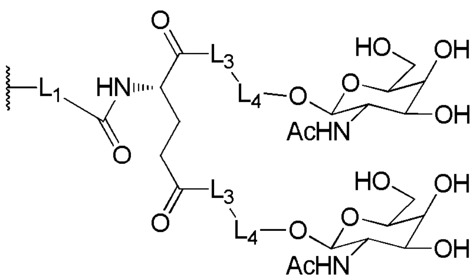, 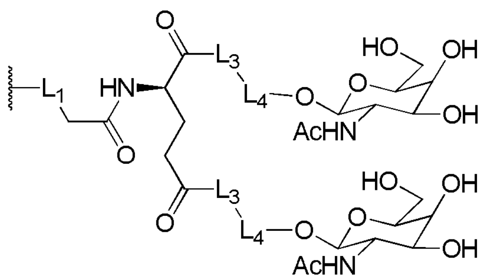,

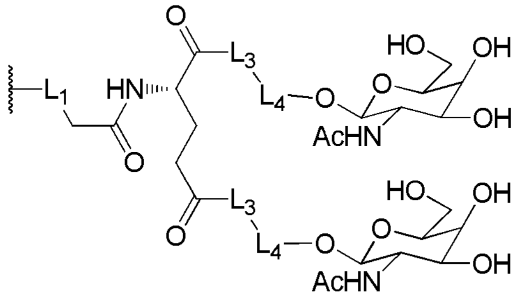
,
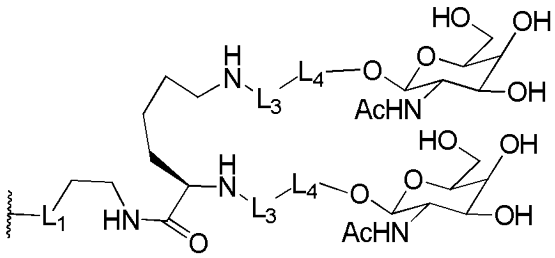

or

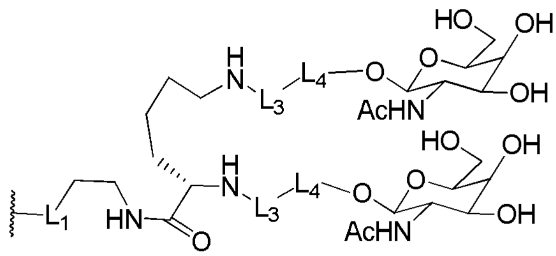
;

wherein $L_1$ is a chemical bond, $-CH_2-$, $-CH_2CH_2-$, -C(O)-, $-CH_2O-$, $-CH_2O-CH_2CH_2O-$ or - $NHC(O)-(CH_2NHC(O))_a-$;

$L_2$ is a chemical bond or $-CH_2CH_2C(O)-$;

$L_3$ is a chemical bond, $-(NHCH_2CH_2)_b-$, $-(NHCH_2CH_2CH_2)_b-$ or $-C(O)CH_2-$;

L4 is $-(OCH_2CH_2)_c-$, $-(OCH_2CH_2CH_2)_c-$, $-(OCH_2CH_2CH_2CH_2)_c-$, $-(OCH_2CH_2CH_2CH_2CH_2)_c-$ or $-NHC(O)-(CH_2)_d-$;

wherein a = 0, 1, 2, or 3;

b = 1, 2, 3, 4 or 5;

c = 1, 2, 3, 4 or 5;

d = 1, 2, 3, 4, 5, 6, 7, or 8;

L is a chemical bond, $-CH_2O-$ or -NHC(O)-;

L' is a chemical bond, -C(O)NH-, -NHC(O)- or $-O(CH_2CH_2O)_e-$;

wherein e is 1, 2, 3, 4 or 5;

T is a chemical bond, $-CH_2-$, -M-, $-CH_2-M-$ or -C(O)-M-;

wherein M is

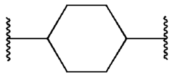
, or
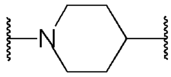
;

$R_1$ and $R_2$ together form $-CH_2CH_2O-$ or $-CH_2CH(R)-O-$, and $R_3$ is H;

or $R_1$ and $R_3$ together form $-C_{1-2}$ alkylene-, and $R_2$ is H;

wherein R is -OR', $-CH_2OR'$ or $-CH_2CH_2OR'$, wherein R' is H, a hydroxyl protecting group, or a solid support,

preferably the hydroxyl protecting group is $-C(O)CH_2CH_2C(O)OH$ or 4,4'-dimethoxytrityl;

m = 0, 1, 2, 3, 4, 5, 6, 7, 8, 9 or 10;

n = 0, 1, 2, 3, 4, 5, 6, 7, 8, 9, or 10.

In some specific embodiments, wherein,
T is-M-, $-CH_2$-M-, or-C(O)-M-, wherein M is

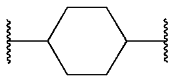
, or
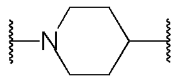
.

In some specific embodiments, wherein,

Q is independently H or

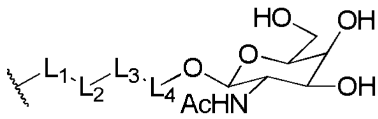
;

wherein $L_1$ is $-CH_2O-$ or $-NHC(O)-(CH_2NHC(O))_a-$;
$L_2$ is $-CH_2CH_2C(O)-$;
$L_3$ is $-(NHCH_2CH_2)_b-$ or $-(NHCH_2CH_2CH_2)_b-$;
$L_4$ is $-(OCH_2CH_2)_c-$ or $-NHC(O)-(CH_2)_d-$;
wherein a = 0, 1, 2, or 3;
b = 1, 2, 3, 4 or 5;
c = 1, 2, 3, 4 or 5;
d = 1, 2, 3, 4, 5, 6, 7, or 8;
L is a chemical bond or $-CH_2O-$;
L' is a chemical bond or $-O(CH_2CH_2O)_e-$;
wherein e is 1, 2, 3, 4 or 5;
$R_1$ and $R_2$ together form $-CH_2CH_2O-$ or $-CH_2CH(R)-O-$, and $R_3$ is H;
or $R_1$ and $R_3$ together form $-C_{1-2}$ alkylene-, and $R_2$ is H;
wherein R is -OR', $-CH_2OR'$ or $-CH_2CH_2OR'$, wherein R' is H, a hydroxyl protecting group, or a solid support, preferably the hydroxyl protecting group is $-C(O)CH_2CH_2C(O)OH$ or 4,4'-dimethoxytrityl;
m = 0, 1, 2, 3, 4, 5, 6, 7, 8, 9 or 10;
n = 0, 1, 2, 3, 4, 5, 6, 7, 8, 9, or 10;
wherein T is as defined in the above embodiments.

In some embodiments, wherein the conjugating group is as represented by Formula (III'-1), Formula (III'-2), or Formula (III'-3):

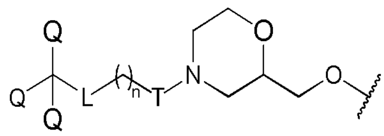
(III'-1),

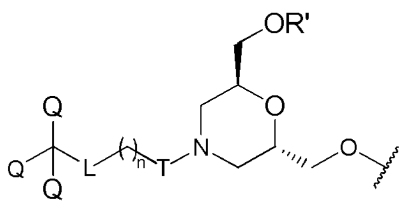
(III'-2)

or

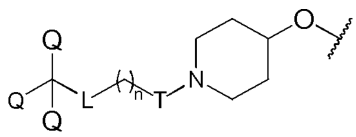

(III'-3)

wherein Q is

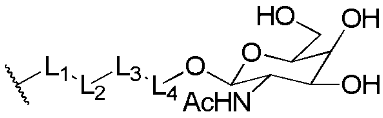

;

wherein $L_1$ is $-CH_2O-$ or -NHC(O)-;
$L_2$ is $-CH_2CH_2C(O)-$;
$L_3$ is $-(NHCH_2CH_2)_b-$ or $-(NHCH_2CH_2CH_2)_b-$;
$L_4$ is $-(OCH_2CH_2)_c-$ or $-NHC(O)-(CH_2)_d-$;
wherein b = 1, 2, 3, 4 or 5;
c = 1, 2, 3, 4 or 5;
d = 1, 2, 3, 4, 5, 6, 7, or 8;
L is a chemical bond or $-CH_2O-$;
wherein R' is H, a hydroxyl protecting group or a solid support, preferably the hydroxyl protecting group is -C(O) $CH_2CH_2C(O)OH$ or 4,4'-dimethoxytrityl, ;
n = 0, 1, 2, 3, 4, 5, 6, 7, 8, 9, or 10;
wherein T is as defined in the above embodiments.

In some specific embodiments, wherein,

Q is independently H,

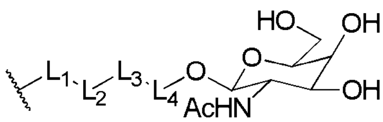

,

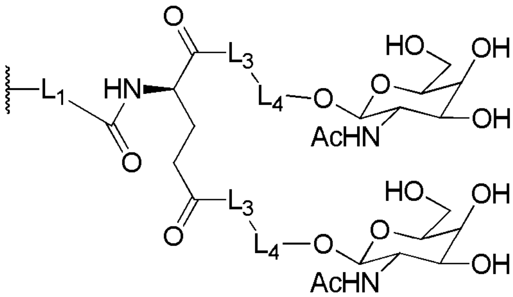

,

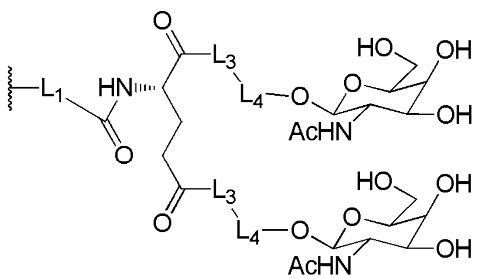

,

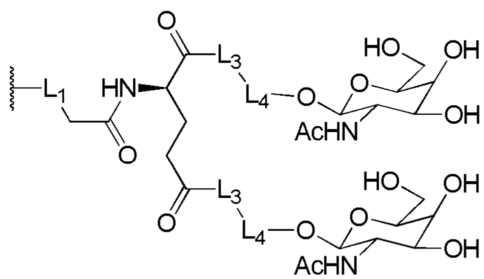

,

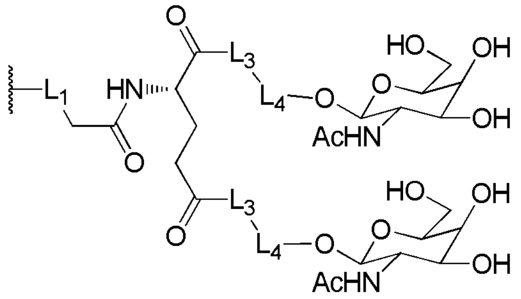

,

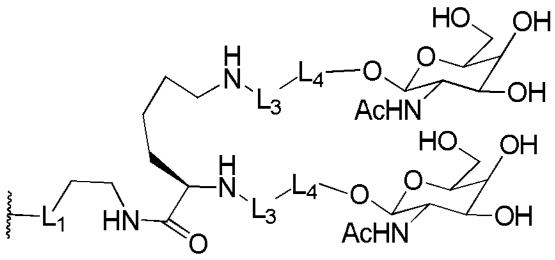

or

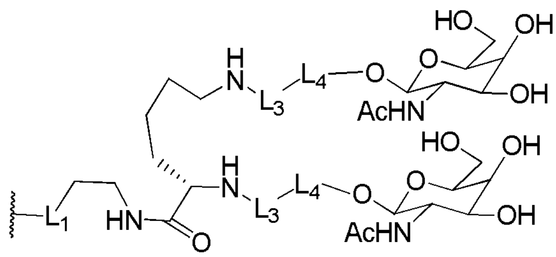

;

wherein $L_1$ is $-CH_2-$, $-CH_2O-$ or -C(O)-;
$L_2$ is a chemical bond;
$L_3$ is $-(NHCH_2CH_2)_b-$, $-(NHCH_2CH_2CH_2)_b-$ or $-C(O)CH_2-$;
$L_4$ is $-(OCH_2CH_2)_c-$ or $-NHC(O)-(CH_2)_d-$;
wherein b = 1, 2, 3, 4 or 5;
c = 1, 2, 3, 4 or 5;
d = 1, 2, 3, 4, 5, 6, 7, or 8;
L is a chemical bond or -NHC(O)-;
L' is a chemical bond;
$R_1$ and $R_2$ together form $-CH_2CH_2O-$ or $-CH_2CH(R)-O-$, and $R_3$ is H;
or $R_1$ and $R_3$ together form $-C_{1-2}$ alkylene-, and $R_2$ is H;
wherein R is -OR', $-CH_2OR'$ or $-CH_2CH_2OR'$ or a solid support, wherein R' is H, a hydroxyl protecting group, preferably the hydroxyl protecting group is $-C(O)CH_2CH_2C(O)OH$ or 4,4'-dimethoxytrityl;
m = 0, 1, 2, 3, 4, 5, 6, 7, 8, 9 or 10;
n = 0, 1, 2, 3, 4, 5, 6, 7, 8, 9, or 10;
wherein T is as defined in the above embodiments.

In some embodiments, wherein the conjugating group is as shown in Formula (IV-1) or Formula (IV-2):

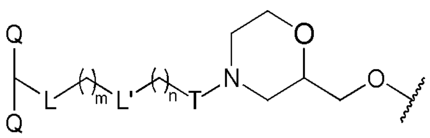
(IV-1) or
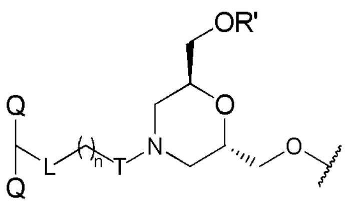
(IV-2)

wherein,

Q is independently

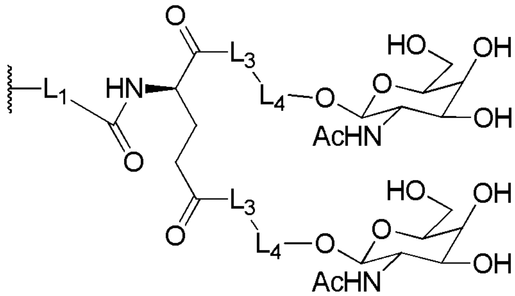

,

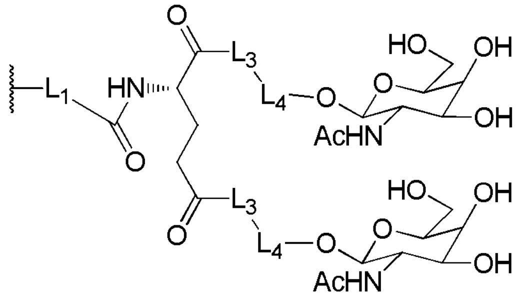

,

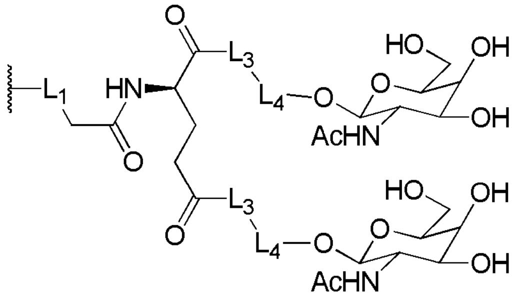

or

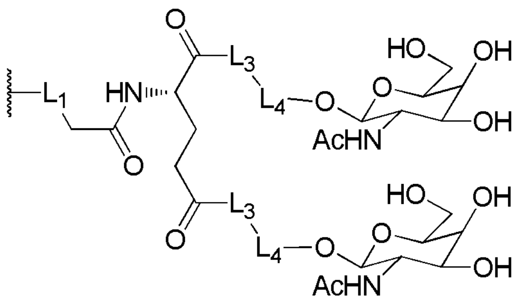

;

wherein $L_1$ is $-CH_2-$, $-CH_2O-$ or -C(O)-;

$L_3$ is $-(NHCH_2CH_2)_b-$, $-(NHCH_2CH_2CH_2)_b-$ or $-C(O)CH_2-$;

$L_4$ is $-(OCH_2CH_2)_c-$ or $-NHC(O)-(CH_2)_d-$;

wherein b = 1, 2, 3, 4 or 5;

c = 1, 2, 3, 4 or 5;

d = 1, 2, 3, 4, 5, 6, 7, or 8;

L is a chemical bond or -NHC(O)-;

L' is a chemical bond;

wherein R' is H, a hydroxyl protecting group, or a solid support, preferably the hydroxyl protecting group is $-C(O)CH_2CH_2C(O)OH$ or 4,4'-dimethoxytrityl;

m = 0, 1, 2, 3, 4, 5, 6, 7, 8, 9 or 10;

n = 0, 1, 2, 3, 4, 5, 6, 7, 8, 9, or 10;

wherein T is as defined in the above embodiments.

In some specific embodiments, wherein:

Q is independently H,

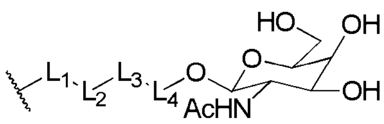,

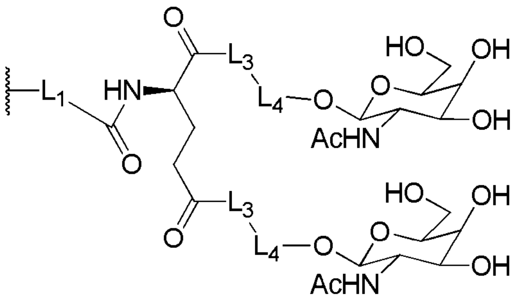,

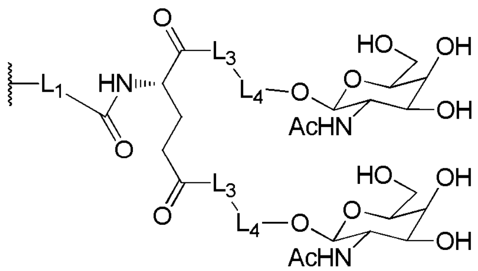,

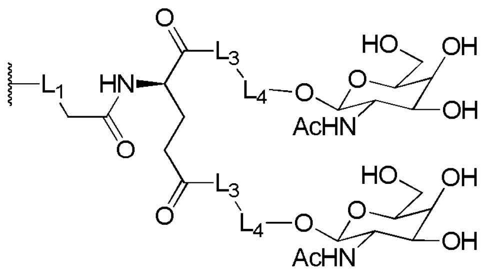,

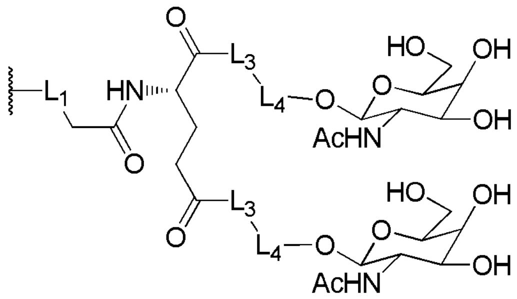,

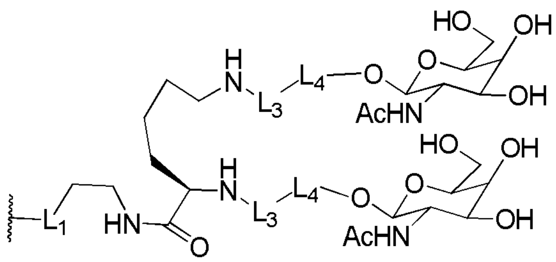

or

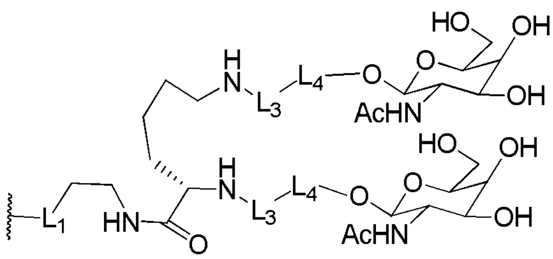;

wherein $L_1$ is a chemical bond, $-CH_2-$, $-CH_2CH_2-$, -C(O)-, $-CH_2O-$, $-CH_2O-CH_2CH_2O-$ or - $NHC(O)-(CH_2NHC(O))_a-$;

$L_2$ is a chemical bond or $-CH_2CH_2C(O)-$;

$L_3$ is a chemical bond, $-(NHCH_2CH_2)_b-$, $-(NHCH_2CH_2CH_2)_b-$或$-C(O)CH_2-$;

L4 is $-(OCH_2CH_2)_c-$, $-(OCH_2CH_2CH_2)_c-$, $-(OCH_2CH_2CH_2CH_2)_c-$, $-(OCH_2CH_2CH_2CH_2CH_2)_c-$ or $-NHC(O)-(CH_2)_d-$;

wherein a = 0, 1, 2, or 3;

b = 1, 2, 3, 4 or 5;

c = 1, 2, 3, 4 or 5;

d = 1, 2, 3, 4, 5, 6, 7, or 8;

L is a chemical bond, $-CH_2O-$ or -NHC(O)-;

L' is $-O(CH_2CH_2O)_e-$;

wherein e is 1, 2, 3, 4 or 5;

T is a chemical bond, $-CH_2-$, -C(O)-, -M-, $-CH_2-M-$ or -C(O)-M-;

wherein M is

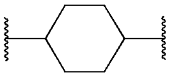 , or 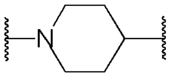 ;

$R_1$ and $R_2$ together form $-CH_2CH_2O-$ or $-CH_2CH(R)-O-$, and $R_3$ is H;

or $R_1$ and $R_3$ together form $-C_{1-2}$ alkylene-, and $R_2$ is H;

wherein R is -OR', $-CH_2OR'$ or $-CH_2CH_2OR'$, wherein R' is H, a hydroxyl protecting group, or a solid support, preferably the hydroxyl protecting group is $-C(O)CH_2CH_2C(O)OH$ or 4,4'-dimethoxytrityl;

m = 0, 1, 2, 3, 4, 5, 6, 7, 8, 9 or 10;

n = 0, 1, 2, 3, 4, 5, 6, 7, 8, 9, or 10.

In some preferred embodiments, wherein the conjugating group is selected from Table 1 below:

Table 1

| No. | Structure |
|---|---|
| 1 | 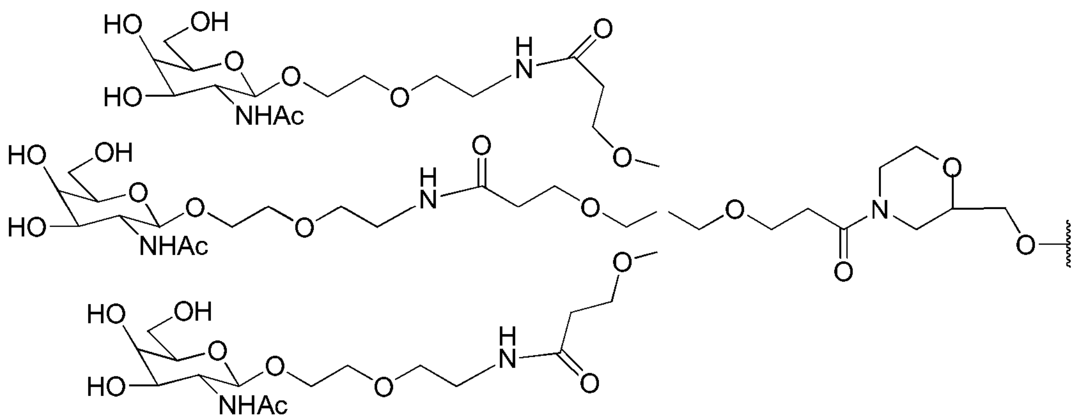 |

(continued)

| No. | Structure |
|---|---|
| 2 | 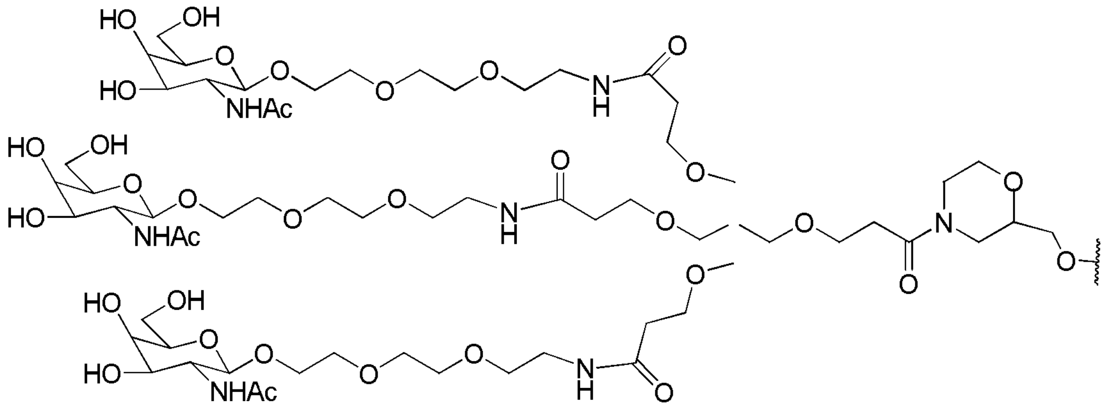 |
| 3 | 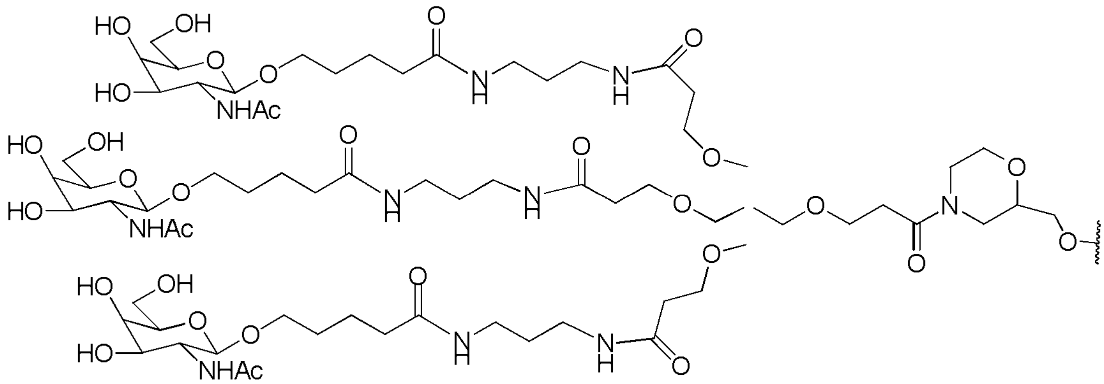 |
| 4 | 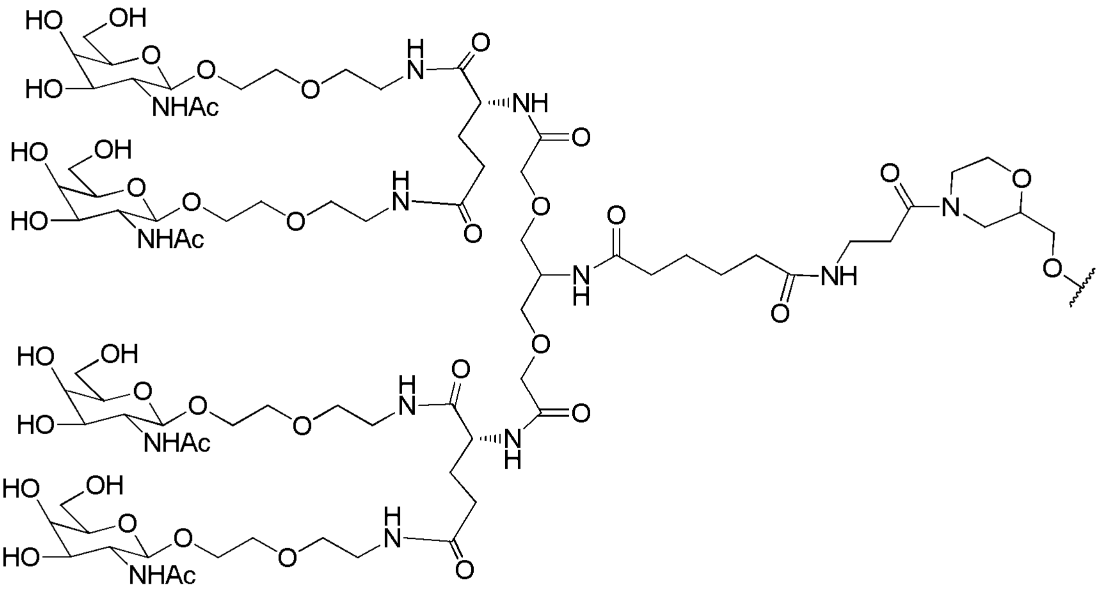 |

(continued)

| No. | Structure |
|---|---|
| 5 | 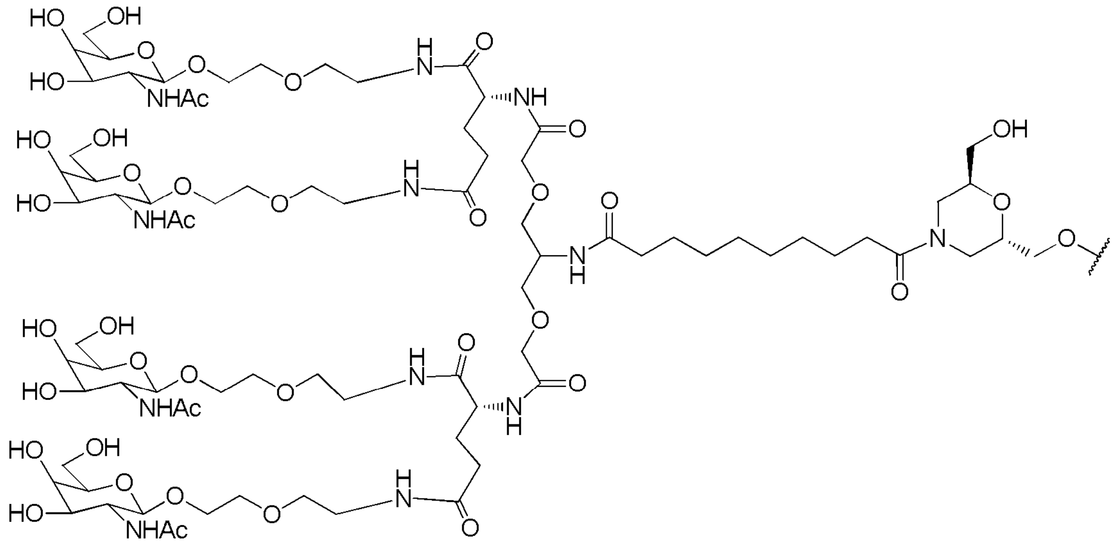 |
| 6 | 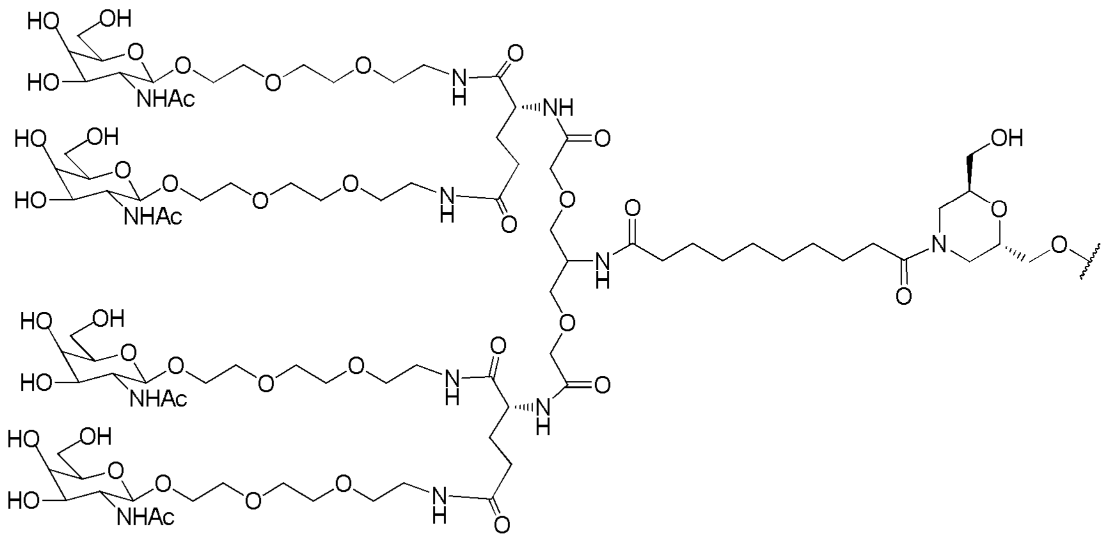 |
| 7 | 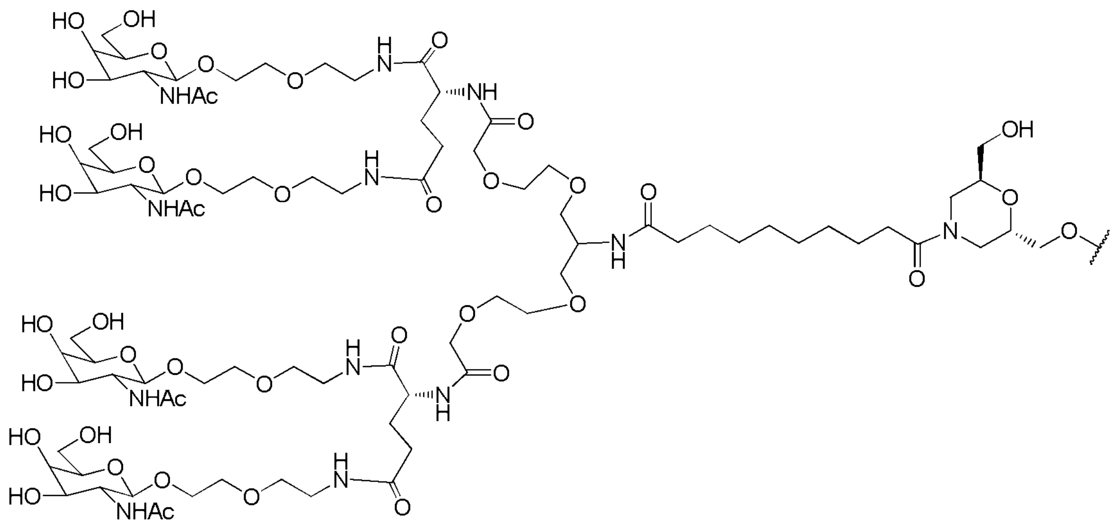 |

(continued)

| No. | Structure |
|---|---|
| 8 | 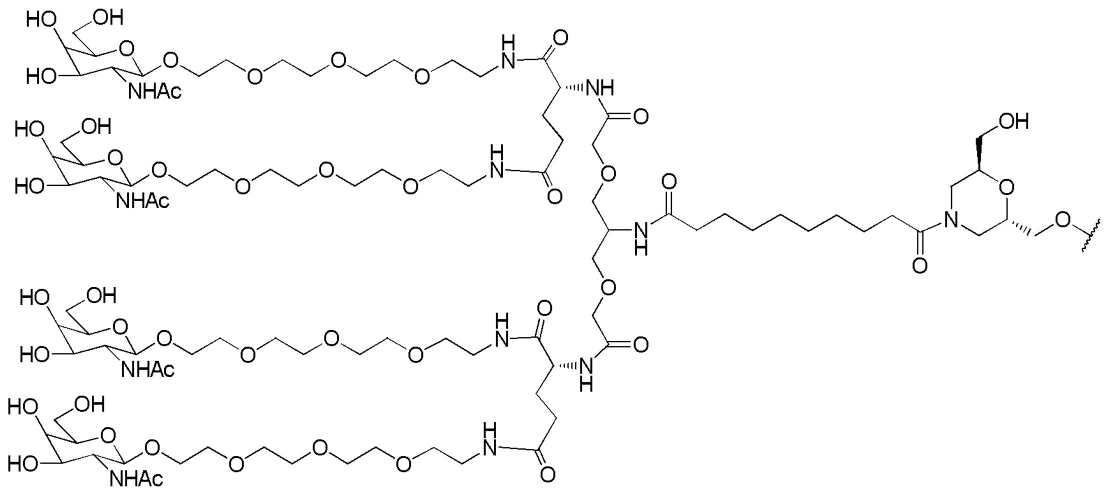 |
| 9 | 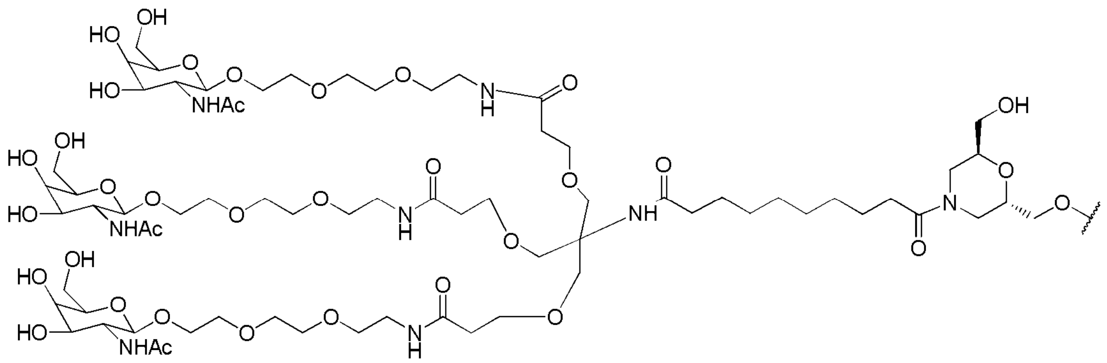 |
| 10 | 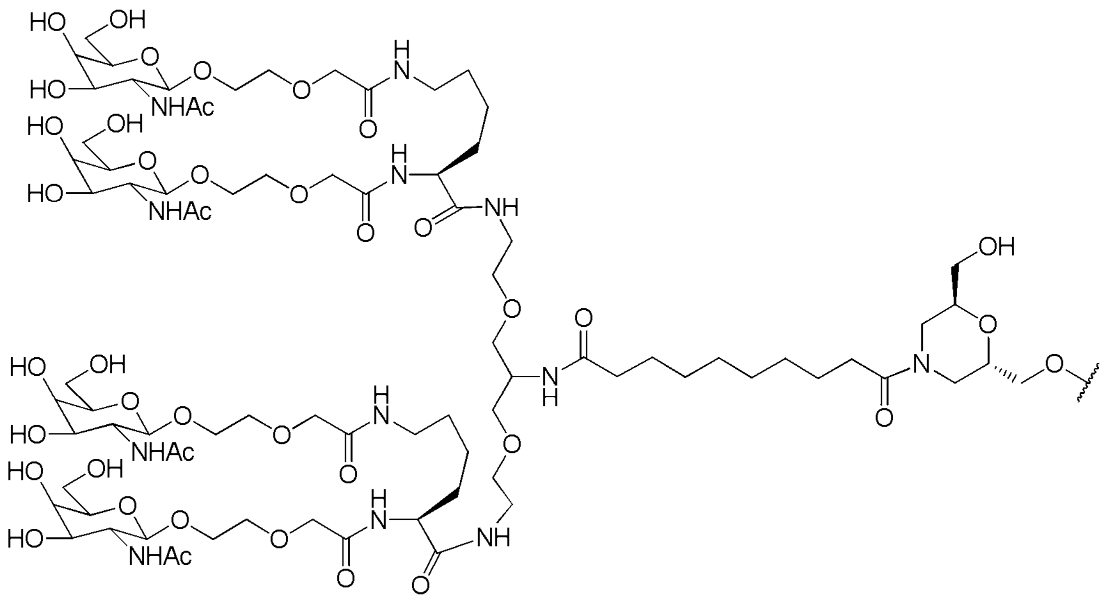 |

(continued)

| No. | Structure |
|---|---|
| 11 | 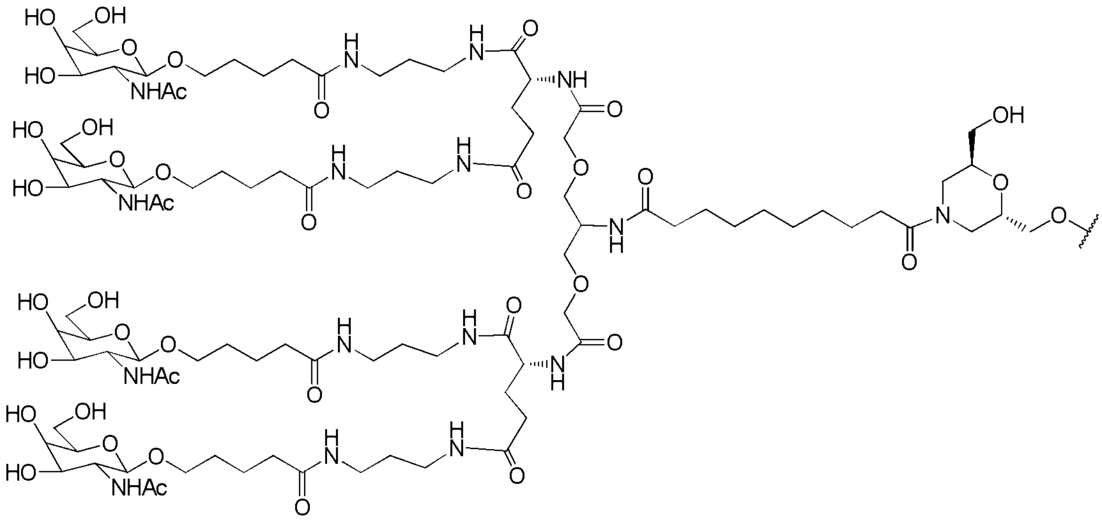 |
| 12 | 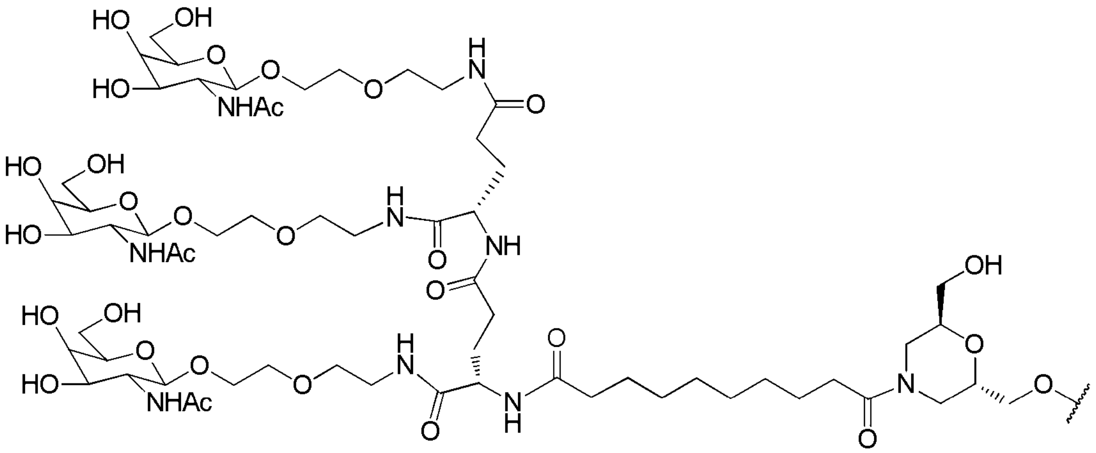 |
| 13 | 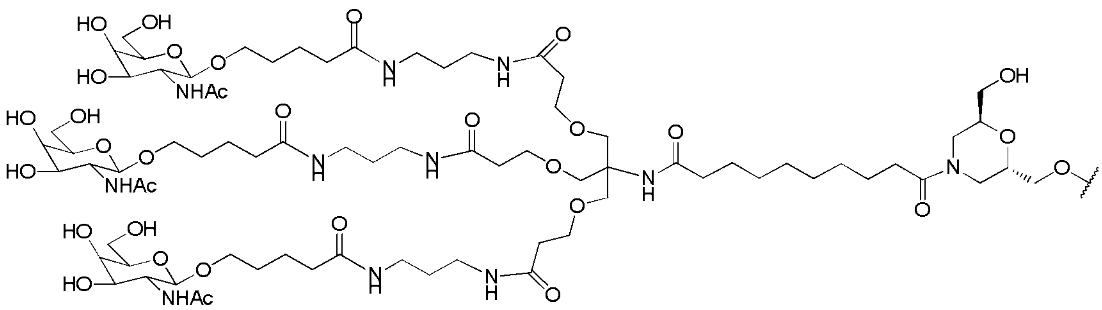 |

(continued)

| No. | Structure |
|---|---|
| 14 | 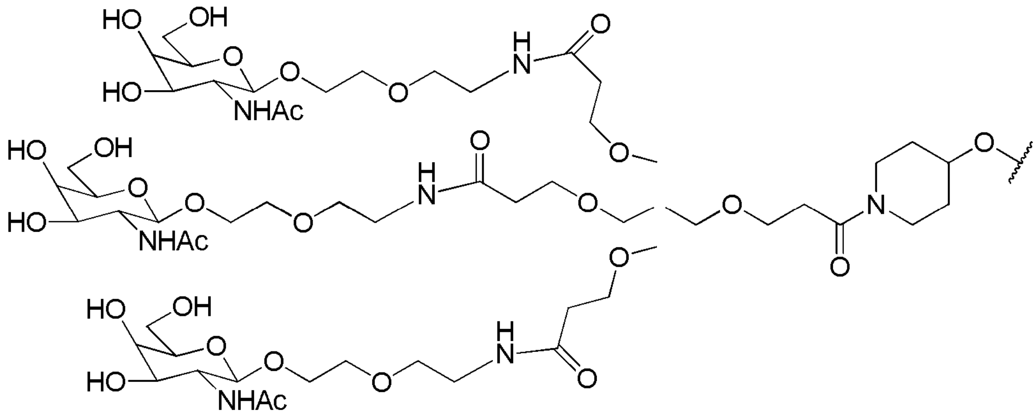 |
| 15 | 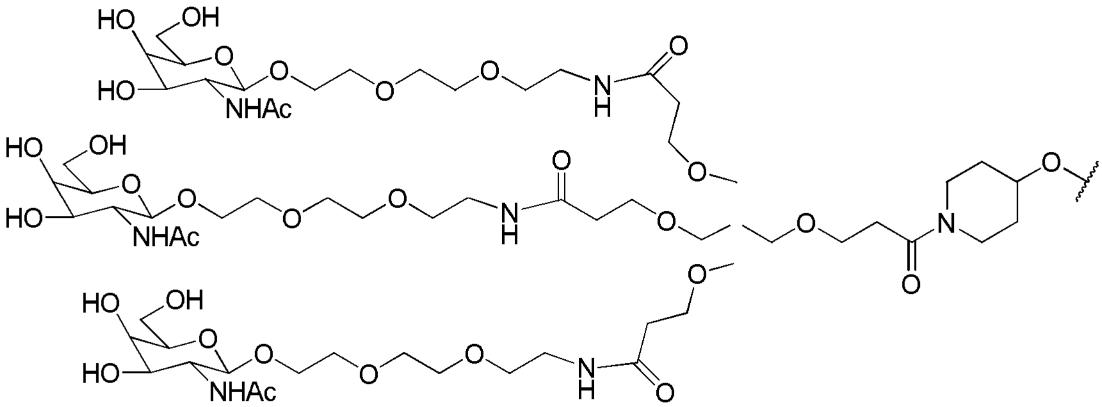 |
| 16 | 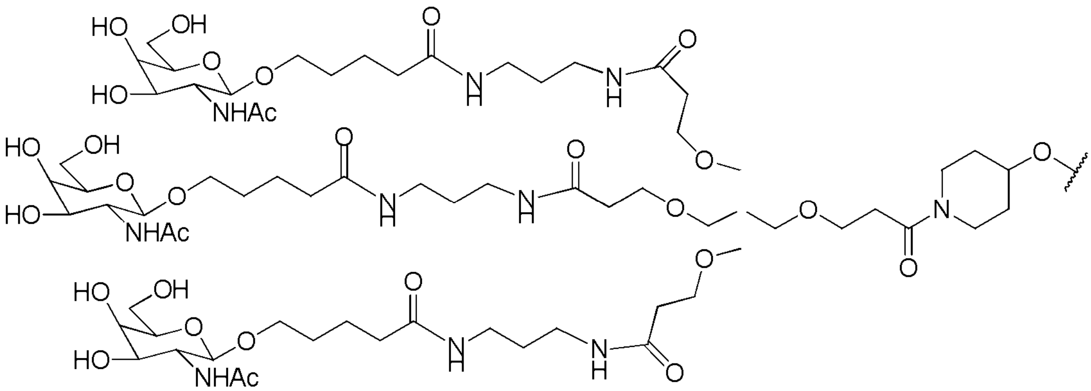 |

In some preferred embodiments, wherein the conjugating group is selected from Table 2 below:

Table 2

| NO. | Structure |
|---|---|
| 17 | 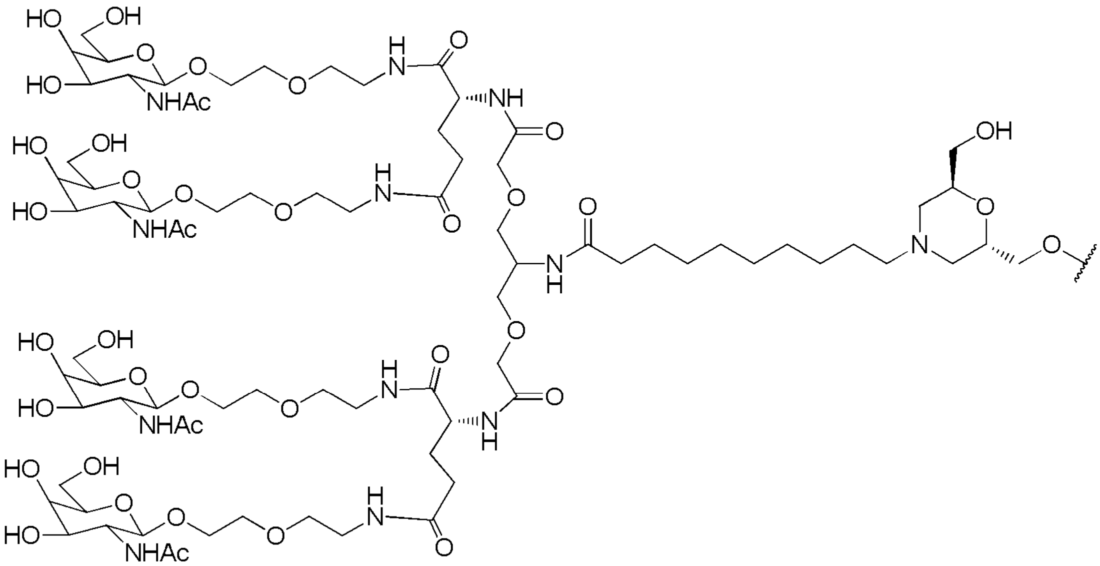 |
| 18 | 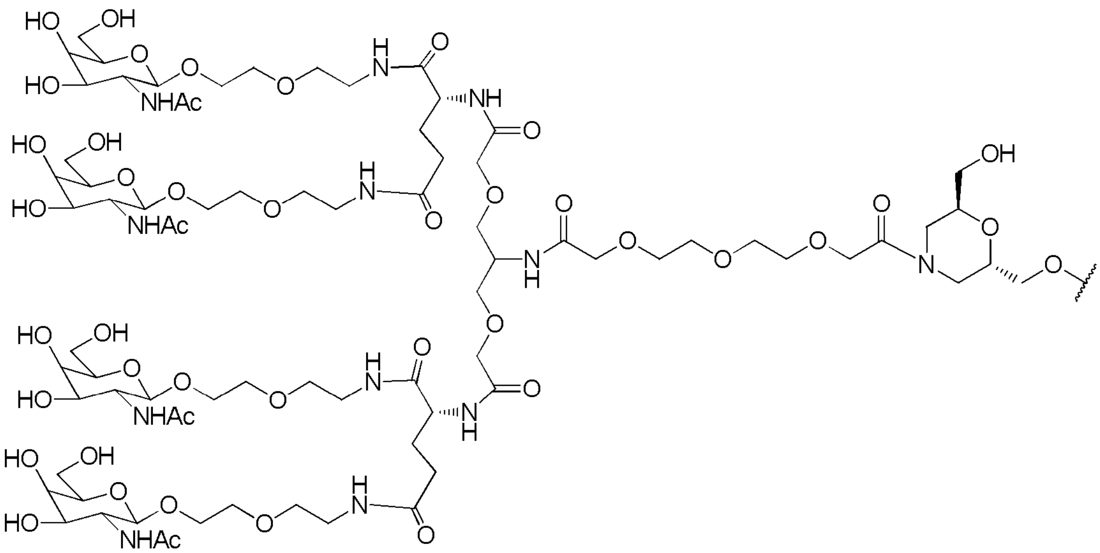 |
| 19 | 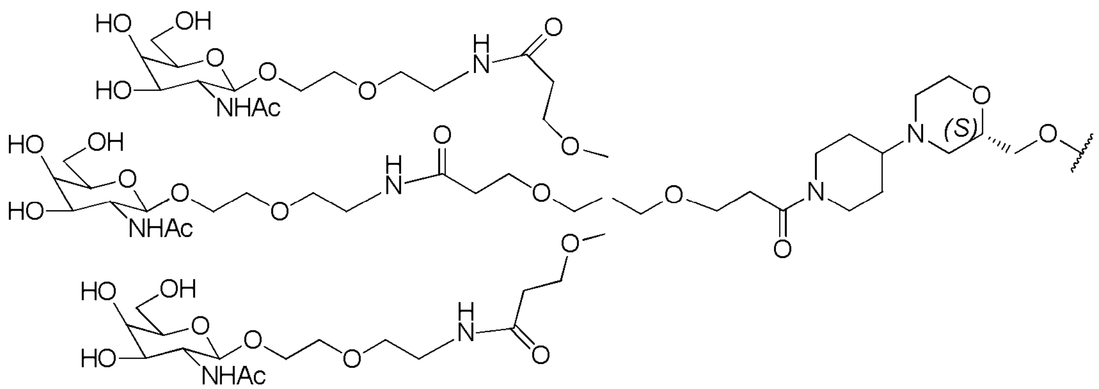 |

(continued)

| NO. | Structure |
|---|---|
| 20 | 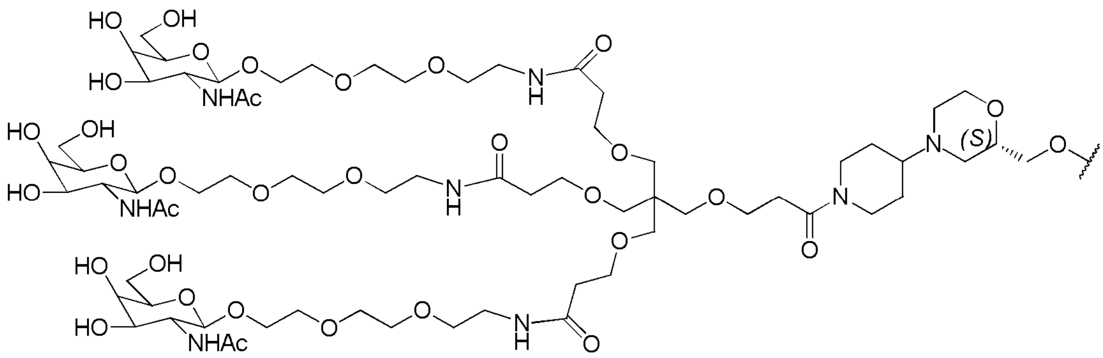 |
| 21 | 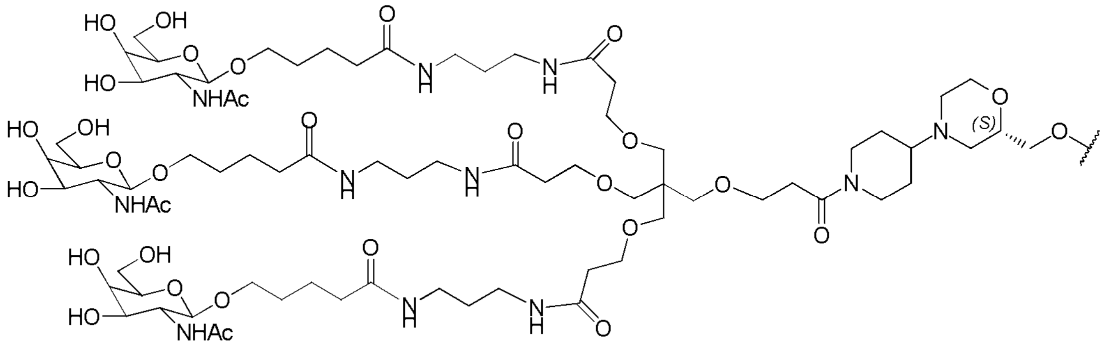 |
| 22 | 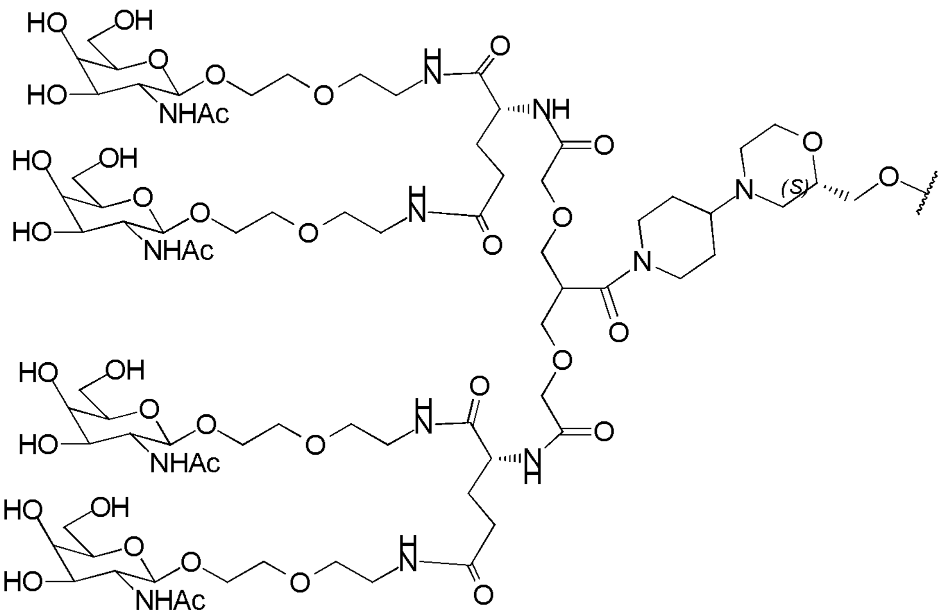 |

(continued)

| NO. | Structure |
|---|---|
| 23-1 | 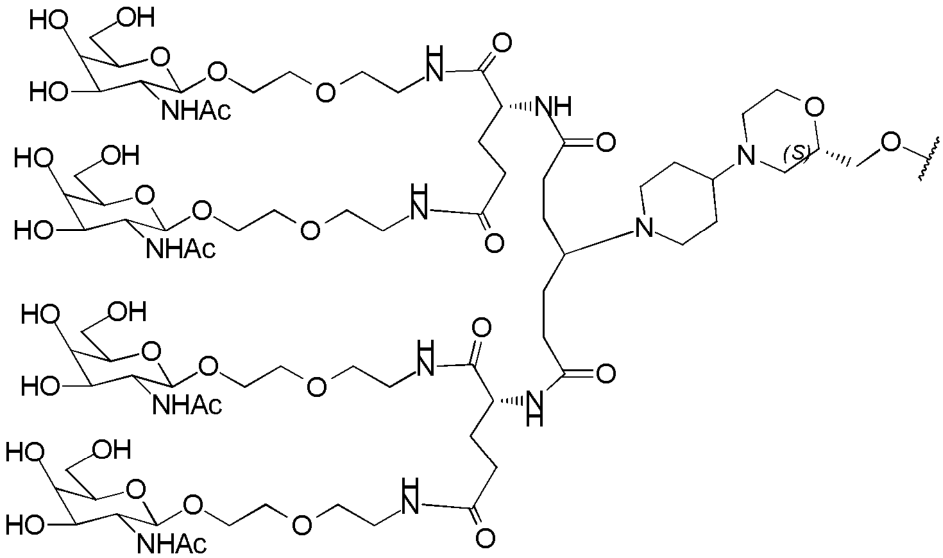 |
| 23-2 | 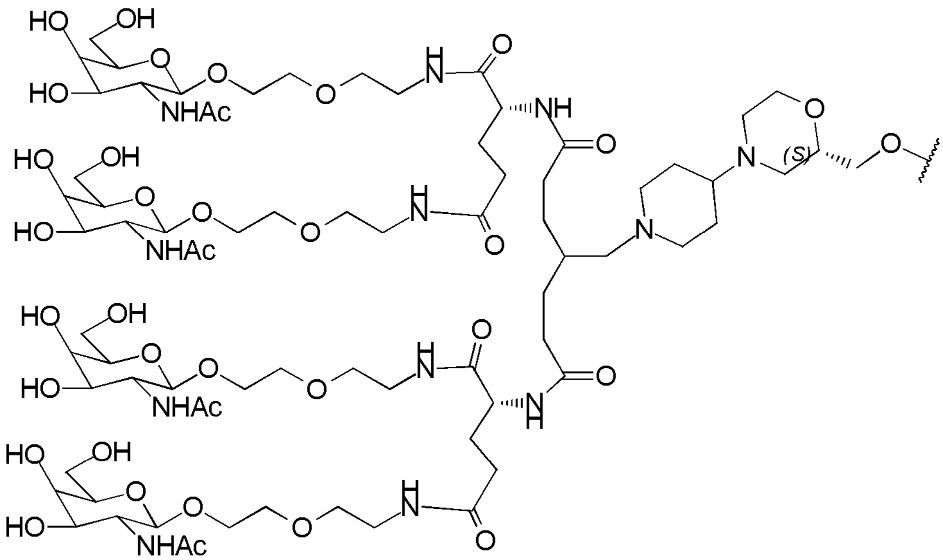 |

(continued)

| NO. | Structure |
|---|---|
| 24 | 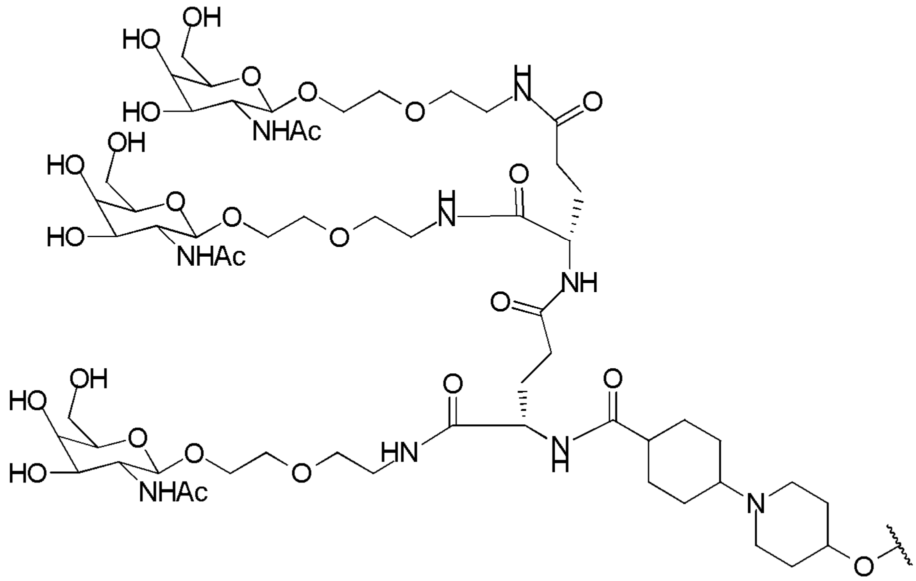 |
| 25 | 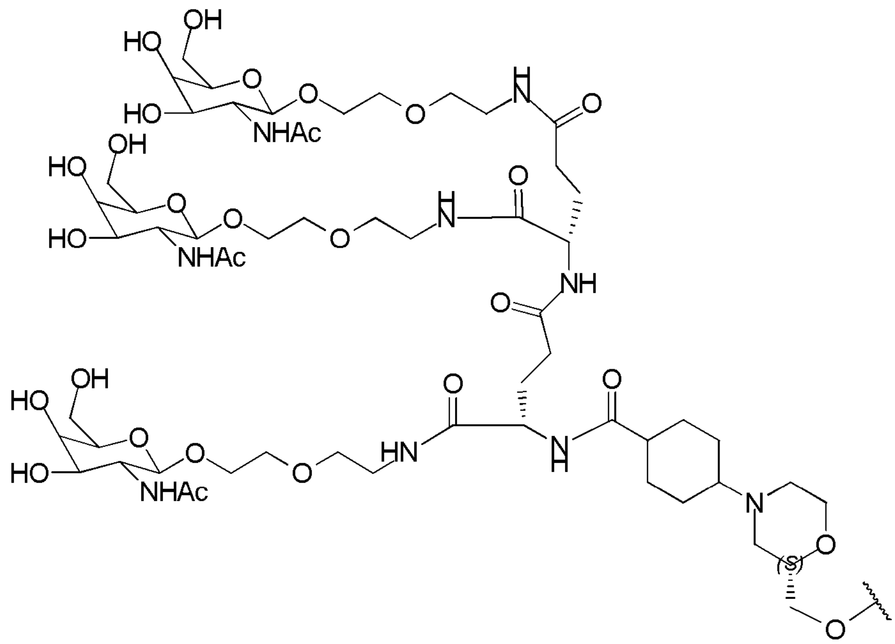 |

(continued)

| NO. | Structure |
|---|---|
| 26 | 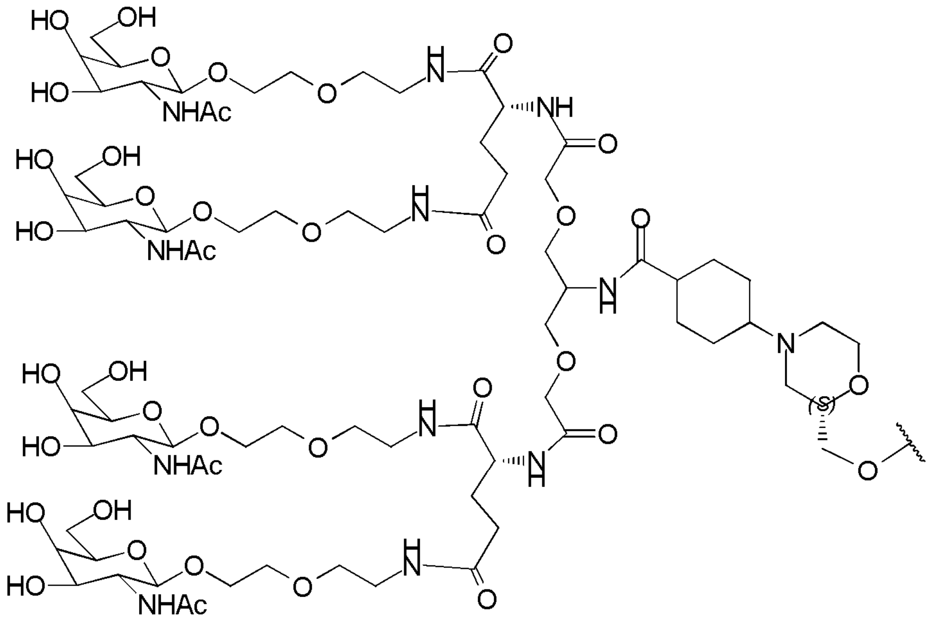 |
| 27 | 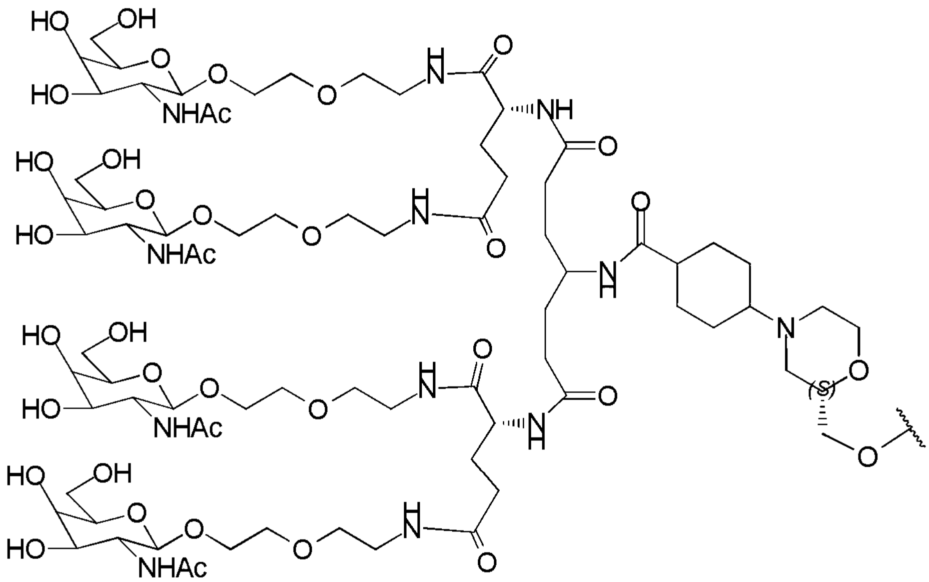 |
| 28 | 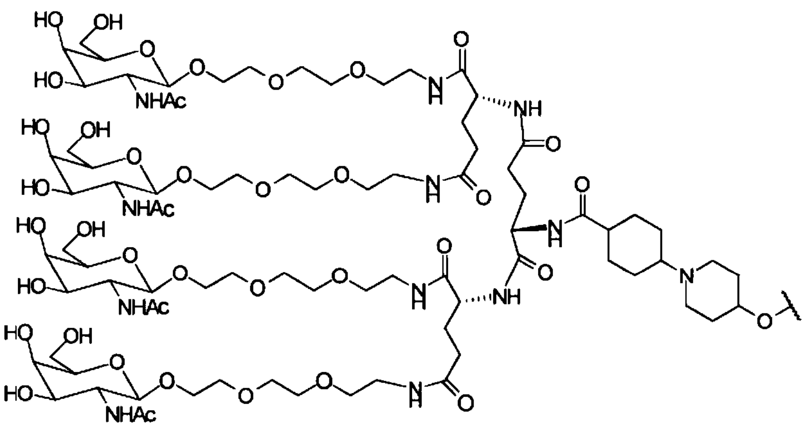 |

(continued)

| NO. | Structure |
|---|---|
| 29 | 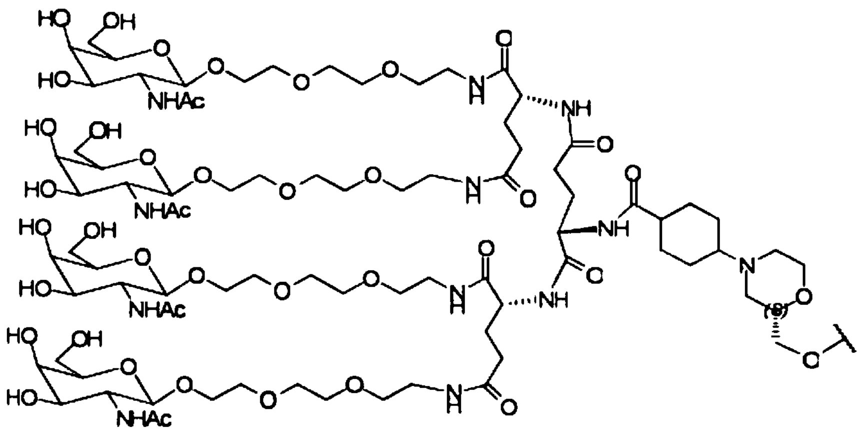 |

In some embodiments, the ligand targets asialoglycoprotein receptor (ASGPR). In some embodiments, the ligand targets asialoglycoprotein receptor (ASGPR) on hepatocytes.

In some embodiments, the ligand has the following structure:

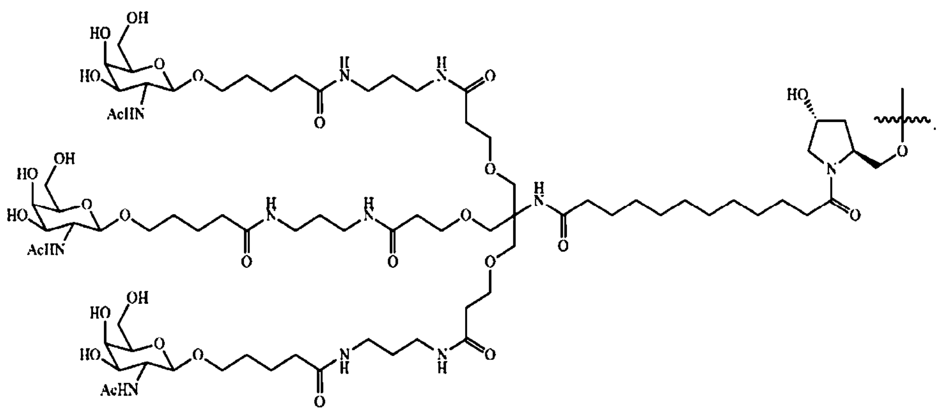

,

wherein

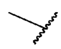

represents the point of attachment to the dsRNA through a phosphate group or a phosphorothioate group.

In some embodiments, the ligand has the following structure:

(NAG37) =

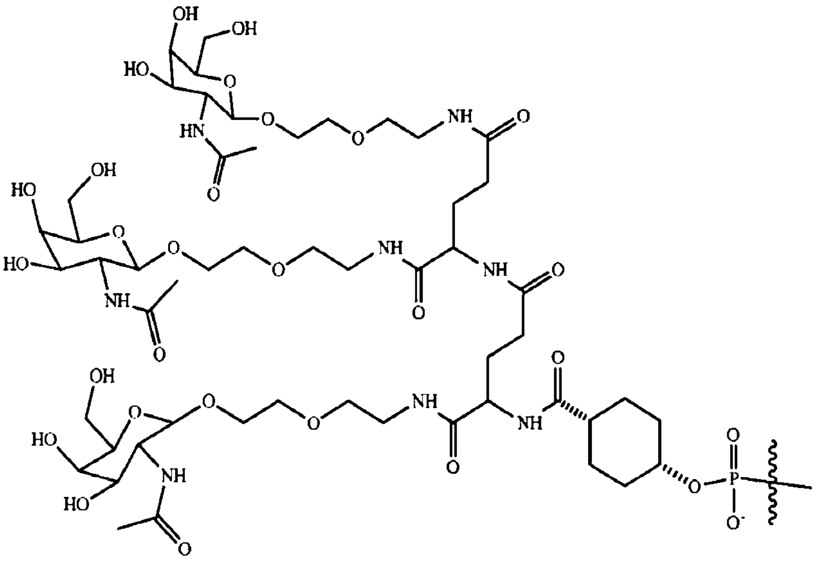

,

wherein

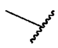

represents the point of attachment to the dsRNA through a phosphate group or a phosphorothioate group.

In some embodiments, the ligand has the following structure:

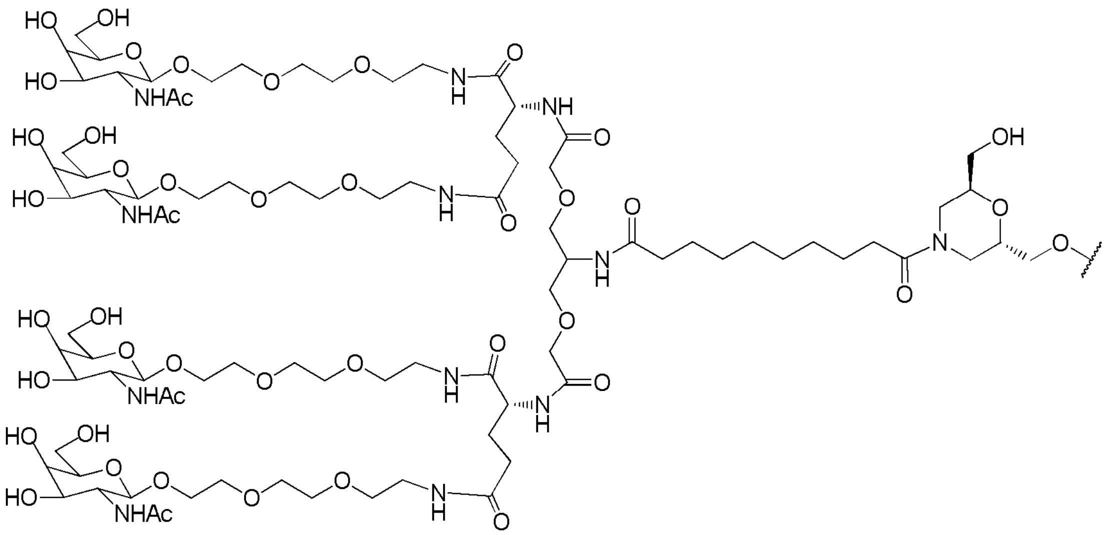

,

wherein

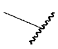

represents the point of attachment to the sense strand of the dsRNA via a phosphate group or a phosphorothioate group.

In a preferred embodiment, the ligand has the following structure:

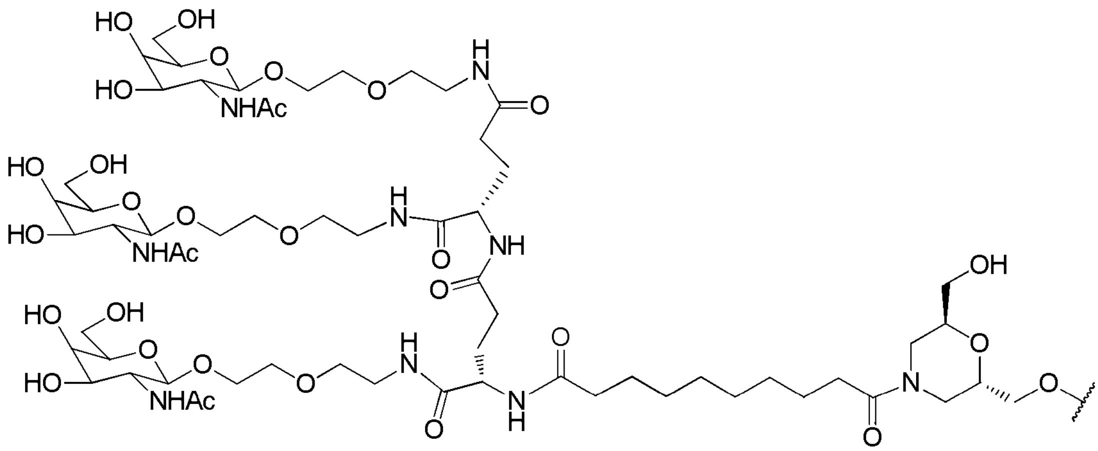

,

wherein

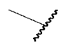

represents the point of attachment to the sense strand of the dsRNA via a phosphate group or a phosphorothioate group.

In some embodiments, the sense strand comprises a sense strand sequence conjugated to a ligand selected from any one of SEQ ID NOs: 60-79. In some preferred embodiments, the dsRNA comprises any of the paired sense strand sequences and antisense strand sequences selected from those set forth in Table 7.

## IV. Inhibition of *AGT* Gene Expression

The dsRNA of the present disclosure is capable of inhibiting *AGT* gene expression by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.

Inhibition of *AGT* gene expression may be manifested by a reduction in the levels of mRNA expressed by a first cell or population of cells (such cells may be present, for example, in a sample derived from a subject), wherein the *AGT* gene is transcribed and the cell or these cells have been treated (e.g., by contacting the cell or cells with the dsRNA of the present disclosure, or by administering the dsRNA of the present disclosure to a subject in which the cells are present or previously present, such that the *AGT* gene expression is inhibited compared to a second cell or population of cells (one or more control cells) that is substantially identical to the first cell or population of cells but has not been so treated.

In a preferred embodiment, the inhibition is assessed by the level of mRNA in treated cells as a percentage of the level of mRNA in control cells using the following formula. In some specific embodiments, the $2^{-\triangle\triangle Ct}$ value is calculated to compare the difference between the experimental group and the control group, where $\triangle\triangle Ct = [(Ct_{target\ gene,\ experimental\ group} - Ct_{reference\ gene,\ experimental\ group}) - (Ct_{target\ gene,\ control\ group} - Ct_{reference\ gene,\ control\ group})]$.

Alternatively, inhibition of *AGT* gene expression, e.g., AGT protein expression, may be assessed in terms of a decrease in parameters functionally related to *AGT* gene expression, e.g., lipid levels, cholesterol levels, such as LDLc levels. *AGT* gene silencing may be determined by any assay known in the art in any cell expressing AGT constitutively or by genomic engineering. The liver and kidney are the primary sites of AGT expression, and other significant sites for expression include small intestine, white adipose tissue, and colon.

Inhibition of the expression of the AGT protein can be manifested by a reduction in the levels of AGT protein expressed by a cell or population of cells (e.g., levels of the protein expressed in a sample derived from a subject). As explained above with respect to the assessment of mRNA inhibition, the inhibition of protein expression levels in a treated cell or population of cells may similarly be expressed as a percentage of the level of the protein in a control cell or population of cells.

A control cell or population of cells that may be used to assess inhibition of *AGT* gene expression includes a cell or population of cells that has not been exposed to the dsRNA of the present disclosure. For example, the control cell or population of cells may be derived from an individual subject (e.g., a human or animal subject) prior to the treatment of the subject with the dsRNA.

## V. Vectors

The present disclosure provides a vector comprising a nucleotide sequence encoding the dsRNA of the present disclosure. The vectors of the disclosure are capable of amplifying and/or expressing a nucleotide encoding the dsRNA of the disclosure linked thereto. The vector of the present disclosure may be a virus, a plasmid or a cosmid.

The dsRNA targeting the *AGT* gene can be expressed from a transcription unit inserted into a DNA or RNA vector. The expression can be transient (within hours to weeks) or sustained (for weeks to months or longer), depending on the particular construct used and the type of target tissue or target cell. The nucleotide encoding the dsRNA targeting the AGT mRNA can be introduced into a linear construct, a circular plasmid, or a viral vector. The nucleotides encoding the dsRNA targeting the AGT mRNA can be stably expressed by integration into the genome of the cell, or be expressed by extrachromosomal stable inheritance. In general, dsRNA expression vectors are typically DNA plasmids or viral vectors. Viral vector systems comprising coding sequences for dsRNA targeting AGT mRNA include, but are not limited to: (a) adenoviral vectors; (b) retroviral vectors; (c) adeno-associated virus vectors; (d) herpes simplex virus vectors; (e) SV40 vectors; (f) polyomavirus vectors; (g) papillomavirus vectors; (h) a picornavirus vectors; or (i) poxvirus vectors.

## VI. Cells

The present disclosure provides a cell comprising the dsRNA or vector of the present disclosure.

## VII. Pharmaceutical Composition

The present disclosure provides a pharmaceutical composition comprising the dsRNA, the vector, or the cell of the present disclosure, and optionally a pharmaceutically acceptable carrier or excipient.

As used herein, "pharmaceutically acceptable" refers to those compounds, materials, compositions, and/or dosage forms which, within the scope of sound medical judgment, are suitable for contact with tissues of human subjects and animal subjects without undue toxicity, irritation, allergic reactions, or other problems or complications, commensurate with a reasonable benefit/risk ratio.

As used herein, a pharmaceutically acceptable carrier refers to a pharmaceutical carrier that facilitates the administration of a dsRNA, a vector or a cell comprising the sequence encoding the same to the human body and/or facilitates absorption or action thereof. For example: diluents, excipients such as water, and fillers such as starch and sucrose; binders such as cellulose derivatives, alginates, gelatin, and polyvinylpyrrolidone; wetting agents such as glycerin; disintegrants such as agar, calcium carbonate, and sodium bicarbonate; absorption accelerators such as quaternary ammonium compounds; surfactants such as cetyl alcohol; adsorption carriers such as kaolin and bentonite; lubricants such as talc, calcium/magnesium stearate, and polyethylene glycol. Additional adjuvants such as flavoring agents and sweeteners can also be added to the composition.

The pharmaceutical composition of the present disclosure may comprise a pharmaceutically acceptable diluent or a sustained release matrix into which the dsRNA or vector of the present disclosure is embedded.

The pharmaceutical composition of the present disclosure may comprise a drug delivery system for delivering dsRNA. Drug delivery systems of the present dicslosure include, but are not limited to, nanoparticles (e.g., lipid nanoparticles, polymer-based nanoparticles), polymeric, PEG, or cationic delivery systems, polylactic acid (PLA) microspheres, poly(lactic-co-glycolic acid) (PLGA) microspheres, liposomes, micelles, inverse micelles, lipid cochleates, or lipid microtubules. Cholesterol, PEG lipids, such as PEG-2000-C-DMG, and PEG-2000-DMG (Moderna), ALC-0159, and DSPC.

In some embodiments, the siRNA or the vector in the pharmaceutical composition of the disclosure may be comprised in a polymer and a polymer-based nanoparticle.

In some specific embodiments, the polymer is a polymer based on poly (lactic-co-glycolic acid) (PLGA). In some specific embodiments, the polymer based on PLGA is engineered to contain an individual cationic group.

In some specific embodiments, the polymer contains an amine group capable of becoming cationic, such as polyethyleneimine (PEI) and poly (L-lysine) (PLL), which is capable of forming a complex with the siRNA through electrostatic interaction and delivering the siRNA into the cell. In some embodiments, the PEG and PLL are chemically modified to improve in vivo potency and tolerability.

In some embodiments, the siRNA, vector, or cell in the pharmaceutical composition of the disclosure can be delivered by a cationic polymer poly (β-aminoester) (PBAE).

## VIII. Kit

The present disclosure provides a kit comprising the dsRNA, vector, or cell of the present disclosure.

The present disclosure also provides kits for using the dsRNA of the present disclosure and/or performing the

methods of the present disclosure. Such kits include one or more dsRNAs, vector, or cells of the disclosure, and may further include instructions for use. The instructions for inhibiting expression of AGT in a cell by contacting the cell with the dsRNA or vector of the present disclosure in an amount effective to inhibit expression of AGT may be recorded in the instructions for use.

In the case where the dsRNA of the present disclosure is contacted with a cell in vitro, optionally, the kit of the present disclosure may further comprise a means for contacting the cell with the dsRNA or vector of the present disclosure (e.g., an injection device) or a means for measuring the inhibitory effect of AGT (e.g., a means for measuring the inhibition of AGT mRNA or protein). Such means for measuring inhibition of AGT may comprise means for obtaining a sample (e.g., a plasma sample) from a subject.

In the case where the dsRNA or vector of the present disclosure, or a cell into which the dsRNA or vector has been introduced in vitro is administered into the body, the kit of the present disclosure may optionally further comprise a device for administering the dsRNA, the vector, or the cell of the present disclosure to a subject, or a device for determining a therapeutically effective amount or a prophylactically effective amount.

## IX. Treatment Methods and Pharmaceutical Uses

The present disclosure provides a method of reducing angiotensinogen (AGT) in a subject, comprising the step of administering to the subject the dsRNA, vector, cell, or pharmaceutical composition of the disclosure.

The present disclosure provides a method of treating, preventing, inhibiting or alleviating a disease or disorder benefiting from reduced expression of angiotensinogen (AGT) in a subject, comprising the step of administering to the subject the dsRNA, vector, cell, or pharmaceutical composition of the present disclosure. The present disclosure also provides a method of treating, preventing, inhibiting or alleviating at least one symptom in a patient suffering from a disease or disorder benefiting from reduced expression of angiotensinogen (AGT).

In some embodiments, the disease or disorder benefiting from reduced expression of angiotensinogen (AGT) is an AGT-associated disease.

In some embodiments, the method of reducing angiotensinogen (AGT) in a subject, the method of treating, preventing, inhibiting, or alleviating a disease or disorder benefiting from reduced expression of angiotensinogen (AGT) in a subject, or the method of treating, preventing, inhibiting, or alleviating at least one symptom in a patient suffering from a disease or disorder benefiting from reduced expression of angiotensinogen (AGT) provided in the present disclosure comprises subcutaneously, locally or intravenously administering the dsRNA, vector, cell or pharmaceutical composition to the subject. In some embodiments, the subject is a human patient.

The present disclosure also relates to the dsRNA, vector, cell or pharmaceutical composition of the present disclosure for use in treating a disease or symptom associated with AGT expression in a subject.

The present disclosure also relates to the use of the dsRNA, vector, cell, or pharmaceutical composition of the present disclosure for the manufacture of a medicament for treating, preventing, inhibiting or alleviating a disease or disorder benefiting from reduced expression of angiotensinogen (AGT) in a subject. The disclosure also relates to the use of the dsRNA, vector, cell, or pharmaceutical composition of the disclosure for the manufacture of a medicament for reducing angiotensinogen (AGT) in a subject. The disclosure also relates to the use of the dsRNA, vector, cell, or pharmaceutical composition of the disclosure for the manufacture of a medicament for treating, preventing, inhibiting or alleviating at least one symptom in a patient suffering from a disease or disorder benefiting from reduced expression of angiotensinogen (AGT).

### Sequence

The RNA sequence provided by the present disclosure targets the human *AGT* gene (or target gene, target mRNA sequence, target sequence). The target AGT mRNA sequence involves the gene as shown in Genbank Accession No. NM _ 001393338.1.

Table 3. Nucleotide sequences of the sense and antisense strands targeting *AGT* mRNA

| SEQ ID NO: | Sense strand sequence (5'->3') | SEQ ID NO: | Antisense strand sequence (5'->3') |
|---|---|---|---|
| 1 | CUGUUUGCUGUGUAUGAUA | 21 | UAUCAUACACAGCAAACAGGA |
| 2 | AUUCCUGUUUGCUGUGUAU | 22 | AUACACAGCAAACAGGAAUGG |
| 3 | UUUGCUGUGUAUGAUCAAA | 23 | UUUGAUCAUACACAGCAAACA |
| 4 | UGAGAAGAUUGACAGGUUU | 24 | AAACCUGUCAAUCUUCUCAGC |
| 5 | CUCCCACCUUUUCUUCUAA | 25 | UUAGAAGAAAAGGUGGGAGAC |

(continued)

| SEQ ID NO: | Sense strand sequence (5'->3') | SEQ ID NO: | Antisense strand sequence (5'->3') |
|---|---|---|---|
| 6 | UCCCACCUUUUCUUCUAAU | 26 | AUUAGAAGAAAAGGUGGGAGA |
| 7 | CCCACCUUUUCUUCUAAUA | 27 | UAUUAGAAGAAAAGGUGGGAG |
| 8 | UUCUUCUAAUGAGUCGACU | 28 | AGUCGACUCAUUAGAAGAAAA |
| 9 | UCUUCUAAUGAGUCGACUU | 29 | AAGUCGACUCAUUAGAAGAAA |
| 10 | CUUCUAAUGAGUCGACUUU | 30 | AAAGUCGACUCAUUAGAAGAA |
| 11 | AAGCAGCCGUUUCUCCUUA | 31 | UAAGGAGAAACGGCUGCUUUC |
| 12 | AGCCGUUUCUCCUUGGUCU | 32 | AGACCAAGGAGAAACGGCUGC |
| 13 | GCCGUUUCUCCUUGGUCUA | 33 | UAGACCAAGGAGAAACGGCUG |
| 14 | CCGUUUCUCCUUGGUCUAA | 34 | UUAGACCAAGGAGAAACGGCU |
| 15 | CGUUUCUCCUUGGUCUAAA | 35 | UUUAGACCAAGGAGAAACGGC |
| 16 | GUUUCUCCUUGGUCUAAGU | 36 | ACUUAGACCAAGGAGAAACGG |
| 17 | CUGCAAAAAUUGAGCAAUA | 37 | UAUUGCUCAAUUUUUGCAGGU |
| 18 | UCAACUGGAUGAAGAAACU | 38 | AGUUUCUUCAUCCAGUUGAGG |
| 19 | UCUCCCACCUUUUCUUCUA | 39 | UAGAAGAAAAGGUGGGAGACU |
| 20 | GUUUCUCCUUGGUCUAAGA | 40 | UCUUAGACCAAGGAGAAACGG |

Tables 4 and 5 show the modified RNA sequences used in the present disclosure, respectively.

The meanings of the abbreviations used herein are as follows:
"A", "U", "G", and "C" represent a natural adenine ribonucleotide, an uracil ribonucleotide, a guanine ribonucleotide, and a cytosine ribonucleotide, respectively.

"d" represents that the nucleotide adjacent to its right side is a deoxyribonucleotide. For example, "dA", "dT", "dG" and "dC" represent adenine deoxyribonucleotide, a thymine deoxyribonucleotide, a guanine deoxyribonucleotide, and a cytosine deoxyribonucleotide, respectively.

"m" represents that the nucleotide adjacent to its left side is a 2'-$OCH_3$ modified nucleotide. For example, "Am", "Um", "Gm", and "Cm" represent 2'-$OCH_3$ modified A, U, G, and C, respectively.

"f" represents that the nucleotide adjacent to its left side is a 2'-fluoro modified nucleotide. For example, "Af", "Uf", "Gf", and "Cf" represent 2'-fluoro modified A, U, G, and C, respectively.

"s" represents that two adjacent nucleotides to the left and right sides are linked by a phosphorothioate linkage.

"s-" represents that the nucleotide adjacent to its left side and the delivery ligand adjacent to its right side are linked via a phosphorothioate linkage.

The "VP" indicates that the nucleotide adjacent to its right side is a vinyl phosphate-modified nucleotide, which is well known in the art and can be seen, for example, in PCT Publication Nos. WO2011139702, WO2013033230 and WO2019105419.

Tgn and Agn, respectively, are the adenosine-ethylene glycol nucleic acid (GNA) S-isomer and the thymidine-ethylene glycol nucleic acid (GNA) S-isomer, and are well known in the art, see, for example, PCT Publication Nos. WO2019222166A1 and WO2020132227A2, and it has the structure of

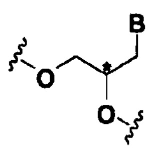

,

wherein B is thymine (for thymidine-ethylene glycol nucleic acid (GNA) S-isomer) or adenine (for adenosine-ethylene glycol nucleic acid (GNA) S-isomer).

"IB" represents an inverted abasic deoxyribonucleotide, which can include the following three structures depending on its position/conjugating mode in dsRNA. IB is well known in the art, see, for example, Czauderna, Nucleic Acids Res., 2003, 31(11), 2705-16 and PCT Publication Nos. WO2016011123 and WO2019051402:

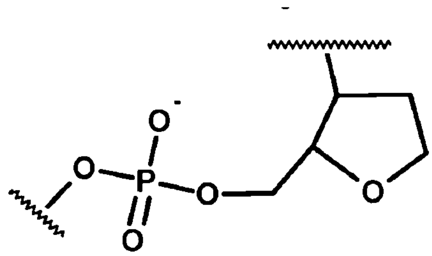

,

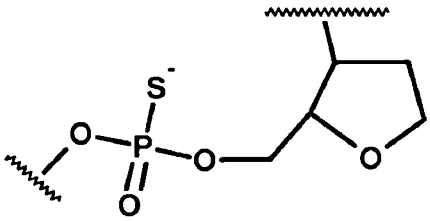

,

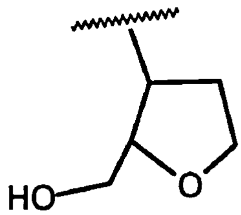

.

"L96" represents a GalNAc delivery ligand of the following structure well known in the art, wherein

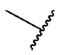

represents the point of attachment to the dsRNA through a phosphate group or a phosphorothioate group. See, for example, PCT Publication Nos. WO2009073809 and WO2009082607.

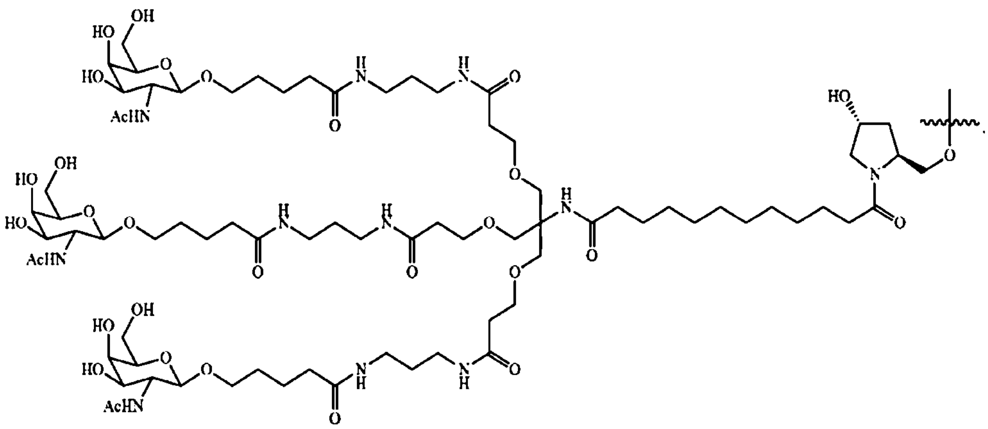

.

"GL6" represents a GalNAc delivery ligand of the following structure, wherein

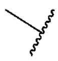

represents the point of attachment to the dsRNA through a phosphate group or a phosphorothioate group

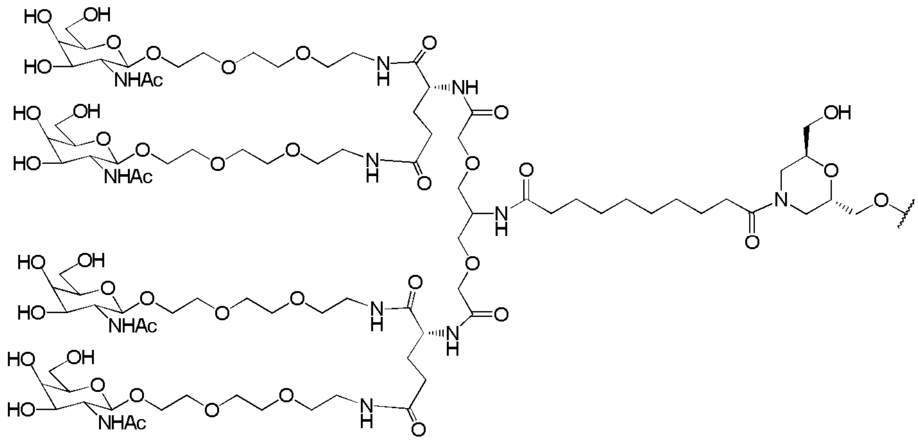

.

An "SCP-modified nucleotide" refers to a modified nucleotide having the structure:

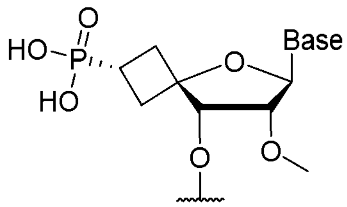

,

wherein Base is independently selected from H, a modified or unmodified Base, or a leaving group. Preferably, Base is an unmodified base, including an adenine base, a guanine base, an uracil base, and a cytosine base. For example, SCP-U means that Base is an uracil base.

Table 4. Modified sense strand sequence of the siRNA targeting the AGT mRNA

| No. of single sense strand | Sequence (5'->3') | SEQ ID NO |
|---|---|---|
| unconjugated to a ligand | | |
| SR009936S | CmsUmsGmUmUmUmGfCfUfGmUmGmUmAmUmGmAmsUmsAm | 41 |
| SR009932S | AmsUmsUmCmCmUmGfUfUfUmGmCmUmGmUmGmUmsAmsUm | 42 |
| SR009939S | UmsUmsUmGmCmUmGfUfGfUmAmUmGmAmUmCmAmsAmsAm | 43 |
| SR009659S | UmsGmsAmGmAmAmGfAfUfUmGmAmCmAmGmGmUmsUmsUm | 44 |
| SR010001S | CmsUmsCmCmCmAmCfCfUfUmUmUmCmUmUmCmUmsAmsAm | 45 |
| SR010002S | UmsCmsCmCmAmCmCfUfUfUmUmCmUmUmCmUmAmsAmsUm | 46 |
| SR010003S | CmsCmsCmAmCmCmUfUfUfUmCmUmUmCmUmAmAmsUmsAm | 47 |
| SR010010S | UmsUmsCmUmUmCmUfAfAfUmGmAmGmUmCmGmAmsCmsUm | 48 |
| SR010011S | UmsCmsUmUmCmUmAfAfUfGmAmGmUmCmGmAmCmsUmsUm | 49 |
| SR010012S | CmsUmsUmCmUmAmAfUfGfAmGmUmCmGmAmCmUmsUmsUm | 50 |
| SR010023 S | AmsAmsGmCmAmGmCfCfGfUmUmUmCmUmCmCmUmsUmsAm | 51 |
| SR010026S | AmsGmsCmCmGmUmUfUfCfUmCmCmUmUmGmGmUmsCmsUm | 52 |
| SR010027S | GmsCmsCmGmUmUmUfCfUfCmCmUmUmGmGmUmCmsUmsAm | 53 |
| SR010028S | CmsCmsGmUmUmUmCfUfCfCmUmUmGmGmUmCmUmsAmsAm | 54 |
| SR010029S | CmsGmsUmUmUmCmUfCfCfUmUmGmGmUmCmUmAmsAmsAm | 55 |
| SR010030S | GmsUmsUmUmCmUmCfCfUfUmGmGmUmCmUmAmAmsGmsUm | 56 |

(continued)

| No. of single sense strand | Sequence (5'->3') | SEQ ID NO |
|---|---|---|
| unconjugated to a ligand | | |
| SR009865S | CmsUmsGmCmAmAmAfAfAfUmUmGmAmGmCmAmAmsUmsAm | 57 |
| SR009807S | UmsCmsAmAmCmUmGfGfAfUmGmAmAmGmAmAmAmsCmsUm | 58 |
| SR010000S | UmsCmsUmCmCmCmAfCfCfUmUmUmUmCmUmUmCmsUmsAm | 59 |
| the 3' end is phosphorothioate-modified to conjugate a ligand | | |
| CmsUmsGmUmUmUmGfCfUfGmUmGmUmAmUmGmAmUmsAm | | 113 |
| AmsUmsUmCmCmUmGfUfUfUmGmCmUmGmUmGmUmAmsUm | | 114 |
| UmsUmsUmGmCmUmGfUfGfUmAmUmGmAmUmCmAmAmsAm | | 115 |
| UmsGmsAmGmAmAmGfAfUfUmGmAmCmAmGmGmUmUmsUm | | 116 |
| CmsUmsCmCmCmAmCfCfUfUmUmUmCmUmUmCmUmAmsAm | | 117 |
| UmsCmsCmCmAmCmCfUfUfUmUmCmUmUmCmUmAmAmsUm | | 118 |
| CmsCmsCmAmCmCmUfUfUfUmCmUmUmCmUmAmAmUmsAm | | 119 |
| UmsUmsCmUmUmCmUfAfAfUmGmAmGmUmCmGmAmCmsUm | | 120 |
| UmsCmsUmUmCmUmAfAfUfGmAmGmUmCmGmAmCmUmsUm | | 121 |
| CmsUmsUmCmUmAmAfUfGfAmGmUmCmGmAmCmUmUmsUm | | 122 |
| AmsAmsGmCmAmGmCfCfGfUmUmUmCmUmCmCmUmUmsAm | | 123 |
| AmsGmsCmCmGmUmUfUfCfUmCmCmUmUmGmGmUmCmsUm | | 124 |
| GmsCmsCmGmUmUmUfCfUfCmCmUmUmGmGmUmCmUmsAm | | 125 |
| CmsCmsGmUmUmUmCfUfCfCmUmUmGmGmUmCmUmAmsAm | | 126 |
| CmsGmsUmUmUmCmUfCfCfUmUmGmGmUmCmUmAmAmsAm | | 127 |
| GmsUmsUmUmCmUmCfCfUfUmGmGmUmCmUmAmAmGmsUm | | 128 |
| CmsUmsGmCmAmAmAfAfAfUmUmGmAmGmCmAmAmUmsAm | | 129 |
| UmsCmsAmAmCmUmGfGfAfUmGmAmAmGmAmAmAmCmsUm | | 130 |
| UmsCmsUmCmCmCmAfCfCfUmUmUmUmCmUmUmCmUmsAm | | 131 |
| conjugated to a ligand | | |
| SR011428S | CmsUmsGmUmUmUmGfCfUfGmUmGmUmAmUmGmAmUmsAms-GL6 | 60 |
| SR011429S | AmsUmsUmCmCmUmGfUfUfUmGmCmUmGmUmGmUmAmsUms-GL6 | 61 |
| SR011430S | UmsUmsUmGmCmUmGfUfGfUmAmUmGmAmUmCmAmAmsAms-GL6 | 62 |
| SR011431S | UmsGmsAmGmAmAmGfAfUfUmGmAmCmAmGmGmUmUmsUms-GL6 | 63 |
| SR011432S | CmsUmsCmCmCmAmCfCfUfUmUmUmCmUmUmCmUmAmsAms-GL6 | 64 |
| SR011433S | UmsCmsCmCmAmCmCfUfUfUmUmCmUmUmCmUmAmAmsUms-GL6 | 65 |
| SR011434S | CmsCmsCmAmCmCmUfUfUfUmCmUmUmCmUmAmAmUmsAms-GL6 | 66 |
| SR011435S | UmsUmsCmUmUmCmUfAfAfUmGmAmGmUmCmGmAmCmsUms-GL6 | 67 |

(continued)

| No. of single sense strand | Sequence (5'->3') | SEQ ID NO |
|---|---|---|
| unconjugated to a ligand | | |
| SR011436S | UmsCmsUmUmCmUmAfAfUfGmAmGmUmCmGmAmCmUmsUms-GL6 | 68 |
| SR011437S | CmsUmsUmCmUmAmAfUfGfAmGmUmCmGmAmCmUmUmsUms-GL6 | 69 |
| SR011438S | AmsAmsGmCmAmGmCfCfGfUmUmUmCmUmCmCmUmUmsAms-GL6 | 70 |
| SR011439S | AmsGmsCmCmGmUmUfUfCfUmCmCmUmUmGmGmUmCmsUms-GL6 | 71 |
| SR011440S | GmsCmsCmGmUmUmUfCfUfCmCmUmUmGmGmUmCmUmsAms-GL6 | 72 |
| SR011441S | CmsCmsGmUmUmUmCfUfCfCmUmUmGmGmUmCmUmAmsAms-GL6 | 73 |
| SR011442S | CmsGmsUmUmUmCmUfCfCfUmUmGmGmUmCmUmAmAmsAms-GL6 | 74 |
| SR011443S | GmsUmsUmUmCmUmCfCfUfUmGmGmUmCmUmAmAmGmsUms-GL6 | 75 |
| SR011444S | CmsUmsGmCmAmAmAfAfAfUmUmGmAmGmCmAmAmUmsAms-GL6 | 76 |
| SR011445S | UmsCmsAmAmCmUmGfGfAfUmGmAmAmGmAmAmAmCmsUms-GL6 | 77 |
| SR011446S | UmsCmsUmCmCmCmAfCfCfUmUmUmUmCmUmUmCmUmsAms-GL6 | 78 |
| SR017723S | GmsUmsUmUmCmUmCfCfUfUmGmGmUmCmUmAmAmGmsAms-GL6 | 79 |
| SR008688S | GmsUmsCmAmUmCmCfAmCfAfAfUmGmAmGmAmGmUmAmCmAm-L96 | 80 |

Table 5. Modified antisense strand sequence of the siRNA targeting the AGT mRNA

| No. of single antisense strand | Sequence (5'->3') | SEQ ID NO |
|---|---|---|
| SR010906A | UmsAfsUmCfAmUfAmCfAmCfAmGfCmAfAmAfCmAfGmsGfsAm | 81 |
| SR010902A | AmsUfsAmCfAmCfAmGfCmAfAmAfCmAfGmGfAmAfUmsGfsGm | 82 |
| SR010909A | UmsUfsUmGfAmUfCmAfUmAfCmAfCmAfGmCfAmAfAmsCfsAm | 83 |
| SR010629A | AmsAfsAmCfCmUfGmUfCmAfAmUfCmUfUmCfUmCfAmsGfsCm | 84 |
| SR010971A | UmsUfsAmGfAmAfGmAfAmAfAmGfGmUfGmGfGmAfGmsAfsCm | 85 |
| SR010972A | AmsUfsUmAfGmAfAmGfAmAfAmAfGmGfUmGfGmGfAmsGfsAm | 86 |
| SR010973A | UmsAfsUmUfAmGfAmAfGmAfAmAfAmGfGmUfGmGfGmsAfsGm | 87 |
| SR010980A | AmsGfsUmCfGmAfCmUfCmAfUmUfAmGfAmAfGmAfAmsAfsAm | 88 |
| SR010981A | AmsAfsGmUfCmGfAmCfUmCfAmUfUmAfGmAfAmGfAmsAfsAm | 89 |
| SR010982A | AmsAfsAmGfUmCfGmAfCmUfCmAfUmUfAmGfAmAfGmsAfsAm | 90 |
| SR010993A | UmsAfsAmGfGmAfGmAfAmAfCmGfGmCfUmGfCmUfUmsUfsCm | 91 |
| SR010996A | AmsGfsAmCfCmAfAmGfGmAfGmAfAmAfCmGfGmCfUmsGfsCm | 92 |

(continued)

| No. of single antisense strand | Sequence (5'->3') | SEQ ID NO |
|---|---|---|
| SR010997A | UmsAfsGmAfCmCfAmAfGmGfAmGfAmAfAmCfGmGfCmsUfsGm | 93 |
| SR010998A | UmsUfsAmGfAmCfCmAfAmGfGmAfGmAfAmAfCmGfGmsCfsUm | 94 |
| SR010999A | UmsUfsUmAfGmAfCmCfAmAfGmGfAmGfAmAfAmCfGmsGfsCm | 95 |
| SR011000A | AmsCfsUmUfAmGfAmCfCmAfAmGfGmAfGmAfAmAfCmsGfsGm | 96 |
| SR010835A | UmsAfsUmUfGmCfUmCfAmAfUmUfUmUfUmGfCmAfGmsGfsUm | 97 |
| SR010777A | AmsGfsUmUfUmCfUmUfCmAfUmCfCmAfGmUfUmGfAmsGfsGm | 98 |
| SR010970A | UmsAfsGmAfAmGfAmAfAmAfGmGfUmGfGmGfAmGfAmsCfsUm | 99 |
| SR018485A | UmsAfsGmAfCmCfAmAfGmGfAmGfAmAfAmCfGmGfCmsUmsGm | 100 |
| SR018479A | UmsAfsGmAfCmCf(Agn)AfGmGfAmGfAmAfAmCfGmGfCmsUmsGm | 101 |
| SR017718A | (SCP-U)sdAsGmAmdCCmdAAmGmGmAmdGAmAfAmCmGmGmCmsUmsG m | 102 |
| SR018482A | (SCP-U)sAfsGmAfCmCf(Agn)AfGmGfAmGfAmAfAmCfGmGfCmsUmsGm | 103 |
| SR018483A | (SCP-U)sCfsUmUfAmGf(Agn)CfCmAfAmGfGmAfGmAfAmAfCmsGmsGm | 104 |
| SR017720A | (SCP-U)sdCsUmUmdAGmdACmCmAmAmdGGmAfGmAmAmAmCmsGmsG m | 105 |
| SR011274A | UmsGfsUmAmCm(Tgn)CmUmCmAmUmUmGmUfGmGfAmUmGmAm CmsGmsAm | 106 |

Table 6. Paired sense and antisense strands of the siRNA targeting the AGT mRNA

| No. of double strand | No. of sense strand | No. of antisense strand |
|---|---|---|
| DR005325 | SR009936S | SR010906A |
| DR005321 | SR009932S | SR010902A |
| DR005328 | SR009939S | SR010909A |
| DR005048 | SR009659S | SR010629A |
| DR005390 | SR010001S | SR010971A |
| DR005391 | SR010002S | SR010972A |
| DR005392 | SR010003S | SR010973A |
| DR005399 | SR010010S | SR010980A |
| DR005400 | SR010011S | SR010981A |
| DR005401 | SR010012S | SR010982A |
| DR005412 | SR010023S | SR010993A |
| DR005415 | SR010026S | SR010996A |
| DR005416 | SR010027S | SR010997A |
| DR005417 | SR010028S | SR010998A |
| DR005418 | SR010029S | SR010999A |
| DR005419 | SR010030S | SR011000A |
| DR005254 | SR009865S | SR010835A |
| DR005196 | SR009807S | SR010777A |
| DR005389 | SR010000S | SR010970A |

Table 7. Paired sense and antisense strands of the siRNA targeting the AGT mRNA

| No. of double strand | No. of sense strand | No. of antisense strand |
|---|---|---|
| DR005822 | SR011428S | SR010906A |
| DR005823 | SR011429S | SR010902A |
| DR005824 | SR011430S | SR010909A |
| DR005825 | SR011431S | SR010629A |
| DR005826 | SR011432S | SR010971A |
| DR005827 | SR011433S | SR010972A |
| DR005828 | SR011434S | SR010973A |
| DR005829 | SR011435S | SR010980A |
| DR005830 | SR011436S | SR010981A |
| DR005831 | SR011437S | SR010982A |
| DR005832 | SR011438S | SR010993A |
| DR005833 | SR011439S | SR010996A |
| DR005834 | SR011440S | SR010997A |
| DR005835 | SR011441S | SR010998A |
| DR005836 | SR011442S | SR010999A |
| DR005837 | SR011443S | SR011000A |
| DR005838 | SR011444S | SR010835A |
| DR005839 | SR011445S | SR010777A |
| DR005840 | SR011446A | SR010970A |
| DR005677 | SR008688S | SR011274A |
| DR009637 | SR011440S | SR018485A |
| DR009631 | SR011440S | SR018479A |
| DR009219 | SR011440S | SR017718A |
| DR009634 | SR011440S | SR018482A |
| DR009635 | SR017723S | SR018483A |
| DR009221 | SR017723S | SR017720A |

Hereinafter, the content of the present disclosure will be further described with reference to Examples. It is to be understood that the following examples are merely illustrative and should not be construed as limiting the scope of the disclosure.

## Examples

Unless otherwise specified, the materials used in the examples are commercially available from the following sources.

Huh7 cell line was purchased from Cobioer Biosciences Co., LTD., Cat. No. CBP60202

Hep3B cell line was purchased from Cobioer Biosciences Co., LTD., Cat. No. CBP60197

PHH cells were purchased from Shanghai Xuanyi Biotechnology Co., Ltd., Cat. No. QYLF-HPMC

HEK293A cell line was purchased from Cobioer Biosciences Co., LTD., Cat. No. CBP60436

Balb/c mice are purchased from Vital River Laboratory Animal Technology Co., Ltd., Cat. No. Balb/c

**Example 1 Preparation of Compound E7**

**1. Preparation of Intermediate 3-4**

*1.1 Preparation of Compound 2*

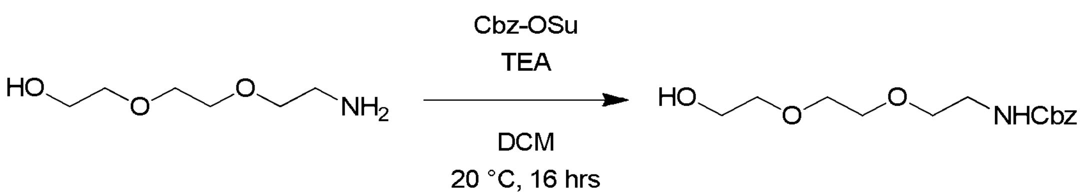


1 2

To Compound 1 (300 g, 2.01 mol) in DCM (1.80 L), benzyl(2,5-dioxopyrrolidin-1-yl) carbonate (600 g, 2.40 mol) was added slowly, and TEA (203 g, 2.01 mol, 280 mL) was added dropwise at 15°C. The mixture was then stirred at 25°C for 16 h until TLC (dichloromethane: methanol = 10:1) showed retention of reactant 1 (Rf = 0.32) and detection of a significant new spot (Rf = 0.52). The reaction mixture was washed with saturated sodium bicarbonate solution (1.00 L × 2). The organic phase was washed with brine (1.00 L), dried over anhydrous Na2SO4, and subjected to vacuum concentration. Compound 2 (about 385 g) was obtained as a yellow oil without further purification.

*1.2 Preparation of Compound 2A*

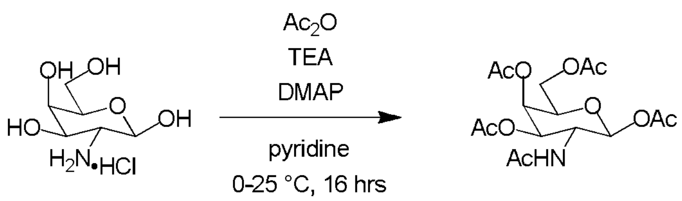


4 2A

To a solution of Compound 4 (350 g, 1.62 mol, HCl) and $Ac_2O$ (994 g, 9.74 mol, 912 mL) in pyridine (1.75 L), DMAP (19.8 g, 162 mmol) was added in one portion and TEA (164 g, 1.62 mol, 226 mL) was added dropwise at 0-15°C. The mixture was stirred at 25°C for 16 h until LCMS (product: RT = 0.687 min) showed complete consumption of the starting reactant. EtOAc (1.40 L) was added to the mixture at 25°C and stirred for 30 min. The resulting mixture was then filtered, and the filter cake was washed with EtOAc (300 mL). The filter cake was ground with water (1.45 L) at 25°C for 30 min. The mixture was filtered and the filter cake was washed with water (175 mL × 3). The filter cake was collected to obtain Compound 2A (about 580 g) as a white solid.

*1.3 Preparation of Compound 2B*

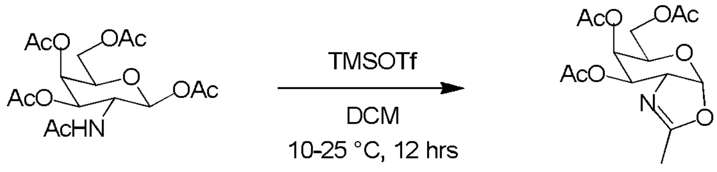


2A 2B

Three reactions were performed in parallel.

To a solution of Compound 2A (200 g, 514 mmol) in DCM (800 mL), TMSOTf (137 g, 616 mmol, 111 mL) was added dropwise over 0.5 h at 10-15°C. The mixture was then stirred at 25°C for 3 h until TLC (dichloromethane: methanol = 20:1) showed complete consumption of Compound 2A ($R_f$ = 0.54) and formation of a new spot ($R_f$ = 0.24). The three reactions were combined. The mixture was cooled to 0-15°C and slowly poured into $NaHCO_3$ (300 g dissolved in 3.00 L of water) at 0-5°C. The organic phase was separated, and the aqueous phase was extracted with DCM (1.00 L × 3). The combined organic phases were dried over $Na_2SO_4$, filtered, and subjected to vacuum concentration. Compound 2B (about 507 g) was obtained as a yellow oil for the next step without further purification.

*1.4 Preparation of Compound 3*

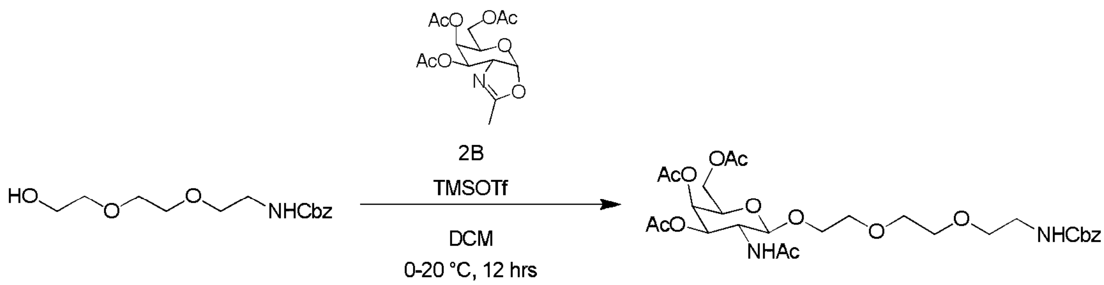


2 3

To a mixture of Compound 2B (250 g, 759 mmol) and Compound 2 (151 g, 531 mmol) in DCM (1.00 L), TMSOTf (84.4 g, 380 mmol, 69.0 mL) was added dropwise at 0-10°C. The mixture was stirred at 20°C for 12 h until TLC (dichloromethane: methanol = 20:1) showed complete consumption of Compound 2 ($R_f$ = 0.33) and formation of a new spot ($R_f$ = 0.03). The combined reaction mixture was cooled to 0-5°C and then poured into $NaHCO_3$ (100 g in 1 L of water) and stirred at 5-10°C for 10 min to separate the phases. The aqueous phase was extracted with DCM (500 mL × 2). The combined organic phases were dried over $Na_2SO_4$, filtered, and subjected to vacuum concentration. Compound 3 (about 360 g) was obtained as a yellow oil without further purification.

$^1H$ NMR : (400 MHz, DMSO).

δ=7.79-7.37 (m, 1H), 7.35-7.26 (m, 5H), 5.21-5.20 (m, 1H), 5.00-4.95 (m, 3H), 4.55-4.53 (m, 1H), 4.03-3.86 (m, 3H), 3.61-3.59 (m, 1H), 3.59-3.57 (m, 1H), 3.48-3.40 (m, 6H), 3.39-3.31 (m, 2H), 3.14-3.13 (m, 2H), 2.09 (s, 3H), 1.99 (s, 3H), 1.88 (s, 3H), 1.76-1.74 (m, 3H)

*1.5 Preparation of Intermediate 3-4 (TFA salt)*

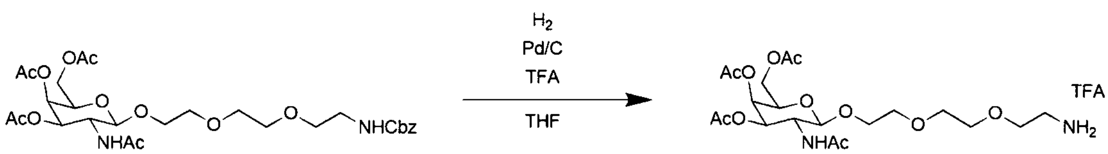


3 Intermediate 3-4 (TFA salt)

Three reactions were performed in parallel.

To a mixture of Pd/C (18.0 g, 16.3 mmol, 10% content) in THF (1.80 L), Compound 3 (180 g, 293 mmol) and TFA (33.5 g, 293 mmol, 21.8 mL) were added under an argon atmosphere. The suspension was degassed and purged three times with hydrogen. The mixture was stirred under $H_2$ (50 Psi) at 30°C for 2 h until LCMS (product: RT = 0.697 min) showed consumption of Compound 3 and detection of the product peak. The three reactions were combined. The mixture was filtered through celite and the filtrate was concentrated under reduced pressure to remove the solvent. Intermediate 3-4 (TFA salt) (393 g, 660 mmol, yield 74.8%, purity 99.6%, TFA) was obtained as a yellow solid without further purification.

$^1$H NMR : (400 MHz, DMSO-d6)

$\delta$ = 7.92 (d, *J* = 9.1 Hz, 4H), 5.27-5.17 (m, 1H), 5.03-4.91 (m, 1H), 4.60-4.50 (m, 1H), 4.09-3.97 (m, 4H), 3.85 (s, 2H), 3.65-3.46 (m, 10H), 3.04-2.92 (m, 2H), 2.10 (s, 3H), 2.00 (s, 3H), 1.94-1.86 (m, 3H), 1.82-1.71 (m, 4H).

**2. Preparation of Intermediate 3-3**

<u>*2.1 Preparation of Compound 5*</u>

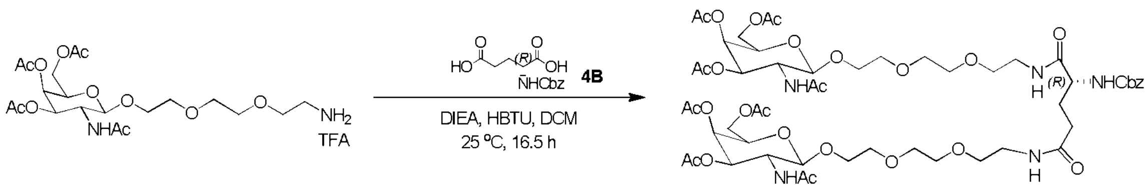


3-4

5

To a solution of Compound 4B (10.0 g, 35.5 mmol, 1.00 eq) and Compound 3-4 (46.3 g, 78.2 mmol, 2.20 eq, TFA) prepared above in DCM (1.00 L), DIEA (30.3 g, 234 mmol, 40.8 mL, 6.60 eq) was added in one portion at 25°C. The mixture was stirred at 25°C for half an hour. HBTU (30.3 g, 234 mmol, 40.8 mL, 6.60 eq) was added to the mixture. The mixture was stirred at 25°C for 16 h until LCMS (product: RT = 0.681 min) showed completion of the reaction. The mixture was subjected to vacuum concentration. The mixture was added with 0.50 N HCl (200 mL × 2) at 20°C, and then extracted with DCM (3 × 500 mL). The combined organic phases were washed with saturated $NaHCO_3$ (3 × 800 mL) until the pH = 8, then washed with brine (3 × 500 mL), dried over $Na_2SO_4$, and subjected to vacuum concentration. The residue was purified by column chromatography ($SiO_2$, DCM: MeOH = 50:1-15:1). The residue was subjected to vacuum concentration at 40°C and purified by preparative-MPLC (column: 800 g Agela C18; mobile phase: [water-ACN]; 15-45%, 25 min; 45%, 10 min). Compound 5 (about 180 g + 75.0 g + 87.0 g + 40.0 g + 38.0 g) was obtained by vacuum drying as a yellow solid.

417.0 g of Compound 3-4 was converted to Compound 5 over 9 batches.

<u>*2.2 Preparation of Intermediate 3-3*</u>

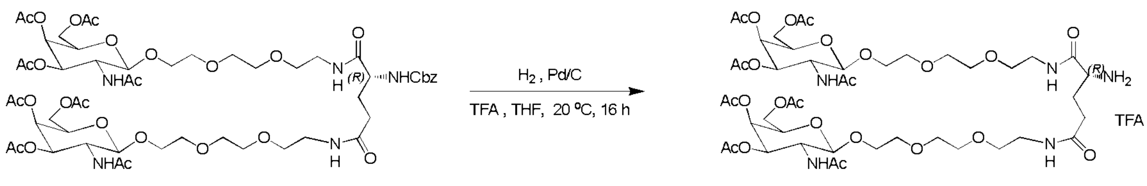


5

3-3

To Pd/C (3.00 g, 10% content) in THF (300 mL), Compound 5 (73.0 g, 61.7 mmol, 1.00 eq) and TFA (7.04 g, 61.7 mmol, 4.57 mL, 1.00 eq) were added under an argon atmosphere. The suspension was degassed and purged three times with hydrogen. The mixture was stirred under $H_2$ (20 Psi) at 20°C for 16 h until TLC (dichloromethane: methanol = 8:1, $R_f$ = 0.0) showed completion of the reaction. The mixture was filtered through celite, and the filtrate was concentrated under pressure to remove the solvent to obtain Compound 3-3 (about 33.4 g + 129 g + 75.0 g) as a white solid.

$^1$H NMR: (400 MHz, DMSO)

$\delta$=8.53 (t, *J* = 5.2 Hz, 1H), 8.18 (d, *J* = 2.4 Hz, 3H), 8.03 (t, *J* = 5.2 Hz, 1H), 7.84 (dd, *J* = 3.6 Hz, 2H), 5.22 (d, *J* = 3.2

Hz, 2H), 4.96 (dd, *J* = 3.2 Hz, 2H), 4.55 (d, *J* =8.4 Hz, 2H), 4.02 (t, *J* =8.8 Hz, 6H), 3.77-3.59 (m, 5H), 3.58-3.45 (m, 21H), 3.40-3.20 (m, 4H), 2.18 (t, *J* = 7.6 Hz, 2H), 2.17 (d, *J* =8.0 Hz, 6H), 2.10 (s, 6H), 1.99 (s, 6H), 1.90-1.80 (m, 8H), 1.77 (s, 6H).

**3. Preparation of Compound E7**

*3.1 Preparation of Compound 3*

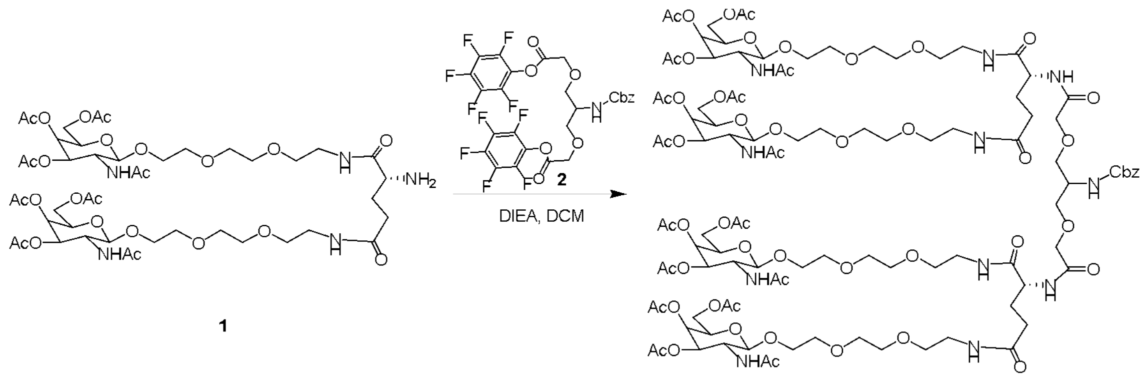


1

3

Compound 1 (2.00 g, 1.87 mmol, prepared as described above for intermediate 3-3) was dissolved in DCM (20.0 mL) at room temperature. DIEA (0.135 mL, 0.814 mmol) and Compound 2 (0.550 g, 0.814 mmol) were added sequentially to the solution, which was then replaced with nitrogen three times. The reaction mixture was stirred at 25°C for 16 h until Liquid Chromatography-Tandem Mass Spectrometry (LC-MS/MS) detected the MS response of the product, and Thin Layer Chromatography (dichloromethane/methanol = 5/1) showed the disappearance of the starting material and formation of a new spot. The reaction mixture was concentrated under reduced pressure. The resulting crude product was purified by column chromatography (dichloromethane/methanol = 5/1) to obtain Compound 3 (about 780 mg) as a white solid.

$^1$H NMR (400 MHz, $CD_3OD$)

$\delta$=7.28-7.42 (m, 5H), 5.30-5.34 (m, 4H), 5.04-5.14 (m, 6H), 4.63-4.67 (m, 4H), 4.36-4.44 (m, 2H), 4.00-4.20 (m, 23H), 3.91-3.95 (m, 4H), 3.69-3.77 (m, 9H), 3.52-3.67 (m, 32H), 3.34-3.43 (m, 9H), 2.29-2.31 (m, 4H), 2.14 (s, 12H), 2.03 (s, 12H), 1.92-1.96 (m, 24H). LCMS: m/z = 1221.6 $(M/2+H)^+$.

*3.2 Preparation of Compound 4*

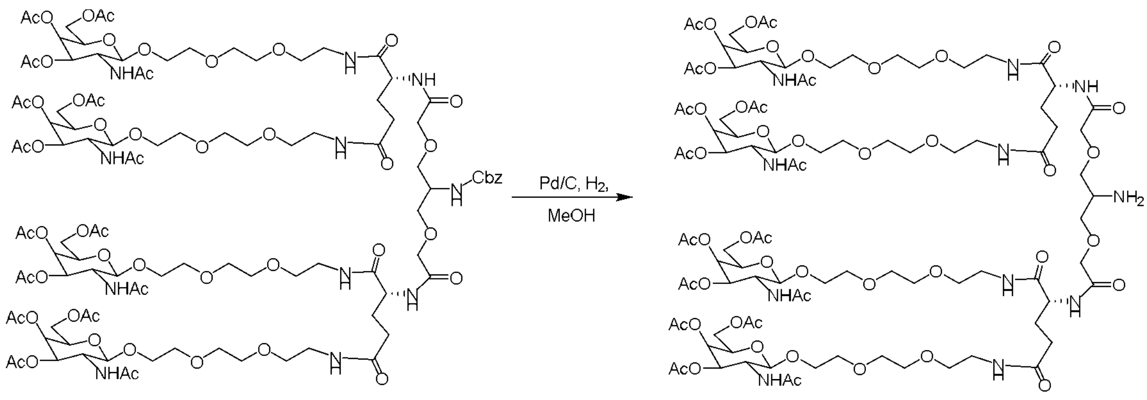


3

4

Compound 3 (1.10 g, 0.451 mmol) was dissolved in MeOH (10.0 mL) at room temperature, and wet Pd/C (0.050 g, 0.451 mmol) with a mass fraction of 10% was added to the solution. The reaction mixture was replaced with hydrogen three times and then stirred at 25°C for 18 h under a hydrogen atmosphere (14.696 psi) until LC-MS/MS detected the MS

response of the product, and TLC (dichloromethane/methanol = 10/1, color development: phosphomolybdic acid) showed complete consumption of the starting material and formation of a new spot. The reaction mixture was filtered and the filtrate was concentrated under reduced pressure to obtain Compound 4 (about 840 mg) as a white solid.

$^1$H NMR (400 MHz, $CD_3OD$)

$\delta$=5.32-5.34 (m 4H), 5.06-5.10 (m, 4H), 4.63-4.65 (m, 4H), 4.38-4.40 (m, 2H), 3.99-4.20 (m, 20H), 3.90-3.97 (m, 4H), 3.69-3.76 (m, 6H), 3.50-3.68 (m, 36H), 3.35-3.44 (m, 11H), 2.28-2.38 (m, 4H), 2.15 (s, 12H), 2.03 (s, 12H), 1.90-1.94 (m, 24H). LCMS: m/z = 1154.7 $(M/2+H)^+$.

*3.3 Preparation of Compound 6*

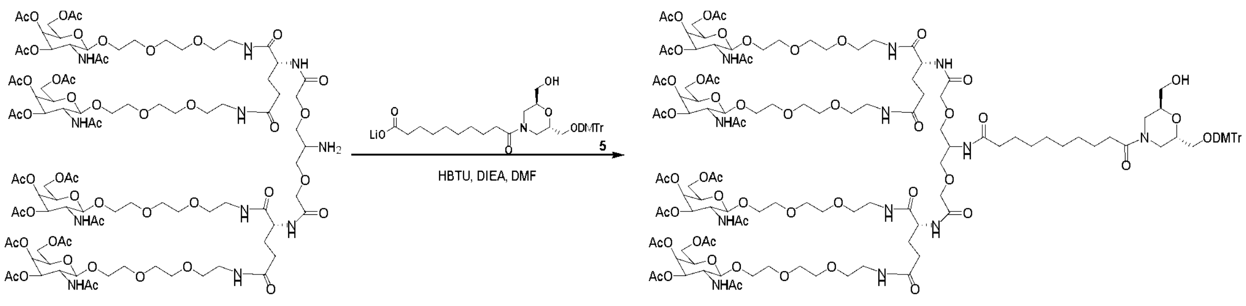


4

6

Compound 5 (232 mg, 0.364 mmol) was dissolved in DCM (10.0 mL) at room temperature, and HBTU (207 mg, 0.546 mmol), DIEA (0.181 mL, 1.09 mmol), and Compound 4 (840 mg, 0.364 mmol) were added sequentially to the solution, which was then replaced with nitrogen three times The reaction mixture was stirred at 25°C for 1 h until LC-MS/MS detected the disappearance of the starting material, and TLC (dichloromethane/methanol = 5/1) showed the disappearance of the starting material and formation of a new spot. The reaction mixture was concentrated under reduced pressure. The resulting crude product was purified by column chromatography (dichloromethane/methanol = 8/1-5/1) to obtain Compound 6 (about 620 mg) as a white solid.

$^1$H NMR (400 MHz, $CD_3OD$)

$\delta$=7.41-7.43 (m, 2H), 7.23-7.34 (m, 7H), 6.83-6.90 (m, 4H), 5.31-5.35 (m, 4H), 5.01-5.12 (m, 4H), 4.63-4.65 (m, 4H), 4.41-4.45 (m, 2H), 4.31-4.33 (m, 1H), 3.99-4.22 (m, 22H), 3.87-3.97 (m, 6H), 3.58-3.81 (m, 45H), 3.34-3.43 (m, 10H), 2.19-2.40 (m, 10H), 2.14 (s, 12H), 2.02 (s, 12H), 1.92-1.96 (mz, 24H), 1.48-1.63 (m, 4H), 1.28-1.38 (m, 8H). LCMS: m/z = 1460.0 $(M/2+H)^+$.

*4. Preparation of Compound E7*

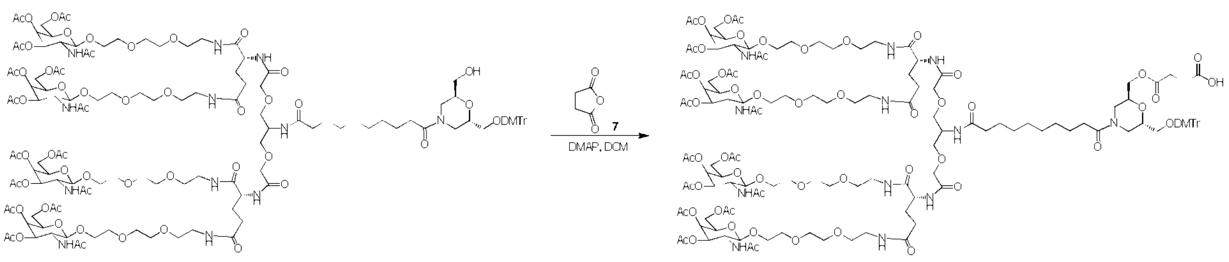


6

E7

Compound 6 (300 mg, 0.103 mmol) was dissolved in DCM (10.0 mL) at room temperature, and DIEA (0.102 mL, 0.618 mmol), Compound 7 (10.3 mg, 0.103 mmol), and DMAP (12.6 mg, 0.103 mmol) were added sequentially to the solution, which was then replaced with nitrogen three times. The reaction mixture was stirred at 25°C for 2 h until LC-MS/MS detected the disappearance of the starting material. The reaction mixture was concentrated under reduced pressure. The resulting crude product was separated by preparative-MPLC (prep-HPLC, column: Waters Xbridge BEH C18 100*30 mm*10 μm; mobile phase: water-ACN; B%: 17%-57%, 5 min) to obtain Compound E7 (53.0 mg, yield 17.08%, purity 78.94%) as a white solid.

$^1H$ NMR (400 MHz, $CD_3OD$)
$\delta$=7.41-7.45 (m, 2H), 7.17-7.34 (m, 7H), 6.85-6.89 (m, 4H), 5.32-5.36 (m, 4H), 5.03-5.13 (m, 4H), 4.63-4.67 (m, 4H), 4.38-4.47 (m, 2H), 4.32-4.34 (m, 1H), 4.01-4.26 (m, 22H), 3.88-4.00 (m, 6H), 3.77-3.81 (m, 7H), 3.49-3.76 (m, 45H), 3.33-3.47 (m, 10H), 2.56-2.62 (m, 2H), 2.45-2.55 (m, 3H), 2.21-2.38 (m, 7H), 2.14 (s, 12H), 2.05-2.11 (m, 2H), 2.02 (s, 12H), 1.92-1.96 (m, 24H), 1.47-1.68 (m, 4H), 1.28-1.34 (m, 8H)
MS: m/z = 3022.36 $(M+H)^+$.

**Example 2 Preparation of siRNA**

The siRNA of the present disclosure was prepared using a solid phase phosphoramidite method well known in the art. Specific methods can be found, for example, in PCT Publication Nos. WO2016081444 and WO2019105419, and are briefly described below.

1. Preparation of siRNA unconjugated to a ligand

*1.1 <u>Synthesis of Sense Strand (SS Strand)</u>*

Through solid-phase phosphoramidite synthesis method, the blank CPG solid-phase support was used as the initial cycle, and nucleoside monomers were linked one by one from 3'-5' direction according to the nucleotide sequence of the sense chain. Each addition of a nucleoside monomer involves four sequential reactions: deprotection, coupling, capping, and oxidation or sulfurization. The synthesis conditions for oligonucleotides at a 5 μmol scale were as follows:
Commercially available phosphoramidites with 2'-F, 2'-O-methyl, and other modifications were used. The nucleoside monomers were supplied as a 0.05 mol/L acetonitrile solution. The reaction conditions for each step were identical: the temperature was 25°C, and deprotection was performed three times using a 3% trichloroacetic acid-dichloromethane solution. For the coupling reaction, the activator used was a 0.25 mol/L ETT-acetonitrile solution, with coupling conducted twice. For capping, 10% acetic anhydride-acetonitrile and pyridine/N-methylimidazole/acetonitrile (10:14:76, v/v/v) were used, with capping performed twice. For oxidation: a 0.05 mol/L iodine/tetrahydrofuran/pyridine/-water solution (70:20:10, v/v/v) was used, with oxidation performed twice. For thiosulfurization, 0.2 mol/L PADS in acetonitrile/3-methylpyridine (1:1, v/v) was used, with thiosulfurization performed twice.

*1.2 <u>Synthesis of Antisense Strand (AS Strand)</u>*

Through solid phase phosphoramidite synthesis method, the blank CPG solid support was used as the initial cycle, and nucleoside monomers were linked one by one from 3'-5' direction according to the nucleotide sequence of antisense strand. Each addition of a nucleoside monomer involves four sequential reactions: deprotection, coupling, capping, and oxidation or sulfurization, and the synthesis conditions for the 5 μmol-scale oligonucleotide of the antisense strand were the same as those for the sense strand.

*1.3 <u>Purification and annealing of oligonucleotides</u>*

1.3.1 Aminolysis

The synthesized solid-phase support (either the sense strand or the antisense strand) was added into a 5 mL centrifuge tube. A 3% diethylamine/ammonia solution (v/v) was added, and the reaction was carried out in a constant-temperature water bath at 35°C for 16 hours (or at 55°C for 8 hours). The mixture was filtered, and the solid-phase support was washed three times with ethanol/water, using 1 mL each time. After the filtrate was centrifuged and concentrated, the crude product was purified.

1.3.2 Purification

The methods for purification and desalting are well-known to those skilled in the art. For example, a strong anionic filler was used to pack the column, and a sodium chloride-sodium hydroxide system was employed for elution and purification, followed by collecting and pooling the products. A gel filler purification column could be used for desalting, with pure water as the elution system.

1.3.3 Annealing

According to Table 6, the sense strand (SS strand) and the antisense strand (AS strand) were mixed in a molar

ratio (SS strand/AS strand = 1/1.05), heated to 70-95°C in a water bath for 3-5 min, naturally cooled to room temperature, and the system was freeze-dried to obtain the product.

The siRNAs in Table 6 were finally obtained.

## 2. Preparation of siRNA with Sense Strand Conjugated to a Ligand

### 2.1 Conjugation of ligand to CPG Support

Conjugation of Compound E7 to CPG Support

Compound E7 (53 mg, 0.018 mmol) and HBTU (13.3 mg, 0.035 mmol) were mixed and dissolved in acetonitrile (5 mL) by shaking. DIEA (9.0 mg, 0.07 mmol) and DMAP (2.1 mg, 0.018 mmol) were then added and dissolved by shaking until clear. Blank carrier Resin (550 mg, CPG pore size 1000Å) was weighed and added to the reaction mixture, which was then placed on a shaker at 20°C overnight. A sample was taken and monitored until Thin Layer Chromatography (TLC) (developing solvent: DCM/methanol = 4/1; color development: phosphomolybdic acid) showed completion of the reaction. The reaction mixture was filtered through a sand core funnel. The filter cake was washed with anhydrous acetonitrile (20 mL*5), collected, and suction-filtered under reduced pressure using an oil pump for 6 h to obtain 530 mg of an off-white solid.

The above condensed product (530 mg) was transferred to a 50 mL round-bottom flask. CapC (DMAP/acetonitrile), CapB (N-methylimidazole/pyridine/acetonitrile), and CapA (acetic anhydride/acetonitrile) were added sequentially. The mixture was then placed on a shaker at room temperature overnight. After filtration, the filter cake was washed with acetonitrile (20 mL*4), collected, and suction-filtered under reduced pressure using an oil pump for 8 h to obtain 200 mg of an off-white solid for solid-phase synthesis.

### 2.2 Synthesis of Sense Strand (SS Strand)

Using the solid-phase phosphoramidite method, the DL0043 solid support prepared above was used as the starting cycle, and nucleoside monomers were linked one by one from the 3' to 5' direction according to the arrangement of sense strand nucleotides. Each linking of a nucleoside monomer involved four steps of deprotection, coupling, capping, and oxidation or thiolation to synthesize 5 umol oligonucleotide with the synthesis conditions as follows:

Nucleoside monomers were provided in a 0.05 mol/L acetonitrile solution. The conditions for each step were the same: temperature 25°C; deprotection for 3 times using a 3% trichloroacetic acid-dichloromethane solution; coupling twice using a 0.25 mol/L ETT-acetonitrile solution as an activator; capping twice using 10% acetic anhydride-acetonitrile and pyridine/N-methylimidazole/acetonitrile (10:14:76, v/v/v); oxidation twice using 0.05 mol/L of iodine in tetrahydrofuran/pyridine/water (70:20:10, v/v/v); thiolation twice using 0.2 mol/L PADS in acetonitrile/3-methylpyridine (1:1, v/v).

### 2.3 Synthesis of Antisense Strand (AS Strand)

Using the solid-phase phosphoramidite method, a blank CPG solid support was used as the starting cycle, and nucleoside monomers were linked one by one from the 3' to 5' direction according to the arrangement of antisense strand nucleotides. Each linkage of a nucleoside monomer involved four steps of deprotection, coupling, capping, and oxidation or thiolation. The conditions for the synthesis of a 5 μmol oligonucleotide for the antisense strand were the same as those for the sense strand.

### 2.4 Purification and annealing of oligonucleotides

#### 2.4.1 Ammonolysis

The synthesized solid support (sense or antisense strand) was transferred to a 5 mL centrifuge tube, followed by the addition of 3% diethylamine/ammonia (v/v). The mixture was allowed to react in a thermostatic water bath at 35°C for 16 h (or 55°C for 8 h), and then filtered. The solid support was washed three times with ethanol/water, 1 mL each time. The filtrate was concentrated by centrifugation, and the crude product was purified.

#### 2.4.2 Purification

The methods for purification and desalting are well known to those skilled in the art. For example, a strong anionic packing column and a sodium chloride-sodium hydroxide system may be used for elution and purification. The product can be collected in tubes and desalted using a gel packing purification column, with pure water as the eluent.

2.4.3 Annealing

According to Table 7, the sense strand (SS Strand) was mixed with the antisense strand (AS Strand) at a molar ratio (SS Strand/AS Strand = 1/1.05). The mixture was heated in a water bath to 70-95°C, held for 3-5 min, and then allowed to cool naturally to room temperature. The system was freeze-dried to obtain the product. The siRNAs in Table 7 were finally obtained, with DR005677 used as a control.

**Example 3 Screening for Activity in Huh7 Cell Line**

*Cell transfection*

On the first day, the Huh7 cell line was digested and resuspended and counted. The cell resuspension was plated onto 96-well plates at 100 μL/well (1 x $10^4$ cells/well), and transfection was performed after 18 h.

On Day 2, 20 μM of siRNA stock solution was diluted with Opti-MEM. 198 μL of Opti-MEM was added to 2 μL of siRNA stock solution. The final concentration of siRNA was shown below. And the solutions were mixed well by pipetting and until used.

On Day 2, 9 μL of RNAiMAX (Thermo, 13778150) was diluted with 14.1 μL of Opti-MEM, mixed gently by pipetting, and allowed to stand at room temperature for 5 min. Then, 15 μL of the prepared RNAi-MAX mixture and 15 μL of the diluted siRNA were mixed gently by pipetting (avoiding bubbles), and allowed to stand at room temperature for 10 min. The mixture was transferred to a 96-well plate at 10 μL/well. The plate was extracted after incubation in a 5% CO2 incubator at 37°C for 24 h (control group was not added with siRNA).

*RNA extraction*

Cellular RNA was extracted using a nucleic acid extractor (Hangzhou Allsheng, Auto-pure96) according to the protocol of the high-throughput cell RNA extraction kit (FireGen, FG0417-L).

*RNA reverse transcription*

The denatured reaction mixture was prepared with reference to PrimeScript™ II 1st Strand cDNA Synthesis Kit (Takara, 6210B). single well formulation volume: 1 μL of Oligo dT Primer, 1 μL of dNTP Mixture, and 12.5 μL of template RNA. The reaction was incubated in a conventional PCR instrument at 65°C for 5 min and then rapidly cooled on ice for 2 min.

The reverse transcription reaction mixture was prepared with reference to PrimeScript™ II 1st Strand cDNA Synthesis Kit (Takara, 6210B). Each well contained 4 μL of 5×Prime Script II Buffer, 0.5 μL of RNase Inhibitor, and 1 μL of PrimeScript II RTase.

The denatured reaction mixture (14.5 μL) was mixed gently with the reverse transcription reaction mixture. Reverse transcription was performed by incubation in a conventional PCR instrument at 42°C for 45 min, followed by incubation at 95°C for 5 min to inactivate the enzyme. The reverse transcription product (cDNA) was then cooled at 4°C.

After reverse transcription, 30 μL of DNase/RNase-Free Distilled Water was added to the cDNA sample in each well.

*Fluorescent quantitative PCR*

Fluorescence quantitative PCR reaction was performed in a 20 μL system (ABI, QuantStudio3) with reference to the procedure of TaqMan™ Fast Advanced Master Mix (ABI, 4444965). The reaction program was: (50°C, 2 min) × 1 Cycle; (95°C, 20 s) × 1 Cycle; (95°C, 1 s; 60°C, 24 s) × 40 Cycles.

Table 8. Primer Information

| Primer name | Sequence Information (5'-3') | SEQ ID NO | Fluorophore |
|---|---|---|---|
| hACTB-PF | ACGTGGACATCCGCAAAGAC | 107 | / |
| hACTB-PR | TCTTCATTGTGCTGGGTGCC | 108 | / |
| hACTB-P | AACACAGTGCTGTCTGGCGGCACCA | 109 | 5'TET, 3'BHQ2 |
| hAGT-PF | TGTGAGCAGCTGGCAAAGG | 110 | / |
| hAGT-PR | GATGTCTTGGCCTGAATTGGA | 111 | / |

(continued)

| Primer name | Sequence Information (5'-3') | SEQ ID NO | Fluorophore |
|---|---|---|---|
| hAGT-P | CAATGCCGGGAAGC | 112 | 5'6-FAM, 3'MGB |

*Data statistics*

Calculation of $2^{-\triangle\triangle Ct}$ values and conversion to percentages to obtain residual inhibition rate;

△△Ct = [(Ct of target gene of experimental group - Ct of endogenous reference of experimental group) - (Ct of target gene of control group - Ct of endogenous reference of control group)].

The target gene was hAGT, and the internal reference was hACTB.
The final concentration of siRNA was 10 nM for siRNA compound cell line activity screening, and the experimental screening results are shown in Table 9.

Table 9. Huh7 cell line activity screening results

| Compound Number | Residual inhibition rate | SD |
|---|---|---|
| DR005325 | 15.50% | 0.61% |
| DR005321 | 13.62% | 2.65% |
| DR005328 | 15.63% | 3.42% |
| DR005048 | 10.50% | 1.59% |
| DR005390 | 12.91% | 1.70% |
| DR005391 | 15.08% | 2.57% |
| DR005392 | 17.28% | 1.35% |
| DR005399 | 13.44% | 3.13% |
| DR005400 | 14.90% | 1.39% |
| DR005401 | 14.92% | 0.29% |
| DR005412 | 16.61% | 0.41% |
| DR005415 | 14.88% | 0.95% |
| DR005416 | 13.68% | 0.20% |
| DR005417 | 15.21% | 2.23% |
| DR005418 | 14.34% | 2.17% |
| DR005419 | 15.83% | 0.54% |
| DR005254 | 11.91% | 0.35% |
| DR005196 | 18.25% | 6.73% |
| DR005389 | 13.10% | 1.15% |

**Example 4 Screening for Activity in a Human Primary Hepatocyte (PHH Cell)**

*Cell transfection*

1.4 mL of rat tail collagen solution (Sigma, C3867) was added to 40.6 mL of DNase/RNase-Free Distilled Water and mixed well. The mixture was added to a 96-well culture plate at 40 μL/well, and coated overnight at 4°C. The coating medium was discarded the next day.
On Day 2, prior to use, the coated cell plate was rinsed with DPBS, which was then aspirated. PHHs (Shanghai Xuanyi Biotechnology Co., Ltd., Cat#: QYLF-HPMC) were recovered at 37°C, transferred into the recovery medium, centrifuged, resuspended, and counted. PHHs were plated in a 96-well plate at 90 μL/well (2×104 cells/well). The

complete medium was replaced after 4 h, and transfection was performed after 18 h.

On Day 3, the 20 μM siRNA stock solution was diluted with Opti-MEM. 198 μL of Opti-MEM was added to 2 μL of 20 μM siRNA stock solution and mixed well by pipetting, as the first concentration point. Serial dilutions were made as required for the experiment.

On Day 3, 0.9 μL of RNAiMAX (Thermo, 13778150) was diluted with 14.1 μL of Opti-MEM, mixed gently by pipetting, and allowed to stand at room temperature for 5 min. Then, 15 μL of the prepared RNAi-MAX mixture and 15 μL of the diluted compound were mixed gently by pipetting (avoiding bubbles), and allowed to stand at room temperature for 10 min. The mixture was transferred to a 96-well plate at 10 μL/well. The plate was extracted after incubation in a 5% CO2 incubator at 37°C for 24 h before RNA extraction.

*RNA extraction*

Cellular RNA was extracted using a nucleic acid extractor (Hangzhou Allsheng, Auto-pure96) according to the protocol of the high-throughput cell RNA extraction kit (FireGen, FG0412).

*RNA reverse transcription*

The denatured reaction mixture was prepared with reference to PrimeScript™ II 1st Strand cDNA Synthesis Kit (Takara, 6210B). Each well contained 1 μL of Oligo dT Primer, 1 μL of dNTP Mixture, and 12.5 μL of template RNA. The denaturation reaction was performed by incubation in a conventional PCR instrument at 65°C for 5 min. The mixture was rapidly cooled on ice for 2 min.

The reverse transcription reaction mixture was prepared with reference to PrimeScript™ II 1st Strand cDNA Synthesis Kit (Takara, 6210B). Each well contained 4 μL of 5×Prime Script II Buffer, 0.5 μL of RNase Inhibitor, and 1 μL of PrimeScript II RTase.

The denatured reaction mixture (14.5 μL) was mixed gently with the reverse transcription reaction mixture. Reverse transcription was performed by incubation in a conventional PCR instrument at 42°C for 45 min, followed by incubation at 95°C for 5 min to inactivate the enzyme. The reverse transcription product (cDNA) was then cooled at 4°C.

After reverse transcription, 30 μL of DNase/RNase-Free Distilled Water was added to the cDNA sample in each well.

*Fluorescent quantitative PCR*

Fluorescence quantitative PCR reaction was performed in a 20 μL system (ABI, QuantStudio3) with reference to the procedure of TaqMan™ Fast Advanced Master Mix (ABI, 4444965). The reaction program was: (50°C, 2 min) × 1 Cycle; (95°C, 20 s) × 1 Cycle; (95°C, 1 s; 60°C, 24 s) × 40 Cycles.

Table 10 Primer Information

| Primer name | Sequence Information (5'-3') | SEQ ID NO | Fluorophore |
|---|---|---|---|
| hACTB-PF | ACGTGGACATCCGCAAAGAC | 107 | / |
| hACTB-PR | TCTTCATTGTGCTGGGTGCC | 108 | / |
| hACTB-P | AACACAGTGCTGTCTGGCGGCACCA | 109 | 5' TET, 3' BHQ2 |
| hAGT-PF | TGTGAGCAGCTGGCAAAGG | 110 | / |
| hAGT-PR | GATGTCTTGGCCTGAATTGGA | 111 | / |
| hAGT-P | CAATGCCGGGAAGC | 112 | 5' 6-FAM, 3' MGB |

*Data statistics*

Calculation of $2^{-\triangle\triangle 66Ct}$ values and conversion to percentages to obtain residual inhibition rate.

△△Ct = [(Ct of target gene of experimental group - Ct of endogenous reference of experimental group) - (Ct of target gene of control group - Ct of endogenous reference of control group)].

The initial concentration of siRNA was 10 nM, and 5 concentration points (10 nM, 1 nM, 0.1 nM, 0.01 nM, 0.001 nM) were obtained by 5-fold gradient dilution. The activity of siRNA in human liver primary cells was screened. The

screening results are shown in Table 10, in which the residual inhibition rates are listed in columns 2 to 6, and the IC50 values are listed in column 7.

Table 11 Results of human primary hepatocyte screening using siRNA at 5 concentrations

| Compound Number | 10nM | 1nM | 0.1nM | 0.01nM | 0.001nM | IC50 value (nM) |
|---|---|---|---|---|---|---|
| DR005677 | 16.12% | 21.68% | 22.61% | 43.38% | 72.65% | 0.0061 |
| DR005048 | 14.24% | 23.84% | 44.96% | 96.28% | 119.63% | 0.0821 |
| DR005196 | 35.49% | 33.33% | 59.10% | 108.51% | 148.29% | 0.1108 |
| DR005254 | 27.36% | 30.15% | 55.64% | 106.77% | 127.38% | 0.1154 |
| DR005321 | 20.06% | 20.00% | 43.79% | 70.97% | 74.29% | 0.0722 |
| DR005325 | 15.82% | 12.08% | 29.17% | 44.19% | 69.25% | 0.0064 |
| DR005328 | 24.07% | 25.94% | 45.09% | 93.99% | 108.14% | 0.0776 |
| DR005389 | 23.58% | 20.03% | 50.56% | 92.52% | 128.75% | 0.0864 |
| DR005390 | 27.76% | 24.17% | 41.04% | 95.05% | 136.13% | 0.0549 |
| DR005391 | 26.96% | 23.68% | 46.70% | 100.48% | 117.41% | 0.0827 |
| DR005392 | 23.67% | 31.06% | 53.78% | 113.54% | 136.15% | 0.1109 |
| DR005399 | 17.94% | 25.89% | 37.00% | 49.46% | 74.46% | 0.0100 |
| DR005400 | 38.90% | 30.45% | 44.57% | 89.73% | 107.01% | 0.0639 |
| DR005401 | 22.13% | 25.40% | 28.04% | 70.13% | 105.92% | 0.0210 |
| DR005412 | 16.68% | 34.20% | 54.22% | 102.71% | 95.49% | 0.1061 |
| DR005415 | 34.94% | 29.54% | 50.69% | 123.49% | 113.78% | 0.1006 |
| DR005416 | 24.76% | 21.37% | 49.66% | 81.66% | 104.12% | 0.0808 |
| DR005417 | 24.03% | 17.44% | 33.94% | 72.35% | 85.23% | 0.0363 |
| DR005418 | 17.82% | 17.65% | 26.84% | 53.46% | 92.35% | 0.0124 |
| DR005419 | 22.73% | 23.36% | 33.17% | 79.87% | 136.41% | 0.0313 |

**Example 5 Screening for Off-target Activity in psiCHECK2 GSSM-5Hits**

*Plasmid Preparation*

According to the siRNA sequence, the corresponding antisense strand off-target plasmid was designed. The psiCHECK2 GSSM-5Hits recombinant plasmid was prepared by Sangon Biotech (Shanghai) Co., Ltd., and the recombinant plasmid was diluted to 1000 ng/μL for use.

*Cell transfection*

100 μL of HEK293A cells (Cobioer Biosciences Co., LTD., Cat. No. CBP60436) resuspension/well of a 96-well plate was plated (8 x $10^3$ cells/well).

The next day, the complete medium in the wells was aspirated and then replaced with Opti-MEM medium at 80 μL/well, and cells starved for approximately 1.5 h.

siRNA preparation: siRNA was diluted 3-fold from the final concentration of 40nM for 11 concentration points (10 nM, 3.3333 nM, 1.1111 nM, 0.37037 nM, 0.12346 nM, 0.04115 nM, 0.01372 nM, 0.00457 nM, 0.00152 nM, 0.00051 nM, and 0.00017 nM).

Preparation of plasmid mixture: The amount of single well preparation was 0.01 μL/well of plasmid and 8.99 μL/well of Opti-MEM.

Preparation of Lipo mixture: 0.2 μL of Lipo 2000 and 9.8 μL of Opti-MEM were added to each well to dilute Lipo 2000 (Lipofectamine ™ 2000 Transfection Reagent, Thermo, 11668019) with Opti-MEM to obtain a Lipo mixture and let it stand at room temperature for 5 min.

22 μL of the prepared Lipo mixture, 2.2 μL of siRNA, and 19.8 μL of the plasmid mixture were separately split into

the corresponding same well, and designated as "well A" mixture. After mixing by pipetting, co-transfection was performed after incubation at room temperature for 20 min. The "well A" mixture was added to each well of cells at 20 μL/well. With the original 80 μL of Opti-MEM, the final volume was 100 μL per well. After incubation in a 5% CO2 incubator at 37 °C for 4 h, 100 μL of DMEM medium containing 20% fetal bovine serum was added to each well. The plate was extracted after incubation in a 5% CO2 incubator at 37°C for 24 h before detection.

*Results Detection*

The mixed Dual-Glo®Luciferase (Dual-Glo® Luciferase Assay System, Promega, E2940) was thawed before the experiment. After equilibration to room temperature, DMEM was added to each tube at a ratio of 1: 1 to formulate substrate I, which was freshly prepared for use. Dual-Glo® Stop & Glo® Buffer was thawed,. After equilibrium to room temperature, it was mixed with Dual-Glo® Stop & Glo®Substrate at a ratio of 100: 1 to formulate substrate II, which was freshly prepared for use. The old medium in the 96-well culture plate was aspirated with a vacuum pump. 150 μL of Substrate I/well was added and incubated on a shaker at room temperature for 10 min. 120 μL of Substrate I was transferred to a 96-well microplate and the Firefly chemiluminescence value was read on a microplate reader (Tecan, Infinite 200). another 60 μL of Substrate II/well was added and incubate on a shaker at room temperature for 10 min, and then the Renilla chemiluminescence value was read on a microplate reader.

*Data analysis and processing*

The fluorescence activity is measured by a microplate reader, the collected Renilla signal is normalized by the Firefly signal standard, and the inhibitory effect of siRNA is obtained by comparing with the untreated results (residual inhibitory activity). The calculation process is:

$$\text{Normalized Ren/Fir Ratio: Ratio} = \text{Renilla (Renilla luciferase)/Firefly (Firefly luciferase).}$$

Residual inhibition rate = (RatiosiRNA/ Ratiocontrol) * 100%, calculated as the mean of two replicate wells: where Ratiocontrol is the Ratio value of the control well (without siRNA) (calculated as the mean of two replicate wells).

Drawing: Drawing with Graphpad Prism

Half maximal inhibitory concentration (IC50): in this experiment, Top and Bottom are plotted, and the IC50 value is obtained according to the formula Y=Bottom+(Top-Bottom)/(1+10^((LogIC50-X)*HillSlope)), where Y = 50, X = log (concentration).

The results of screening for off-target activity of siRNA in psiCHECK2 GSSM-5Hits are shown in Table 12, in which the residual inhibition rates are listed in columns 2 to 12, and the IC50 values are listed in column 13.

**Table 12 Results of Sreening for Off-target Activity in psiCHECK2 GSSM-5Hits**

| Compound No. | 10n M | 3.3333 nM | 1.1111 nM | 0.3703 7nM | 0.1234 6nM | 0.0411 5nM | 0.0137 2nM | 0.0045 7nM | 0.0015 2nM | 0.0005 1nM | 0.000 17nM | Fitted IC50 (nM) in GSSM -5Hits |
|---|---|---|---|---|---|---|---|---|---|---|---|---|
| DR005 048 | 7.63 % | 5.49% | 4.25% | 6.56% | 19.04% | 55.53% | 80.53% | 90.22% | 93.65% | 95.49% | 92.84 % | 0.044 |
| DR005 196 | 25.3 1% | 21.10 % | 28.15 % | 65.51% | 97.35% | 101.00 % | 106.32 % | 102.24 % | 104.88 % | 104.98 % | 105.9 1% | 0.736 |
| DR005 254 | 6.35 % | 7.47% | 25.32 % | 58.92% | 106.80 % | 95.40% | 100.74 % | 128.32 % | 111.03 % | 66.21% | 86.43 % | 0.542 |
| DR005 321 | 58.6 8% | 76.69 % | 75.58 % | 90.22% | 88.35% | 111.35 % | 106.68 % | 89.64% | 79.79% | 93.10% | 90.98 % | 18.390 |
| DR005 325 | 71.7 7% | 85.73 % | 87.35 % | 110.45 % | 99.31% | 109.38 % | 97.01% | 102.15 % | 91.02% | 90.45% | 89.57 % | 44.890 |
| DR005 328 | 22.6 8% | 16.52 % | 14.95 % | 40.22% | 64.78% | 88.26% | 94.29% | 93.01% | 101.98 % | 92.86% | 110.4 4% | 0.247 |
| DR005 389 | 21.1 8% | 32.90 % | 74.12 % | 103.39 % | 109.02 % | 109.29 % | 112.29 % | 111.50 % | 109.35 % | 109.67 % | 103.0 8% | 2.415 |
| DR005 390 | 47.1 2% | 52.99 % | 72.58 % | 99.53% | 105.93 % | 110.71 % | 105.98 % | 109.58 % | 108.99 % | 109.92 % | 97.87 % | 5.766 |
| DR005 391 | 84.4 % | 87.2% | 100.7 % | 103.9% | 101.9% | 102.3% | 101.7% | 104.4% | 102.0% | 112.0% | 103.7 % | 54.360 |
| DR005 392 | 69.8 % | 71.6% | 88.7% | 100.0% | 106.2% | 107.7% | 104.2% | 106.4% | 107.6% | 112.1% | 112.7 % | 22.320 |
| DR005 399 | 17.2 % | 14.6% | 28.3% | 64.2% | 91.9% | 112.7% | 111.8% | 109.0% | 111.1% | 111.1% | 108.8 % | 0.630 |
| DR005 400 | 52.1 % | 44.1% | 50.1% | 75.4% | 96.7% | 107.8% | 108.4% | 108.8% | 111.4% | 116.6% | 115.3 % | 3.511 |
| DR005 401 | 38.6 % | 31.1% | 41.9% | 68.1% | 98.5% | 110.6% | 112.9% | 112.6% | 109.6% | 110.9% | 109.9 % | 1.469 |
| DR005 412 | 77.7 % | 93.0% | 109.1 % | 119.4% | 109.6% | 113.3% | 104.3% | 96.8% | 106.5% | 94.6% | 108.7 % | 19.980 |
| DR005 415 | 47.0 % | 42.3% | 53.4% | 73.9% | 89.3% | 105.2% | 109.7% | 104.5% | 112.8% | 104.1% | 99.5 % | 2.988 |
| DR005 417 | 22.6 % | 11.8% | 8.4% | 10.9% | 31.6% | 80.1% | 97.3% | 97.3% | 104.7% | 102.7% | 99.9 % | 0.086 |
| DR005 418 | 72.3 % | 78.8% | 85.4% | 88.6% | 97.5% | 99.5% | 99.7% | 101.1% | 94.6% | 99.2% | 95.6 % | 75.300 |

(continued)

| Compound No. | 10n M | 3.3333 nM | 1.1111 nM | 0.3703 7nM | 0.1234 6nM | 0.0411 5nM | 0.0137 2nM | 0.0045 7nM | 0.0015 2nM | 0.0005 1nM | 0.000 17nM | Fitted IC50 (nM) in GSSM -5Hits |
|---|---|---|---|---|---|---|---|---|---|---|---|---|
| DR005 419 | 70.2 % | 68.3% | 84.5% | 98.8% | 103.0% | 104.7% | 109.8% | 107.4% | 99.7% | 105.1% | 96.1 % | 23.200 |
| DR005 677 | 24.6 % | 18.2% | 27.4% | 63.0% | 91.3% | 101.0% | 96.2% | 104.1% | 100.6% | 101.4% | 99.0 % | 1.801 |

**Example 6 Activity Detection in AAV Humanized Mouse Model**

AAV8 virus carrying the human-derived *AGT* gene fragment (purchased from OBiO Tech) was injected into mice via the tail vein at a dose of 1 x $10^{11}$ vg. The virus injection day was designated as Day -14. On Day -1, the serum of mice was taken, and AGT protein was detected by ELISA (detection kit was purchased from Abcam Company, Cat #ab267592) as the baseline. 3 mice per group were devided for dosing. The siRNA drug was administered via tail vein injection on Day 0 at a dose of 1 mg/kg, and serum were taken on Days 7, 14, 28, and 42 (D7, D14, D28, D42) after dosing for the detection of AGT protein levels using the above kit, and the residual rate was calculated with a baseline level of 100%.

Table 13 Results of activity evaluation in AAV mice

| **Compound Number** | **Residual Rate Mean% on D7** | **Residual Rate Mean% on D14** | **Residual Rate Mean% on D28** | **Residual Rate Mean% on D42** |
|---|---|---|---|---|
| DR005677 | 9.71 | 7.94 | 9.42 | 18.64 |
| DR005822 | 8.56 | 7.79 | 12.89 | 26.51 |
| DR005823 | 25.35 | 37.27 | NA | NA |
| DR005824 | 24.23 | 23.47 | NA | NA |
| DR005825 | 8.83 | 7.19 | 13.71 | 29.77 |
| DR005826 | 5.73 | 4.41 | 5.16 | 8.15 |
| DR005827 | 4.23 | 3.05 | 4.94 | 11.84 |
| DR005828 | 7.34 | 8.73 | 11.03 | 25.74 |
| DR005829 | 7.15 | 4.76 | 4.89 | 11.87 |
| DR005830 | 7.29 | 4.52 | 4.59 | 15.32 |
| DR005831 | 7.33 | 4.35 | 6.18 | 13.81 |
| DR005832 | 9.46 | 13.05 | 21.54 | 33.94 |
| DR005833 | 4.85 | 3.39 | 3.88 | 9.58 |
| DR005834 | 5.33 | 3.5 | 4.05 | 7.08 |
| DR005835 | 6.72 | 5.35 | 8.67 | 21.18 |
| DR005836 | 7.67 | 4.67 | 5.8 | 9.11 |
| DR005837 | 4.19 | 2.36 | 3.09 | 5.42 |
| DR005838 | 16.97 | 15.21 | NA | NA |
| DR005839 | 4.97 | 5.31 | 7.19 | 20.16 |
| DR005840 | 7.29 | 4.44 | 5.79 | 13.48 |

**Example 7 In vivo Activity Evaluation in Tansgenic Mouse Model**

After the adaptation period of male C57BL/6J transgenic mice, blood was collected from each mouse through the orbital vein on Day -4 before dosing (D-4), and serum samples were collected after centrifugation. The level of human AGT protein in the serum of each mouse was detected by ELISA assay as the experimental baseline, and the mice were grouped according to the baseline level (4 mice in each group) for dosing. On the day of dosing(D0), mice in each group were administered, respectively, normal saline or siRNA of the present disclosure by subcutaneous injection at a dose of 1 or 3 mg/kg (mpk) and a dosing volume of 5 ml/kg. Mouse serum was collected on Days 7, 14, 21, 28, 35, 42, 56, and 70 post-dose, respectively, and human AGT protein levels were measured by ELISA assay (assay kit purchased from Abcam, Cat. No. ab267592). The siRNA knockdown efficiency was calculated by comparing human AGT protein levels in the serum of each mouse before and after dosing. The experimental results are shown in Table 14.

Table 14 Results of activity evaluation of siRNA compounds in human AGT transgenic mice

| | Mean Residual Rate (%) | | | | | | | |
|---|---|---|---|---|---|---|---|---|
| Group | D7 | D14 | D21 | D28 | D35 | D42 | D56 | D70 |
| DR005677_1mpk | 14.43 | 14.49 | 17.58 | 22.33 | 32.92 | 43.96 | 53.11 | 70.40 |
| DR005677_3mpk | 7.64 | 7.07 | 7.38 | 9.86 | 13.17 | 16.79 | 24.04 | 46.83 |
| DR009219_1mpk | 10.35 | 9.36 | 9.05 | 10.48 | 12.30 | 15.87 | 17.39 | 29.41 |
| DR009219_3mpk | 7.49 | 6.64 | 5.91 | 8.36 | 8.60 | 9.87 | 10.69 | 15.38 |
| DR009635_1mpk | 43.50 | 51.27 | ND | ND | ND | ND | ND | ND |
| DR009635_3mpk | 17.37 | 17.08 | ND | ND | ND | ND | ND | ND |
| DR009637_1mpk | 16.71 | 15.88 | 14.64 | ND | ND | ND | ND | ND |
| DR009637_3mpk | 8.56 | 6.82 | 5.93 | ND | ND | ND | ND | ND |
| DR009631_1mpk | 21.16 | 25.19 | 30.21 | ND | ND | ND | ND | ND |
| DR009634_1mpk | 20.82 | 23.14 | 23.39 | 27.87 | ND | ND | ND | ND |
| DR009221_1mpk | 13.54 | 13.45 | 12.50 | 15.42 | 17.62 | 19.60 | 24.35 | 47.15 |
| ND means not detected | | | | | | | | |

**Example 8 In vivo Pharmacodynamic Evaluation of siRNA in Diet-induced Hypertensive Cynomolgus Monkeys**

16 male hypertensive cynomolgus monkeys were divided into 4 groups according to serum AGT level, body weight and blood pressure. A single subcutaneous injection of normal saline (i.e. vehicle group) or the siRNA of the present disclosure was administered at a dose of 10 mg/kg and a dosing volume of 1 ml/kg. Serum was collected on Day-6 before dosing (D-6), the day of dosing (D0), and Days 3, 7, 14, 21, 28, 35, 42, 55, 70, 77, 84, 91, 98, and 105 after dosing. The level of AGT protein in serum was detected by ELISA assay (IBL-Japan, Cat. No.: 27412), and blood pressure was measured weekly for selected left wrist or tail base of the animal using a high-definition oscilloscope (HDO) equipment (with a tail sleeve device). The siRNA knockdown efficiency was calculated by comparing AGT protein levels in serum on the day of dosing and at each time points after dosing of each monkey. The magnitude of reductions in serum AGT protein, systolic blood pressure (SBP), diastolic blood pressure (DBP), and mean arterial pressure (MAP) by compounds in each group are shown in FIGs. 1, 2, 3, and 4.

The results showed that compounds in each group achieved a very high inhibition rate of serum AGT protein (maximum inhibition rate > 98%). On Day 105 after dosing, DR009219 and DR009221 still had an inhibition rate of more than 90%. After Day 14, all compounds in each group significantly reduced systolic blood pressure, with the maximum decrease ranging from 40 to 50 mmHg. On Day 105 after dosing, the systolic blood pressure of monkeys in each administration group was still significantly lower than that of the control group. Compounds in each group significantly reduced diastolic blood pressure, with the maximum decrease ranging from 20 to 30 mmHg. On day 105 after administration, the diastolic blood pressure of monkeys in each administration group was still significantly lower than that in the control group.

## Claims

1. A double-stranded nucleotide (dsRNA) for inhibiting the expression of angiotensinogen (AGT) in a cell, the dsRNA comprises a sense strand and an antisense strand that form a double-stranded region, wherein the sense strand and the antisense strand are each independently 15-30 nucleotides in length, and the antisense strand comprises a nucleotide sequence of at least 15 contiguous nucleotides from the nucleotide sequence set forth in any one of SEQ ID NOs: 21-40.

2. The dsRNA of claim 1, wherein the sense strand comprises a nucleotide sequence of at least 15 contiguous nucleotides from the nucleotide sequence set forth in any one of SEQ ID NOs: 1-20.

3. The dsRNA of claim 1 or 2, wherein the dsRNA is an siRNA.

4. The dsRNA of any one of claims 1-3, wherein the double-stranded region is 15-25 nucleotide pairs, preferably 16-23 nucleotide pairs, or more preferably 18-20 nucleotide pairs in length.

5. The dsRNA of any one of claims 1-4, wherein the antisense strand comprises a nucleotide sequence of at least 16 contiguous nucleotides, a nucleotide sequence of at least 17 contiguous nucleotides, a nucleotide sequence of at least 18 contiguous nucleotides, a nucleotide sequence of at least 19 contiguous nucleotides, or a nucleotide sequence of at least 20 contiguous nucleotides from the nucleotide sequence set forth in any one of SEQ ID NOs: 21-40, and preferably the antisense strand comprises the nucleotide sequence set forth in any one of SEQ ID NOs: 21-40.

6. The dsRNA of any one of claims 1-5, wherein the sense strand comprises a nucleotide sequence of at least 16 contiguous nucleotides, a nucleotide sequence of at least 17 contiguous nucleotides, or a nucleotide sequence of at least 18 contiguous nucleotides from the nucleotide sequence set forth in any one of SEQ ID NOs: 1-20, and preferably the sense strand comprises the nucleotide sequence set forth in any one of SEQ ID NOs: 1-20.

7. The dsRNA of any one of claims 1-6, wherein the siRNA comprises any of the following paired of sense strand sequences and antisense strand sequences:

(1) sense strand: SEQ ID NO: 1, antisense strand: SEQ ID NO: 21;
(2) sense strand: SEQ ID NO: 2, antisense strand: SEQ ID NO: 22;
(3) sense strand: SEQ ID NO: 3, antisense strand: SEQ ID NO: 23;
(4) sense strand: SEQ ID NO: 4, antisense strand: SEQ ID NO: 24;
(5) sense strand: SEQ ID NO: 5, antisense strand: SEQ ID NO: 25;
(6) sense strand: SEQ ID NO: 6, antisense strand: SEQ ID NO: 26;
(7) sense strand: SEQ ID NO: 7, antisense strand: SEQ ID NO: 27;
(8) sense strand: SEQ ID NO: 8, antisense strand: SEQ ID NO: 28;
(9) sense strand: SEQ ID NO: 9, antisense strand: SEQ ID NO: 29;
(10) sense strand: SEQ ID NO: 10,antisense strand: SEQ ID NO: 30;
(11) sense strand: SEQ ID NO: 11,antisense strand: SEQ ID NO: 31;
(12) sense strand: SEQ ID NO: 12,antisense strand: SEQ ID NO: 32;
(13) sense strand: SEQ ID NO: 13,antisense strand: SEQ ID NO: 33;
(14) sense strand: SEQ ID NO: 14,antisense strand: SEQ ID NO: 34;
(15) sense strand: SEQ ID NO: 15,antisense strand: SEQ ID NO: 35;
(16) sense strand: SEQ ID NO: 16,antisense strand: SEQ ID NO: 36;
(17) sense strand: SEQ ID NO: 17,antisense strand: SEQ ID NO: 37;
(18) sense strand: SEQ ID NO: 18,antisense strand: SEQ ID NO: 38;
(19) sense strand: SEQ ID NO: 19,antisense strand: SEQ ID NO: 39; and
(20) sense strand: SEQ ID NO: 20,antisense strand: SEQ ID NO: 40.

8. The dsRNA of any one of claims 1-7, wherein substantially all nucleotides of the sense strand and substantially all nucleotides of the antisense strand are modified nucleotides, or all nucleotides of the sense strand and all nucleotides of the antisense strand are modified nucleotides.

9. The dsRNA of any one of claims 1-8, wherein each of the sense strand and the antisense strand independently comprises one or more nucleotide modifications selected from the group consisting of a 2'-O-methyl modification, a 2'-fluoro modification, a SCP modification, a glycol modification, and a phosphorothioate internucleotide linkage modification.

10. The dsRNA of any one of claims 1-9, wherein the antisense strand has a length of 21 nucleotides and has

(i) 2'-O-methyl modified nucleotides at positions 1, 3, 5, 9, 11, 13, 15, 17, 19, and 21, and 2'-fluoro modified nucleotides at positions 2, 4, 6, 8, 10, 12, 14, 16, 18, and 20 (numbered from the 5' end); and/or
(ii) phosphorothioate internucleotide linkages between nucleotide positions 1 and 2, between nucleotide positions 2 and 3, between nucleotide positions 19 and 20, and between nucleotide positions 20 and 21 (numbered from the 5' end).

11. The dsRNA of any one of claims 1-9, wherein the antisense strand has a length of 21 nucleotides and has

(i) 2'-O-methyl modified nucleotides at positions 3, 4, 6, 8, 9, 10, 11, 13, 15, 16, 17, 18, 19, 20, and 21 (numbered from the 5' end);
(ii) a 2'-fluoro modified nucleotide at position 14 (numbered from the 5' end);
(iii) 2'-deoxy modifications at positions 2, 5, 7, and 12 (numbered from the 5' end);
(iv) an SCP modification at position 1 (numbered from the 5' end); and/or
(v) phosphorothioate internucleotide linkages between nucleotide positions 1 and 2, between nucleotide positions 2 and 3, between nucleotide positions 19 and 20, and between nucleotide positions 20 and 21 (numbered from the 5' end).

**12.** The dsRNA of any one of claims 1-9, wherein the antisense strand has a length of 21 nucleotides and has

(i) 2'-O-methyl modified nucleotides at positions 3, 5, 9, 11, 13, 15, 17, 19, 20, and 21 (numbered from the 5' end);
(ii) 2'-fluoro modified nucleotides at positions 2, 4, 6, 8, 10, 12, 14, 16, and 18 (numbered from the 5' end);
(iii) a GNA modification at position 7 (numbered from the 5' end);
(iv) an SCP modification at position 1 (numbered from the 5' end); and/or
(v) phosphorothioate internucleotide linkages between nucleotide positions 1 and 2, between nucleotide positions 2 and 3, between nucleotide positions 19 and 20, and between nucleotide positions 20 and 21 (numbered from the 5' end).

**13.** The dsRNA of any one of claims 1-9, wherein the sense strand has a length of 19 nucleotides and has:

(i) 2'-O-methyl modified nucleotides at positions 1 to 6, and 10 to 19, and 2'-fluoro modified nucleotides at positions 7-9 (numbered from the 5' end); and/or
(ii) phosphorothioate internucleotide linkages between nucleotide positions 1 and 2, between nucleotide positions 2 and 3, and between nucleotide positions 18 and 19 (numbered from the 5' end).

**14.** The dsRNA of any one of claims 8-12, wherein the antisense strand comprises a sequence selected from any one of the sequences set forth in SEQ ID NOs: 81-105.

**15.** The dsRNA of any one of claims 8-9 and 13-14, wherein the sense strand comprises a sequence selected from any one of the sequences set forth in SEQ ID NOs: 41-59.

**16.** The dsRNA of claim 15, wherein the dsRNA comprises any of the paired of sense strand sequences and antisense strand sequences selected from:

| | | | |
|---|---|---|---|
| sense strand: | SEQ ID NO: 41, | antisense strand: | SEQ ID NO: 81; |
| sense strand: | SEQ ID NO: 42, | antisense strand: | SEQ ID NO: 82; |
| sense strand: | SEQ ID NO: 43, | antisense strand: | SEQ ID NO: 83; |
| sense strand: | SEQ ID NO: 44, | antisense strand: | SEQ ID NO: 84; |
| sense strand: | SEQ ID NO: 45, | antisense strand: | SEQ ID NO: 85; |
| sense strand: | SEQ ID NO: 46, | antisense strand: | SEQ ID NO: 86; |
| sense strand: | SEQ ID NO: 47, | antisense strand: | SEQ ID NO: 87; |
| sense strand: | SEQ ID NO: 48, | antisense strand: | SEQ ID NO: 88; |
| sense strand: | SEQ ID NO: 49, | antisense strand: | SEQ ID NO: 89; |
| sense strand: | SEQ ID NO: 50, | antisense strand: | SEQ ID NO: 90; |
| sense strand: | SEQ ID NO: 51, | antisense strand: | SEQ ID NO: 91; |
| sense strand: | SEQ ID NO: 52, | antisense strand: | SEQ ID NO: 92; |
| sense strand: | SEQ ID NO: 53, | antisense strand: | SEQ ID NO: 93; |
| sense strand: | SEQ ID NO: 54, | antisense strand: | SEQ ID NO: 94; |
| sense strand: | SEQ ID NO: 55, | antisense strand: | SEQ ID NO: 95; |
| sense strand: | SEQ ID NO: 56, | antisense strand: | SEQ ID NO: 96; |
| sense strand: | SEQ ID NO: 57, | antisense strand: | SEQ ID NO: 97; |
| sense strand: | SEQ ID NO: 58, | antisense strand: | SEQ ID NO: 98; and |
| sense strand: | SEQ ID NO: 59, | antisense strand: | SEQ ID NO: 99. |

**17.** The dsRNA of any one of claims 8-9 and 13-14, wherein the sense strand comprises a sequence selected from any one of the sequences set forth in SEQ ID NOs: 113-131.

**18.** The dsRNA of claim 17, wherein the dsRNA comprises any of the paired of sense strand sequences and antisense strand sequences selected from:

| | | | |
|---|---|---|---|
| sense strand: | SEQ ID NO: 113, | antisense strand: | SEQ ID NO: 81; |
| sense strand: | SEQ ID NO: 114, | antisense strand: | SEQ ID NO: 82; |
| sense strand: | SEQ ID NO: 115, | antisense strand: | SEQ ID NO: 83; |
| sense strand: | SEQ ID NO: 116, | antisense strand: | SEQ ID NO: 84; |
| sense strand: | SEQ ID NO: 117, | antisense strand: | SEQ ID NO: 85; |
| sense strand: | SEQ ID NO: 118, | antisense strand: | SEQ ID NO: 86; |
| sense strand: | SEQ ID NO: 119, | antisense strand: | SEQ ID NO: 87; |
| sense strand: | SEQ ID NO: 120, | antisense strand: | SEQ ID NO: 88; |
| sense strand: | SEQ ID NO: 121, | antisense strand: | SEQ ID NO: 89; |
| sense strand: | SEQ ID NO: 122, | antisense strand: | SEQ ID NO: 90; |
| sense strand: | SEQ ID NO: 123, | antisense strand: | SEQ ID NO: 91; |
| sense strand: | SEQ ID NO: 124, | antisense strand: | SEQ ID NO: 92; |
| sense strand: | SEQ ID NO: 125, | antisense strand: | SEQ ID NO: 93; |
| sense strand: | SEQ ID NO: 126, | antisense strand: | SEQ ID NO: 94; |
| sense strand: | SEQ ID NO: 127, | antisense strand: | SEQ ID NO: 95; |
| sense strand: | SEQ ID NO: 128, | antisense strand: | SEQ ID NO: 96; |
| sense strand: | SEQ ID NO: 129, | antisense strand: | SEQ ID NO: 97; |
| sense strand: | SEQ ID NO: 130, | antisense strand: | SEQ ID NO: 98; and |
| sense strand: | SEQ ID NO: 131, | antisense strand: | SEQ ID NO: 99. |

**19.** The dsRNA of any one of claims 1-18, wherein the dsRNA is further conjugated to a ligand moiety comprising N-acetylgalactosamine, and preferably the 3' end of the sense strand is conjugated to the ligand moiety.

**20.** The dsRNA of any one of claim 19, wherein the ligand has the following structure:

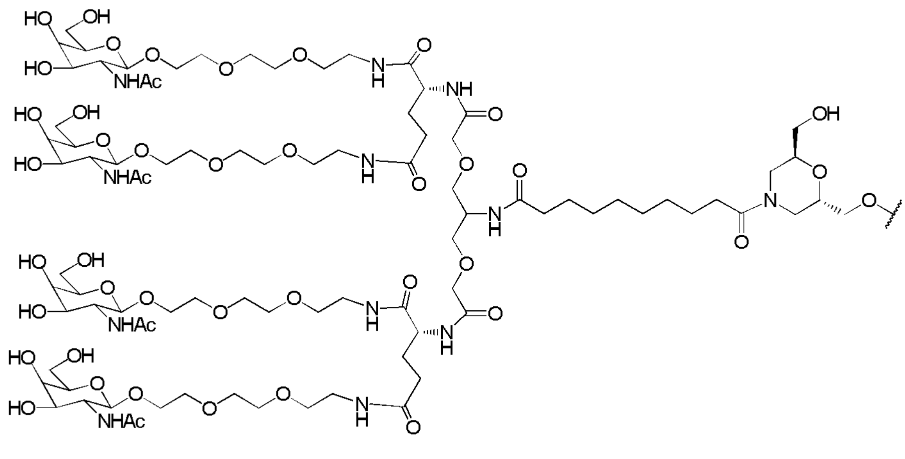

,

wherein

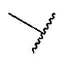

represents the point of attachment to the sense strand of the dsRNA via a phosphate group or a phosphorothioate group.

**21.** The dsRNA of claim 19 or claim 20, wherein the sense strand comprises a sequence selected from any one of the

sequences set forth in SEQ ID NOs: 60-79.

**22.** The dsRNA of claim 21, which comprises any of the paired of sense strand sequences and antisense strand sequences selected from:

| | | | |
|---|---|---|---|
| sense strand: | SEQ ID NO: 60, | antisense strand: | SEQ ID NO: 81; |
| sense strand: | SEQ ID NO: 61, | antisense strand: | SEQ ID NO: 82; |
| sense strand: | SEQ ID NO: 62, | antisense strand: | SEQ ID NO: 83; |
| sense strand: | SEQ ID NO: 63, | antisense strand: | SEQ ID NO: 84; |
| sense strand: | SEQ ID NO: 64, | antisense strand: | SEQ ID NO: 85; |
| sense strand: | SEQ ID NO: 65, | antisense strand: | SEQ ID NO: 86; |
| sense strand: | SEQ ID NO: 66, | antisense strand: | SEQ ID NO: 87; |
| sense strand: | SEQ ID NO: 67, | antisense strand: | SEQ ID NO: 88; |
| sense strand: | SEQ ID NO: 68, | antisense strand: | SEQ ID NO: 89; |
| sense strand: | SEQ ID NO: 69, | antisense strand: | SEQ ID NO: 90; |
| sense strand: | SEQ ID NO: 70, | antisense strand: | SEQ ID NO: 91; |
| sense strand: | SEQ ID NO: 71, | antisense strand: | SEQ ID NO: 92; |
| sense strand: | SEQ ID NO: 72, | antisense strand: | SEQ ID NO: 93; |
| sense strand: | SEQ ID NO: 73, | antisense strand: | SEQ ID NO: 94; |
| sense strand: | SEQ ID NO: 74, | antisense strand: | SEQ ID NO: 95; |
| sense strand: | SEQ ID NO: 75, | antisense strand: | SEQ ID NO: 96; |
| sense strand: | SEQ ID NO: 76, | antisense strand: | SEQ ID NO: 97; |
| sense strand: | SEQ ID NO: 77, | antisense strand: | SEQ ID NO: 98; |
| sense strand: | SEQ ID NO: 78, | antisense strand: | SEQ ID NO: 99; |
| sense strand: | SEQ ID NO: 72, | antisense strand: | SEQ ID NO: 100; |
| sense strand: | SEQ ID NO: 72, | antisense strand: | SEQ ID NO: 101; |
| sense strand: | SEQ ID NO: 72, | antisense strand: | SEQ ID NO: 102; |
| sense strand: | SEQ ID NO: 72, | antisense strand: | SEQ ID NO: 103; |
| sense strand: | SEQ ID NO: 79, | antisense strand: | SEQ ID NO: 104; and |
| sense strand: | SEQ ID NO: 79, | antisense strand: | SEQ ID NO: 105. |

**23.** A vector comprising a nucleotide sequence encoding the dsRNA of any one of claims 1-22.

**24.** A cell comprising the dsRNA of any one of claims 1-22 or the vector of claim 23.

**25.** A pharmaceutical composition comprising the dsRNA of any one of claims 1-22, the vector of claim 23, or the cell of claim 24, and optionally a pharmaceutically acceptable carrier or excipient.

**26.** A kit comprising the dsRNA of any one of claims 1-22, the vector of claim 23, or the cell of claim 24.

**27.** A method of treating a disease or disorder benefiting from reduced expression of angiotensinogen (AGT) in a subject, comprising a step of administering to the subject the dsRNA of any one of claims 1-22, the vector of claim 23, the cell of claim 24, or the pharmaceutical composition of claim 25.

**28.** A method of preventing at least one symptom in a subject that has a disease or disorder benefiting from reduced expression of angiotensinogen (AGT), comprising a step of administering to the subject the dsRNA of any one of claims 1-22, the vector of claim 23, the cell of claim 24, or the pharmaceutical composition of claim 25.

**29.** The method of claim 27 or 28, wherein the disease or disorder benefiting from reduced expression of angiotensinogen (AGT) is an AGT-mediated or AGT-related disease.

**30.** The method of claim 29, wherein the AGT-mediated or AGT-related disease is selected from the group consisting of: hypertension, ocular hypertension, glaucoma, hypertensive heart disease, hypertensive nephropathy, atherosclerosis, arteriosclerosis, vasculopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, heart failure,

myocardial infarction, angina pectoris, stroke, nephropathy, renal failure, systemic sclerosis, intrauterine growth restriction (IUGR), fetal growth restriction, obesity, hepatic steatosis/fatty liver disease, non-alcoholic steatohepatitis (NASH), non-alcoholic fatty liver disease (NAFLD); Glucose intolerance, type 2 diabetes (non-insulin-dependent diabetes ), and metabolic syndrome.

**31.** The method of claim 30, wherein the hypertension is selected from the group consisting of borderline hypertension, essential hypertension, secondary hypertension, isolated systolic or diastolic hypertension, pregnancy-related hypertension, diabetic hypertension, resistant hypertension, refractory hypertension, paroxysmal hypertension, renovascular hypertension, Goldblatt hypertension, pulmonary hypertension, portal hypertension, systemic venous hypertension, systolic hypertension, and unstable hypertension.

**32.** A method of reducing the level of angiotensinogen (AGT) in a subject, comprising a step of administering to the subject the dsRNA of any one of claims 1-22, the vector of claim 23, the cell of claim 24, or the pharmaceutical composition of claim 25.

**33.** The method of any one of claims 27-32, wherein the dsRNA, vector, cell, or pharmaceutical composition is administered by a subcutaneous, topical, or intravenous administration.

**34.** The method of any one of claims 27-33, wherein the subject is a human.

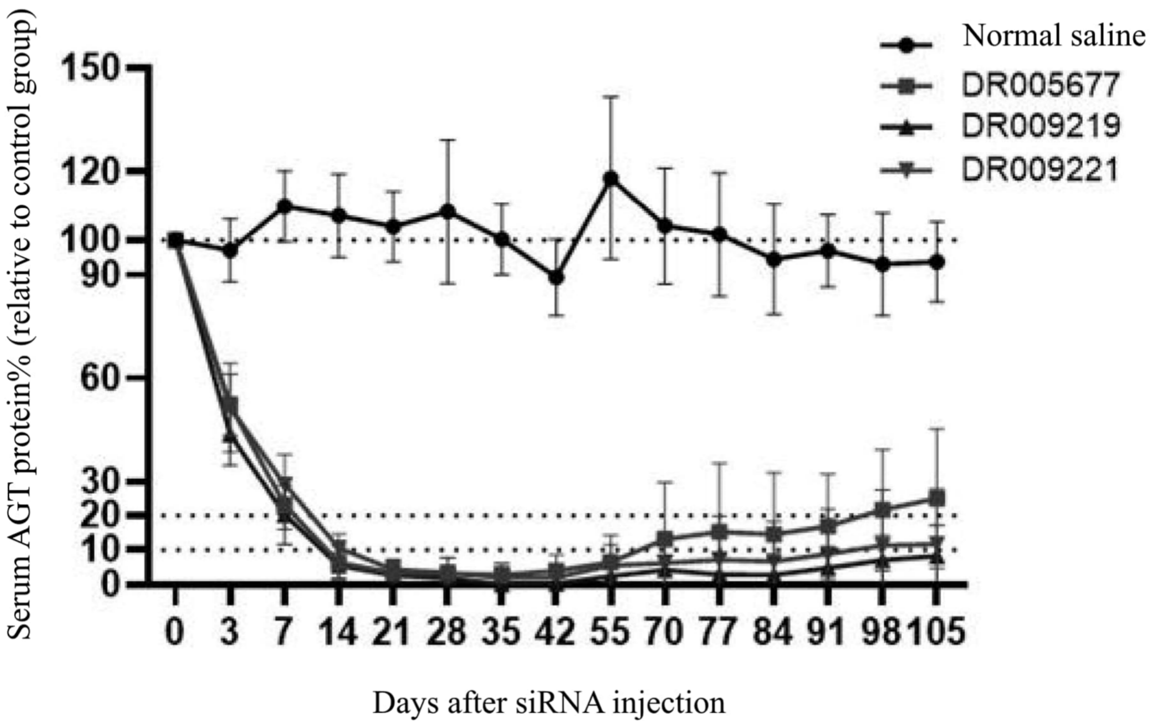
Serum AGT protein% (relative to control group)
150
120
100
90
60
30
20
10
0
0 3 7 14 21 28 35 42 55 70 77 84 91 98 105
Days after siRNA injection
Normal saline
DR005677
DR009219
DR009221

FIG. 1

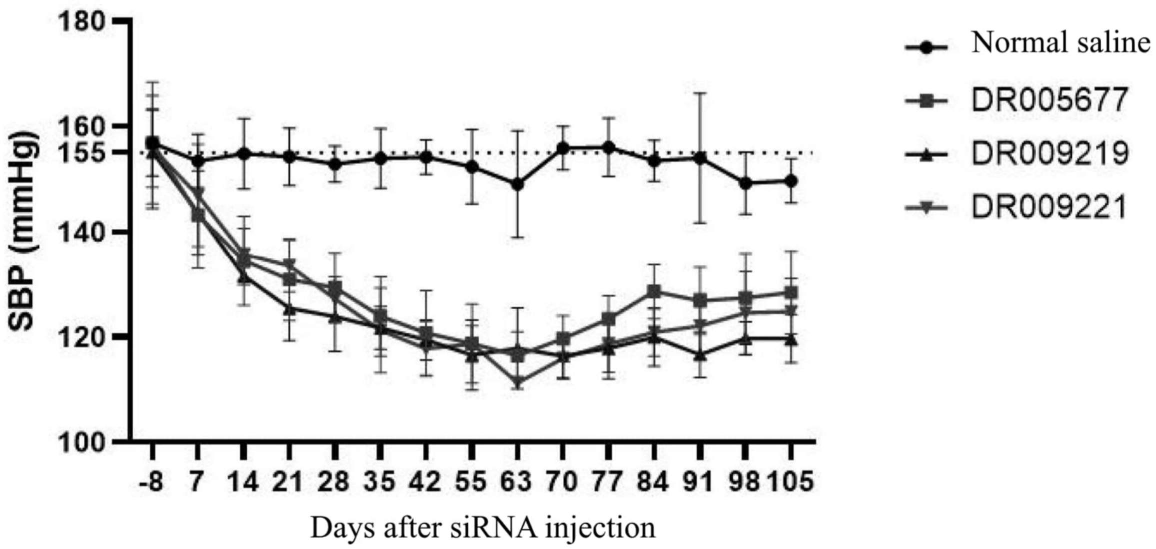
SBP (mmHg)
180
160
155
140
120
100
-8 7 14 21 28 35 42 55 63 70 77 84 91 98 105
Days after siRNA injection
Normal saline
DR005677
DR009219
DR009221

FIG. 2

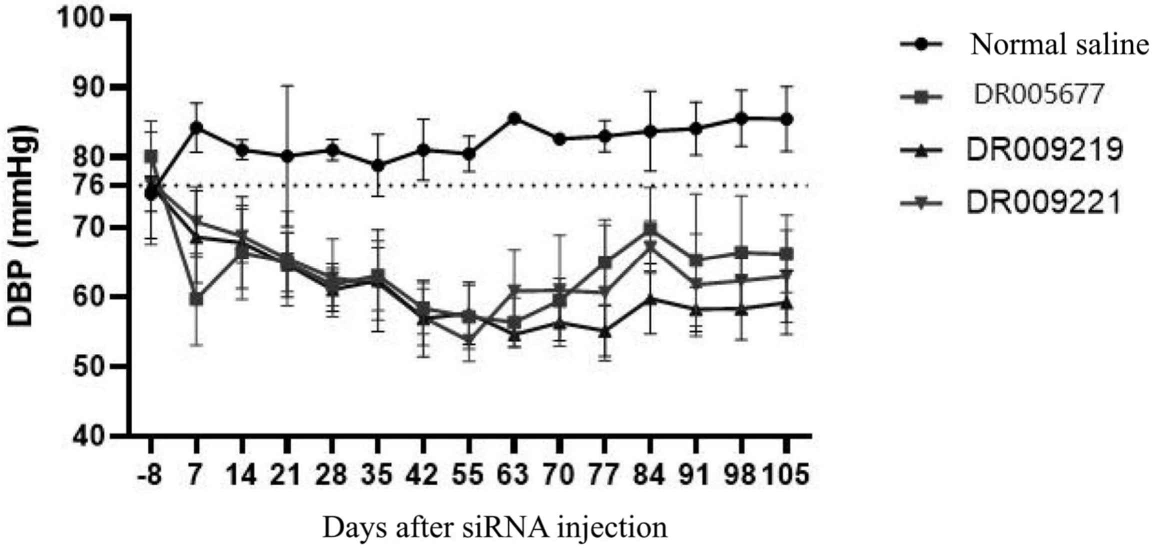
100
90
80
76
70
60
50
40
DBP (mmHg)
-8 7 14 21 28 35 42 55 63 70 77 84 91 98 105
Days after siRNA injection
Normal saline
DR005677
DR009219
DR009221

FIG. 3

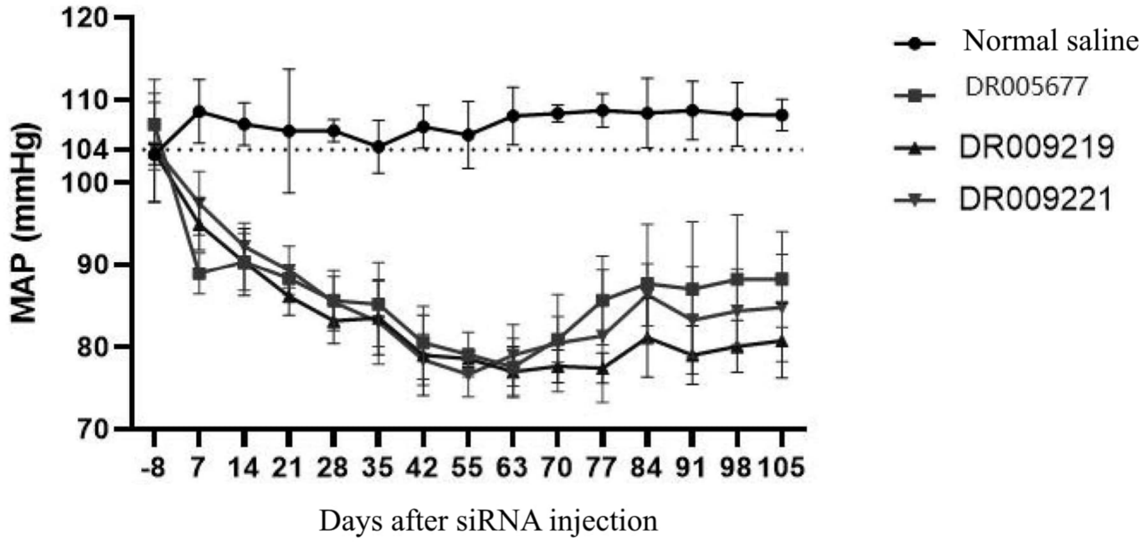
120
110
104
100
90
80
70
MAP (mmHg)
-8 7 14 21 28 35 42 55 63 70 77 84 91 98 105
Days after siRNA injection
Normal saline
DR005677
DR009219
DR009221

FIG. 4

**INTERNATIONAL SEARCH REPORT**

International application No.
**PCT/CN2024/103907**

**A. CLASSIFICATION OF SUBJECT MATTER**

C12N15/113(2010.01)i; A61K31/713(2006.01)i; A61P9/12(2006.01)i

According to International Patent Classification (IPC) or to both national classification and IPC

**B. FIELDS SEARCHED**

Minimum documentation searched (classification system followed by classification symbols)

IPC: C12N, A61K, A61P

Documentation searched other than minimum documentation to the extent that such documents are included in the fields searched

Electronic data base consulted during the international search (name of data base and, where practicable, search terms used)

CNTXT, WPABSC, WPABS, ENTXTC, ENTXT, DPWI, VEN, CNKI, 万方数据, WANFANG DATA, Web of Science, 百度学术, Baidu Scholar, HimmPat, incoPat, 读秀, DUXIU, 中国生物序列检索系统, China Biological Sequence Search System, NCBI, EBI, STNext: 大睿生物, darui biomedical, 黄金宇, 叶飘, huang jinyu, ye piao, 血管紧张素原, AGT, 双链核糖核酸, dsRNA, 正义链, 反义链, angiotensinogen, double stranded ribonucleic acid, sense strand, antisense strand, 序列1, 21, sequences 1 and 21

**C. DOCUMENTS CONSIDERED TO BE RELEVANT**

| Category* | Citation of document, with indication, where appropriate, of the relevant passages | Relevant to claim No. |
|---|---|---|
| PX | WO 2024031101 A1 (SANEGENE BIO USA INC.) 08 February 2024 (2024-02-08) description, paragraphs 5-7, 19, 22, 26, 27, 56 and 64, and sequence 357 | 1-34 |
| X | WO 2023088227 A1 (SHANGHAI ARGO BIOPHARMACEUTICAL CO., LTD.) 25 May 2023 (2023-05-25) description, page 2, paragraph 3, page 5, paragraph 3, page 19, paragraphs 1-3, and page 20, paragraphs 1-3, table 3, and sequences 74, 171, 318, 338, 412 and 432 | 1-34 |
| X | CN 112313335 A (ALNYLAM PHARMACEUTICALS INC.) 02 February 2021 (2021-02-02) description, paragraphs 9, 10, 14, 25, 39 and 44, and sequence 209 | 1-34 |
| A | WO 2023014765 A1 (ALNYLAM PHARMACEUTICALS, INC.) 09 February 2023 (2023-02-09) description, page 2, paragraphs 3-4, page 3, paragraphs 5-7, page 4, paragraphs 2-5, and page 7, paragraphs 3-7, and table 2 | 1-34 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 September 2024** | **26 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2024/103907**

**C. DOCUMENTS CONSIDERED TO BE RELEVANT**

| Category* | Citation of document, with indication, where appropriate, of the relevant passages | Relevant to claim No. |
|---|---|---|
| A | CN 106574268 A (ALNYLAM PHARMACEUTICALS INC.) 19 April 2017 (2017-04-19) description, paragraphs 11-13, 17, 63-69 and 153-158, and table 3 | 1-34 |
| A | CN 114981431 A (ALNYLAM PHARMACEUTICALS INC.) 30 August 2022 (2022-08-30) description, paragraphs 11, 15, 20-24, 27, 28, 32, 35, 36, 56, 65, 98 and 105, and sequences 9 and 10 | 1-34 |

Form PCT/ISA/210 (second sheet) (July 2022)

**INTERNATIONAL SEARCH REPORT**

International application No.
**PCT/CN2024/103907**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

**INTERNATIONAL SEARCH REPORT**

International application No.
**PCT/CN2024/103907**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **27-34**
because they relate to subject matter not required to be searched by this Authority, namely:

Claims 27-31 set forth a method for treating or preventing diseases or conditions that benefit from decreased expression of angiotensinogen (AGT) in a subject, and claims 32-34 set forth a method for reducing the level of angiotensinogen (AGT) in a subject, which both relate to the treatment for diseases related to the decreased expression of angiotensinogen (AGT), and the methods are methods for treatment of diseases; therefore, said claims do not comply with PCT Rule 39.1(iv). However, this search is performed on the basis that the subject matter described above is amended to be a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

Invention 1: claims 1-34 (in part) relate to a double-stranded nucleotide, which inhibits the expression of angiotensinogen (AGT) in cells and of which the nucleotide sequence of a sense strand is as shown in SEQ ID NO: 1 and the nucleotide sequence of an antisense strand is as shown in SEQ ID NO: 21, and the technical solutions related thereto.

Invention 2: claims 1-34 (in part) relate to a double-stranded nucleotide, which inhibits the expression of angiotensinogen (AGT) in cells and of which the nucleotide sequence of a sense strand is as shown in SEQ ID NO: 2 and the nucleotide sequence of an antisense strand is as shown in SEQ ID NO: 22, and the technical solutions related thereto.

Invention 3: claims 1-34 (in part) relate to a double-stranded nucleotide, which inhibits the expression of angiotensinogen (AGT) in cells and of which the nucleotide sequence of a sense strand is as shown in SEQ ID NO: 3 and the nucleotide sequence of an antisense strand is as shown in SEQ ID NO: 23, and the technical solutions related thereto.

Invention 4: claims 1-34 (in part) relate to a double-stranded nucleotide, which inhibits the expression of angiotensinogen (AGT) in cells and of which the nucleotide sequence of a sense strand is as shown in SEQ ID NO: 4 and the nucleotide sequence of an antisense strand is as shown in SEQ ID NO: 24, and the technical solutions related thereto.

Invention 5: claims 1-34 (in part) relate to a double-stranded nucleotide, which inhibits the expression of angiotensinogen (AGT) in cells and of which the nucleotide sequence of a sense strand is as shown in SEQ ID NO: 5 and the nucleotide sequence of an antisense strand is as shown in SEQ ID NO: 25, and the technical solutions related thereto.

Invention 6: claims 1-34 (in part) relate to a double-stranded nucleotide, which inhibits the expression of angiotensinogen (AGT) in cells and of which the nucleotide sequence of a sense strand is as shown in SEQ ID NO: 6 and the nucleotide sequence of an antisense strand is as shown in SEQ ID NO: 26, and the technical solutions related thereto.

Invention 7: claims 1-34 (in part) relate to a double-stranded nucleotide, which inhibits the expression of angiotensinogen (AGT) in cells and of which the nucleotide sequence of a sense strand is as shown in SEQ ID NO: 7 and the nucleotide sequence of an antisense strand is as shown in SEQ ID NO: 27, and the technical solutions related thereto.

Form PCT/ISA/210 (continuation of first sheet) (July 2022)

INTERNATIONAL SEARCH REPORT

International application No. PCT/CN2024/103907

**Box No. III Observations where unity of invention is lacking (Continuation of item 3 of first sheet)**

Invention 8: claims 1-34 (in part) relate to a double-stranded nucleotide, which inhibits the expression of angiotensinogen (AGT) in cells and of which the nucleotide sequence of a sense strand is as shown in SEQ ID NO: 8 and the nucleotide sequence of an antisense strand is as shown in SEQ ID NO: 28, and the technical solutions related thereto.

Invention 9: claims 1-34 (in part) relate to a double-stranded nucleotide, which inhibits the expression of angiotensinogen (AGT) in cells and of which the nucleotide sequence of a sense strand is as shown in SEQ ID NO: 9 and the nucleotide sequence of an antisense strand is as shown in SEQ ID NO: 29, and the technical solutions related thereto.

Invention 10: claims 1-34 (in part) relate to a double-stranded nucleotide, which inhibits the expression of angiotensinogen (AGT) in cells and of which the nucleotide sequence of a sense strand is as shown in SEQ ID NO: 10 and the nucleotide sequence of an antisense strand is as shown in SEQ ID NO: 30, and the technical solutions related thereto.

Invention 11: claims 1-34 (in part) relate to a double-stranded nucleotide, which inhibits the expression of angiotensinogen (AGT) in cells and of which the nucleotide sequence of a sense strand is as shown in SEQ ID NO: 11 and the nucleotide sequence of an antisense strand is as shown in SEQ ID NO: 31, and the technical solutions related thereto.

Invention 12: claims 1-34 (in part) relate to a double-stranded nucleotide, which inhibits the expression of angiotensinogen (AGT) in cells and of which the nucleotide sequence of a sense strand is as shown in SEQ ID NO: 12 and the nucleotide sequence of an antisense strand is as shown in SEQ ID NO: 32, and the technical solutions related thereto.

Invention 13: claims 1-34 (in part) relate to a double-stranded nucleotide, which inhibits the expression of angiotensinogen (AGT) in cells and of which the nucleotide sequence of a sense strand is as shown in SEQ ID NO: 13 and the nucleotide sequence of an antisense strand is as shown in SEQ ID NO: 33, and the technical solutions related thereto.

Invention 14: claims 1-34 (in part) relate to a double-stranded nucleotide, which inhibits the expression of angiotensinogen (AGT) in cells and of which the nucleotide sequence of a sense strand is as shown in SEQ ID NO: 14 and the nucleotide sequence of an antisense strand is as shown in SEQ ID NO: 34, and the technical solutions related thereto.

Invention 15: claims 1-34 (in part) relate to a double-stranded nucleotide, which inhibits the expression of angiotensinogen (AGT) in cells and of which the nucleotide sequence of a sense strand is as shown in SEQ ID NO: 15 and the nucleotide sequence of an antisense strand is as shown in SEQ ID NO: 35, and the technical solutions related thereto.

Invention 16: claims 1-34 (in part) relate to a double-stranded nucleotide, which inhibits the expression of angiotensinogen (AGT) in cells and of which the nucleotide sequence of a sense strand is as shown in SEQ ID NO: 16 and the nucleotide sequence of an antisense strand is as shown in SEQ ID NO: 36, and the technical solutions related thereto.

Invention 17: claims 1-34 (in part) relate to a double-stranded nucleotide, which inhibits the expression of angiotensinogen (AGT) in cells and of which the nucleotide sequence of a sense strand is as shown in SEQ ID NO: 17 and the nucleotide sequence of an antisense strand is as shown in SEQ ID NO: 37, and the technical solutions related thereto.

Invention 18: claims 1-34 (in part) relate to a double-stranded nucleotide, which inhibits the expression of angiotensinogen (AGT) in cells and of which the nucleotide sequence of a sense strand is as shown in SEQ ID NO: 18 and the nucleotide sequence of an antisense strand is as shown in SEQ ID NO: 38, and the technical solutions related thereto.

Invention 19: claims 1-34 (in part) relate to a double-stranded nucleotide, which inhibits the expression of angiotensinogen (AGT) in cells and of which the nucleotide sequence of a sense strand is as shown in SEQ ID NO: 19 and the nucleotide sequence of an antisense strand is as shown in SEQ ID NO: 39, and the technical solutions related thereto.

Invention 20: claims 1-34 (in part) relate to a double-stranded nucleotide, which inhibits the expression of angiotensinogen (AGT) in cells and of which the nucleotide sequence of a sense strand is as shown in SEQ ID NO:

**INTERNATIONAL SEARCH REPORT**

International application No.
**PCT/CN2024/103907**

**Box No. III Observations where unity of invention is lacking (Continuation of item 3 of first sheet)**

20 and the nucleotide sequence of an antisense strand is as shown in SEQ ID NO: 40, and the technical solutions related thereto.

The common technical features comprised in inventions 1 to 20 are: a double-stranded ribonucleic acid (dsRNA) for inhibiting the expression of angiotensinogen (AGT) in cells, wherein the dsRNA comprises a sense strand and an antisense strand, which form a double-stranded region, and the length of the sense strand and the length of the antisense strand are each independently 15-30 nucleotides. However, the technical features described above are disclosed in the prior art in the art, for example, WO 2023014765 A1 (publication date: 09 February 2023) discloses a double-stranded ribonucleic acid (dsRNA) for inhibiting the expression of angiotensinogen (AGT) in cells, wherein the sequences of a sense strand and an antisense strand of the dsRNA are listed in table 2, and the length of the sense strand and the length of the antisense strand are each within the range of 15-30; and CN 106574268 A (publication date: 19 April 2017) discloses a double-stranded ribonucleic acid interference (RNAi) agent for inhibiting the expression of angiotensinogen (AGT) in cells, wherein the double-stranded RNAi agent comprises a sense strand and an antisense strand, which form a double-stranded region, the sequences of the sense strand and antisense strand of the dsRNA are listed in table 3, and the length of the sense strand and the length of the antisense strand are each within the range of 15-30. Therefore, inventions 1 to 20 do not have a same or corresponding special technical features, and thus do not comply with PCT Rule 13(2).

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-34 (in part)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2024/103907**

| Patent document cited in search report | Publication date (day/month/year) | Patent family member(s) | Publication date (day/month/year) |
|---|---|---|---|
| WO 2024031101 A1 | 08 February 2024 | None | |
| WO 2023088227 A1 | 25 May 2023 | KR 20240103025 A | 03 July 2024 |
| | | US 2024084304 A1 | 14 March 2024 |
| | | ECSP 24036299 A | 31 July 2024 |
| | | CO 2024006020 A2 | 08 August 2024 |
| | | AU 2022394667 A1 | 13 June 2024 |
| | | TW 202334422 A | 01 September 2023 |
| | | IL 312811 A | 01 July 2024 |
| | | CA 3238865 A1 | 25 May 2023 |
| CN 112313335 A | 02 February 2021 | ES 2967713 T3 | 03 May 2024 |
| | | PE 20210114 A1 | 19 January 2021 |
| | | EP 3794122 A1 | 24 March 2021 |
| | | EP 3794122 B1 | 09 August 2023 |
| | | US 2021310006 A1 | 07 October 2021 |
| | | US 11834661 B2 | 05 December 2023 |
| | | CL 2023002870 A1 | 28 June 2024 |
| | | JP 2021522841 A | 02 September 2021 |
| | | JP 7346460 B2 | 19 September 2023 |
| | | IL 278539 B | 01 June 2022 |
| | | MX 2020012048 A | 29 January 2021 |
| | | ZA 202108348 B | 29 November 2023 |
| | | RS 64812 B1 | 29 December 2023 |
| | | EP 4324520 A2 | 21 February 2024 |
| | | IL 292877 A | 01 July 2022 |
| | | IL 292877 B1 | 01 April 2024 |
| | | CO 2020015314 A2 | 19 March 2021 |
| | | JP 2023182578 A | 26 December 2023 |
| | | WO 2019222166 A1 | 21 November 2019 |
| | | KR 20210010529 A | 27 January 2021 |
| | | US 2021095290 A1 | 01 April 2021 |
| | | US 11015201 B2 | 25 May 2021 |
| | | SG 11202011144 QA | 30 December 2020 |
| | | CL 2021002326 A1 | 13 May 2022 |
| | | LT 3794122 T | 27 November 2023 |
| | | TW 202016304 A | 01 May 2020 |
| | | PT 3794122 T | 22 November 2023 |
| | | BR 112020022943 A2 | 17 February 2021 |
| | | CA 3100003 A1 | 21 November 2019 |
| | | FI 3794122 T3 | 07 November 2023 |
| | | HUE 064073 T2 | 28 February 2024 |
| | | SI 3794122 T1 | 29 February 2024 |
| | | US 2024191236 A1 | 13 June 2024 |
| | | AU 2019269418 A1 | 07 January 2021 |
| | | CL 2020002865 A1 | 30 April 2021 |
| | | DK 3794122 T3 | 13 November 2023 |
| | | DK 3794122 T5 | 05 August 2024 |
| | | PL 3794122 T3 | 11 March 2024 |
| | | PH 12020551904 A1 | 21 June 2021 |
| | | HRP 20231412 T1 | 16 February 2024 |
| WO 2023014765 A1 | 09 February 2023 | None | |

Form PCT/ISA/210 (patent family annex) (July 2022)

**INTERNATIONAL SEARCH REPORT**
**Information on patent family members**

International application No.
**PCT/CN2024/103907**

| Patent document cited in search report | Publication date (day/month/year) | Patent family member(s) | Publication date (day/month/year) |
|---|---|---|---|
| CN 106574268 A | 19 April 2017 | SG 11201609376 SA | 29 December 2016 |
| | | WO 2015179724 A1 | 26 November 2015 |
| | | WO 2015179724 A8 | 18 February 2016 |
| | | WO 2015179724 A9 | 10 March 2016 |
| | | JP 2017517511 A | 29 June 2017 |
| | | JP 6811094 B2 | 13 January 2021 |
| | | BR 122020023687 B1 | 07 March 2023 |
| | | IL 281638 A | 31 May 2021 |
| | | IL 281638 B1 | 01 March 2023 |
| | | IL 281638 B2 | 01 July 2023 |
| | | IL 248816 A0 | 31 January 2017 |
| | | IL 248816 B | 29 July 2021 |
| | | US 2023123192 A1 | 20 April 2023 |
| | | AU 2015264038 A1 | 01 December 2016 |
| | | AU 2015264038 A2 | 22 December 2016 |
| | | AU 2015264038 B2 | 11 February 2021 |
| | | EP 3739048 A1 | 18 November 2020 |
| | | US 2019298842 A1 | 03 October 2019 |
| | | US 10814007 B2 | 27 October 2020 |
| | | CA 2948381 A1 | 26 November 2015 |
| | | CA 2948381 C | 07 November 2023 |
| | | AU 2021202552 A1 | 27 May 2021 |
| | | BR 112016026950 A2 | 31 October 2017 |
| | | BR 112016026950 B1 | 07 March 2023 |
| | | KR 20170005130 A | 11 January 2017 |
| | | KR 102410687 B1 | 22 June 2022 |
| | | US 2017189541 A1 | 06 July 2017 |
| | | US 10238749 B2 | 26 March 2019 |
| | | JP 2022104985 A | 12 July 2022 |
| | | MX 2021006053 A | 06 July 2021 |
| | | KR 20220087576 A | 24 June 2022 |
| | | JP 2021063085 A | 22 April 2021 |
| | | JP 7101748 B2 | 15 July 2022 |
| | | SG 10202104570 TA | 29 June 2021 |
| | | EA 201692370 A1 | 31 March 2017 |
| | | EP 3146049 A1 | 29 March 2017 |
| | | EP 3146049 B1 | 26 February 2020 |
| | | US 2021046187 A1 | 18 February 2021 |
| | | US 11419942 B2 | 23 August 2022 |
| | | CA 3215908 A1 | 26 November 2015 |
| | | MX 2016015126 A | 23 February 2017 |
| | | ZA 201607666 B | 30 January 2019 |
| CN 114981431 A | 30 August 2022 | JP 2023502038 A | 20 January 2023 |
| | | CL 2022001256 A1 | 10 February 2023 |
| | | KR 20220115946 A | 19 August 2022 |
| | | MX 2022005692 A | 08 June 2022 |
| | | TW 202132568 A | 01 September 2021 |
| | | CO 2022006092 A2 | 20 May 2022 |
| | | BR 112022009216 A2 | 02 August 2022 |
| | | AU 2020382478 A1 | 02 June 2022 |

Form PCT/ISA/210 (patent family annex) (July 2022)

**INTERNATIONAL SEARCH REPORT**
**Information on patent family members**

International application No.
**PCT/CN2024/103907**

| Patent document cited in search report | Publication date (day/month/year) | Patent family member(s) | | | Publication date (day/month/year) |
|---|---|---|---|---|---|
| | | US | 2023002765 | A1 | 05 January 2023 |
| | | PE | 20230179 | A1 | 01 February 2023 |
| | | IL | 292865 | A | 01 July 2022 |
| | | CA | 3161703 | A1 | 20 May 2021 |
| | | WO | 2021096763 | A1 | 20 May 2021 |
| | | WO | 2021096763 | A8 | 24 June 2021 |
| | | EP | 4058577 | A1 | 21 September 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011139702 A
- WO 2013033230 A
- WO 2019105419 A
- WO 2019222166 A1
- WO 2020132227 A2
- WO 2016011123 A
- WO 2019051402 A
- WO 2009073809 A
- WO 2009082607 A
- WO 2016081444 A

### Non-patent literature cited in the description

- **CZAUDERNA**. *Nucleic Acids Res.*, 2003, vol. 31 (11), 2705-16